(19) Europäisches Patentamt
European Patent Office
Office européen des brevets

(11) EP 1 978 108 A2

(12) **EUROPEAN PATENT APPLICATION**

(43) Date of publication:
**08.10.2008 Bulletin 2008/41**

(21) Application number: **08006507.1**

(22) Date of filing: **07.01.2003**

(51) Int Cl.:
***C12Q 1/68*** *(2006.01)* ***C12N 15/12*** *(2006.01)*
***C07K 14/705*** *(2006.01)* ***G01N 33/68*** *(2006.01)*
***A61K 31/713*** *(2006.01)* ***A61P 9/00*** *(2006.01)*

(84) Designated Contracting States:
**AT BE BG CH CY CZ DE DK EE ES FI FR GB GR HU IE IT LI LU MC NL PT SE SI SK TR**

(30) Priority: **08.01.2002 EP 02000153**

(62) Document number(s) of the earlier application(s) in accordance with Art. 76 EPC:
**03729224.0 / 1 465 998**

(71) Applicant: **Siemens Medical Solutions Diagnostics GmbH**
**91052 Erlangen (DE)**

(72) Inventors:
- **Kallabis, Harald, Dr.**
  **51375 Leverkusen (DE)**
- **Schwers, Stephan, Dr.**
  **50827 Köln (DE)**
- **Stropp, Udo, Dr.**
  **42871 Haan (DE)**

(74) Representative: **Maier, Daniel Oliver**
**Siemens AG**
**Postfach 22 16 34**
**80506 München (DE)**

Remarks:
This application was filed on 31-03-2008 as a divisional application to the application mentioned under INID code 62.

(54) **Single nucleotide polymorphisms predicting cardiovascular disease and medication efficacy**

(57) The present invention relates to isolated polynucleotides encoding a cardiovascular associated (CA) gene polypeptide useful in methods to identify therapeutic agents and useful for preparation of a medicament to treat cardiovascular disease, the polynucleotide is selected from the group comprising:SEQ ID 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 with allelic variation as indicated in the sequences section contained in a functional surrounding like full length cDNA for CA gene polypeptide and with or without the CA gene promoter sequence. The invention also provides diagnostic methods and kits including antibodies determining whether a human subject is at risk for a cardiovascular disease. The invention provides further polymorphic sequences and other genes.

EP 1 978 108 A2

## Description

### Technical Field

This invention relates to genetic polymorphisms useful for assessing cardiovascular risks in humans, including, but not limited to; atherosclerosis, ischemia/reperfusion, hypertension, restenosis, arterial inflammation, myocardial infarction, and stroke. Specifically, the present invention identifies and describes gene variations which are individually present in humans with cardiovascular disease states, relative to humans with normal, or non-cardiovascular disease states, and/or in response to medications relevant to cardiovascular disease. Further, the present invention provides methods for the identification and therapeutic use of compounds as treatments of cardiovascular disease. Moreover, the present invention provides methods for the diagnostic monitoring of patients undergoing clinical evaluation for the treatment of cardiovascular disease, and for monitoring the efficacy of compounds in clinical trials. Still further, the present invention provides methods to use gene variations to predict personal medication schemes omitting adverse drug reactions. Additionally, the present invention describes methods for the diagnostic evaluation and prognosis of various cardiovascular diseases, and for the identification of subjects exhibiting a predisposition to such conditions.

### Background of the Invention

Cardiovascular disease is a major health risk throughout the industrialized world.

Cardiovascular diseases include but are not limited by the following disorders of the heart and the vascular system: congestive heart failure, myocardial infarction, atherosclerosis, ischemic diseases of the heart, coronary heart disease, all kinds of atrial and ventricular arrhythmias, hypertensive vascular diseases and peripheral vascular diseases.

Heart failure is defined as a pathophysiologic state in which an abnormality of cardiac function is responsible for the failure of the heart to pump blood at a rate commensurate with the requirement of the metabolizing tissue. It includes all forms of pumping failure such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in an perfusion which is inadequate to meet the myocardial requirement for oxygen. This group of diseases include stable angina, unstable angina and asymptomatic ischemia.

Arrhythmias include all forms of atrial and ventricular tachyarrhythmias (atrial tachycardia, atrial flutter, atrial fibrillation, atrio-ventricular reentrant tachycardia, preexitation syndrome, ventricular tachycardia, ventricular flutter, ventricular fibrillation) as well as bradycardic forms of arrhythmias.

Hypertensive vascular diseases include primary as well as all kinds of secondary arterial hypertension (renal, endocrine, neurogenic, others).

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Atherosclerosis, the most prevalent of vascular diseases, is the principal cause of heart attack, stroke, and gangrene of the extremities, and thereby the principal cause of death. Atherosclerosis is a complex disease involving many cell types and molecular factors (for a detailed review, see Ross, 1993, Nature 362: 801-809 and Lusis, A. J., Nature 407, 233-241 (2000)). The process, in normal circumstances a protective response to insults to the endothelium and smooth muscle cells (SMCs) of the wall of the artery, consists of the formation of fibrofatty and fibrous lesions or plaques, preceded and accompanied by inflammation. The advanced lesions of atherosclerosis may occlude the artery concerned, and result from an excessive inflammatory-fibroproliferative response to numerous different forms of insult. For example, shear stresses are thought to be responsible for the frequent occurrence of atherosclerotic plaques in regions of the circulatory system where turbulent blood flow occurs, such as branch points and irregular structures.

The first observable event in the formation of an atherosclerotic plaque occurs when blood-borne monocytes adhere to the vascular endothelial layer and transmigrate through to the sub-endothelial space. Adjacent endothelial cells at the same time produce oxidized low density lipoprotein (LDL). These oxidized LDLs are then taken up in large amounts by the monocytes through scavenger receptors expressed on their surfaces. In contrast to the regulated pathway by which native LDL (nLDL) is taken up by nLDL specific receptors, the scavenger pathway of uptake is not regulated by the monocytes.

These lipid-filled monocytes are called foam cells, and are the major constituent of the fatty streak. Interactions between foam cells and the endothelial and SMCs which surround them lead to a state of chronic local inflammation which can eventually lead to smooth muscle cell proliferation and migration, and the formation of a fibrous plaque. Such plaques occlude the blood vessel concerned and thus restrict the flow of blood, resulting in ischemia.

Ischemia is a condition characterized by a lack of oxygen supply in tissues of organs due to inadequate perfusion. Such inadequate perfusion can have number of natural causes, including atherosclerotic or restenotic lesions, anemia, or stroke, to name a few. Many medical interventions, such as the interruption of the flow of blood during bypass surgery, for example, also lead to ischemia. In addition to sometimes being caused by diseased cardiovascular tissue, ischemia may sometimes affect cardiovascular tissue, such as in ischemic heart disease. Ischemia may occur in any organ, however, that is suffering a lack of oxygen supply.

The most common cause of ischemia in the heart is atherosclerotic disease of epicardial coronary arteries. By reducing the lumen of these vessels, atherosclerosis causes an absolute decrease in myocardial perfusion in the basal state or limits appropriate increases in perfusion when the demand for flow is augmented. Coronary blood flow can also be limited by arterial thrombi, spasm, and, rarely, coronary emboli, as well as by ostial narrowing due to luetic aortitis. Congenital abnormalities, such as anomalous origin of the left anterior descending coronary artery from the pulmonary artery, may cause myocardial ischemia and infarction in infancy, but this cause is very rare in adults. Myocardial ischemia can also occur if myocardial oxygen demands are abnormally increased, as in severe ventricular hypertrophy due to hypertension or aortic stenosis. The latter can be present with angina that is indistinguishable from that caused by coronary atherosclerosis. A reduction in the oxygen-carrying capacity of the blood, as in extremely severe anemia or in the presence of carboxy-hemoglobin, is a rare cause of myocardial ischemia. Not infrequently, two or more causes of ischemia will coexist, such as an increase in oxygen demand due to left ventricular hypertrophy and a reduction in oxygen supply secondary to coronary atherosclerosis.

The foregoing studies are aimed at defining the role of particular gene variations presumed to be involved in the misleading of normal cellular function leading to cardiovascular disease. However, such approaches cannot identify the full panoply of gene variations that are involved in the disease process.

At present, the only available treatments for cardiovascular disorders are pharmaceutical based medications that are not targeted to an individual's actual defect; examples include angiotensin converting enzyme (ACE) inhibitors and diuretics for hypertension, insulin supplementation for non-insulin dependent diabetes mellitus (NIDDM), cholesterol reduction strategies for dyslipidaemia, anticoagulants, β blockers for cardiovascular disorders and weight reduction strategies for obesity. If targeted treatment strategies were available it might be possible to predict the response to a particular regime of therapy and could markedly increase the effectiveness of such treatment. Although targeted therapy requires accurate diagnostic tests for disease susceptibility, once these tests are developed the opportunity to utilize targeted therapy will become widespread. Such diagnostic tests could initially serve to identify individuals at most risk of hypertension and could allow them to make changes in lifestyle or diet that would serve as preventative measures. The benefits associated by coupling the diagnostic tests with a system of targeted therapy could include the reduction in dosage of administered drugs and thus the amount of unpleasant side effects suffered by an individual. In more severe cases a diagnostic test may suggest that earlier surgical intervention would be useful in preventing a further deterioration in condition.

It is an object of the invention to provide genetic diagnosis of predisposition or susceptibility for cardiovascular diseases. Another related object is to provide treatment to reduce or prevent or delay the onset of disease in those predisposed or susceptible to this disease. A further object is to provide means for carrying out this diagnosis.

Accordingly, a first aspect of the invention provides a method of diagnosis of disease in an individual, said method comprising determining one, various or all genotypes in said individual of the genes listed in the Examples.

In another aspect, the invention provides a method of identifying an individual predisposed or susceptible to a disease, said method comprising determining one, various or all genotypes in said individual of the genes listed in the Examples.

The invention is of advantage in that it enables diagnosis of a disease or of certain disease states via genetic analysis which can yield useable results before onset of disease symptoms, or before onset of severe symptoms. The invention is further of advantage in that it enables diagnosis of predisposition or susceptibility to a disease or of certain disease states via genetic analysis.

The invention may also be of use in confirming or corroborating the results of other diagnostic methods. The diagnosis of the invention may thus suitably be used either as an isolated technique or in combination with other methods and apparatus for diagnosis, in which latter case the invention provides a further test on which a diagnosis may be assessed.

The present invention stems from using allelic association as a method for genotyping individuals; allowing the investigation of the molecular genetic basis for cardiovascular diseases. In a specific embodiment the invention tests for the polymorphisms in the sequences of the listed genes in the Examples. The invention demonstrates a link between this polymorphisms and predispositions to cardiovascular diseases by showing that allele frequencies significantly differ when individuals with "bad" serum lipids are compared to individuals with "good" serum levels. The meaning of "good and bad" serum lipid levels is defined in Table 1.

Certain disease states would benefit, that is to say the suffering of the patient may be reduced or prevented or delayed, by administration of treatment or therapy in advance of disease appearance; this can be more reliably carried out if advance diagnosis of predisposition or susceptibility to disease can be diagnosed.

Detailed Description of the Invention

The present invention is based at least in part on the discovery that a specific allele of a polymorphic region of a so called "candidate gene" is associated with CVD.

"Candidate gene" as used herein includes genes that can be assigned to either normal cardiovascular function or to metabolic pathways that are related to onset and/or progression of cardiovascular diseases. As the development of cardiovascular diseases is not completely understood, the term "candidate gene" may also comprise genes with presently unknown function.

For the present invention the following candidate genes were analyzed:

- Genes found to be expressed in cardiac tissue (Hwang et al., Circulation 1997, 96:4146-4203).
- Genes from the following metabolic pathways and their regulatory elements:

**Lipid metabolism**

Numerous studies have shown a connection between serum lipid levels and cardiovascular diseases. Candidate genes falling into this group include but are not limited by genes of the cholesterol pathway, apolipoproteins and their modifiying factors.

**Coagulation**

Ischemic diseases of the heart and in particular myocardial infarction may be caused by a thrombotic occlusion. Genes falling into this group include all genes of the coagulation cascade and their regulatory elements.

**Inflammation**

Complications of atherosclerosis are the most common causes of death in Western societies. In broad outline atherosclerosis can be considered to be a form of chronic inflammation resulting from interaction modified lipoproteins, monocyte-derived macrophages,T cells, and the normal cellular elements of the arterial wall. This inflammatory process can ultimately lead to the development of complex lesions, or plaques, that protrude into the arterial lumen. Finally plaque rupture and thrombosis result in the acute clinical complications of myocardial infarction and stroke (Glass et al., Cell 2001, 104:503-516).

It follows that all genes related to inflammatory processes, including but not limited by cytokines, cytokine receptors and cell adhesion molecules are candidate genes for CVD.

**Glucose and energy metabolism**

As glucose and energy metabolism is interdependent with the metabolism of lipids (see above) also the former pathways contain candidate genes. Energy metabolism in general also relates to obesity, which is an independent risk factor for CVD (Melanson et al., Cardiol Rev 2001 9:202-207). In addition high blood glucose levels are associated with many microvascular and macrovascular complications and may therefore affect an individuals disposition to CVD (Duckworth, Curr Atheroscler Rep 2001, 3:383-391).

**Hypertension**

As hypertension is an independent risk factor for CVD, also genes that are involved in the regulation of systolic and diastolic blood pressure affect an individuals risk for CVD (Safar, Curr Opin Cardiol 2000, 15:258-263). Interestingly hypertension and diabetes (see above) appear to be interdependent, since hypertension is approximately twice as frequent in patients with diabetes compared with patients without the disease. Conversely, recent data suggest that hypertensive persons are more predisposed to the development of diabetes than are normotensive persons (Sowers et al., Hypertension 2001, 37:1053-1059).

**Unclassified genes**

As stated above, the mechanisms that lead to cardiovascular diseases are not completely elucidated. Hence also candidate genes were analysed, which could not be assigned to the above listed categories. The present invention is based at least in part on the discovery of polymorphisms, that lie in genomic regions of unknown physiological function.

**Results**

After conducting an association study, we surprisingly found polymorphic sites in a number of candidate genes which show a strong correlation between healthy and CVD prone individuals. "Healthy" as used herein refers to individuals that neither suffer from existing CVD, nor exihibit an increased risk for CVD through their serum lipid level profile. "CVD prone" as used herein refers to individuals with existing CVD and/or a serum lipid profile that confers a high risk to get CVD (see Table 1 for definitions of healthy and CVD prone serum lipid levels). Polymorphic sites in candidate genes that were found to be significantly associated with "healthy" or "CVD prone" phenotypes will be refered to as "CVD associated SNPs" (CA SNPs). The respective genomic loci that harbour CA SNPs will be refered to as "CVD associated genes" (CA genes), irrespective of the actual function of this gene locus.

In particular we surprisingly found CA SNPs in the following genes:

**Apolipoprotein E**

Chylomicron remnants and very low density lipoprotein (VLDL) remnants are rapidly removed from the circulation by receptor-mediated endocytosis in the liver. Apolipoprotein E, a main apoprotein of the chylomicron, binds to a specific receptor on liver cells and peripheral cells. ApoE is essential for the normal catabolism of triglyceride-rich lipoprotein constituents. The APOE gene is mapped to chromosome 19 in a cluster with APOC1 and APOC2. Defects in apolipoprotein E result in familial dysbetalipoproteinemia, or type III hyperlipoproteinemia (HLP III), in which increased plasma cholesterol and triglycerides are the consequence of impaired clearance of chylomicron and VLDL remnants.

**Apolipoprotein e receptor 2 (low density lipoprotein receptor-related protein 8, LPR8)**

This gene encodes an apolipoprotein E receptor, a member of the low density lipoprotein receptor (LDLR) family. Apolipoprotein E is a small lipophilic plasma protein and a component of lipoproteins such as chylomicron remnants, very low density lipoprotein (VLDL), and high density lipoprotein (HDL). The apolipoprotein E receptor is involved in cellular recognition and internalization of these lipoproteins. Alternative splicing generates three transcript variants for this gene; additional variants have been described, but their full length nature has not been determined.

**Diazepam binding inhibitor, Endozepine**

Diazepam binding inhibitor (acyl-CoA-binding protein); binds and induces medium-chain acyl-CoA ester synthesis

**Nonmuscle type myosin heavy chain 9**

Non-muscle myosin heavy chain 9; motor protein that provides force for muscle contraction, cytokinesis and phagocytosis; contains an ATPase head domain and a rod-like tail domain

**Apolipoprotein A-I and C-III genomic locus**

APOA1 promotes cholesterol efflux from tissues to the liver for excretion. Apolipoprotein A-I is the major protein component of high density lipoprotein (HDL) in the plasma. Synthesized in the liver and small intestine, it consists of two identical chains of 77 amino acids; an 18-amino acid signal peptide is removed co-translationally and a 6-amino acid propeptide is cleaved post-translationally. Variation in the latter step, in addition to modifications leading to so-called isoforms, is responsible for some of the polymorphism observed. APOA1 is a cofactor for lecithin cholesterolacyltransferase (LCAT) which is responsible for the formation of most plasma cholesteryl esters. The APOA1, APOC3 and APOA4 genes are closely linked in both rat and human genomes. The A-I and A-IV genes are transcribed from the same strand, while the C-III gene is transcribed convergently in relation to A-I. Defects in the apolipoprotein A-1 gene are associated with HDL deficiency and Tangier disease.

Apolipoprotein C-III is a very low density lipoprotein (VLDL) protein. APOC3 inhibits lipoprotein lipase and hepatic lipase; it is thought to delay catabolism of triglyceride-rich particles. The APOA1, APOC3 and APOA4 genes are closely linked in both rat and human genomes. The A-I and A-IV genes are transcribed from the same strand, while the A-1 and C-III genes are convergently transcribed. An increase in apoC-III levels induces the development of hypertriglyceridemia.

**Apolipoprotein B**

Apolipoprotein B (ApoB) is the main apolipoprotein of chylomicrons and low density lipoproteins (LDL). The protein occurs in the plasma in 2 main isoforms, apoB-48 and apoB-100. The first is synthesized exclusively by the gut, the second by the liver. The intestinal (B-48) and hepatic (B-100) forms of apoB are coded by a single gene and by a single mRNA transcript larger than 16 kb. The 2 proteins share a common amino terminal sequence. In the ApoB-100 isoform the precursor has 4,563 amino acids, and the mature apoB-100 has 4,536 amino acid residues. Mature, circulating B-48 is homologous over its entire length (estimated to be between 2,130 and 2,144 amino acid residues) with the amino-terminal portion of B-100 and contains no sequence from the carboxyl end of B-100. From structural studies, it is thought that apoB-48 represents the amino-terminal 47% of apoB-100 and that the carboxyl terminus of apoB-48 is in the vicinity of residue 2151 of apoB-100. Apolipoprotein B-48 may be the product of an intestinal mRNA with an in-frame UAA stop codon resulting from a C-to-U change in the codon CAA encoding Gln(2153) in apoB-100 mRNA. Since only the sequence that codes B-100 is present in genomic DNA, this presents the possibility of an organ-specific introduction of a stop codon to an mRNA and the change from CAA to UAA of codon 2153 of the message as a unique RNA editing process.

**LIM domain kinase 1**

There are approximately 40 known eukaryotic LIM proteins, so named for the LIM domains they contain. LIM domains are highly conserved cysteine-rich structures containing 2 zinc fingers. Although zinc fingers usually function by binding to DNA or RNA, the LIM motif probably mediates protein-protein interactions. LIM kinase-1 and LIM kinase-2 belong to a small subfamily with a unique combination of 2 N-terminal LIM motifs and a C-terminal protein kinase domain. LIMK1 is likely to be a component of an intracellular signaling pathway and may be involved in brain development. LIMK1 hemizygosity is implicated in the impaired visuospatial constructive cognition of Williams syndrome. Two splice variant have been identified.

**Thermostable phenol sulfotransferase (STP2)**

Sulfonation is an important pathway in the biotransformation of many drugs, xenobiotics, neurotransmitters, and steroid hormones. The phenol sulfotransferase STP2 maps to chromosome 16 (Dooley and Huang, 1996) and preferentially catalyzes the sulfonation of 'simple' planar phenols.

**Low Density Lipoprotein Receptor**

The low density lipoprotein receptor (LDLR) gene family consists of cell surface proteins involved in receptor-mediated endocytosis of specific ligands. Low density lipoprotein (LDL) is normally bound at the cell membrane and taken into the cell ending up in lysosomes where the protein is degraded and the cholesterol is made available for repression of microsomal enzyme 3-hydroxy-3-methylglutaryl coenzyme A (HMG CoA) reductase, the rate-limiting step in cholesterol synthesis. At the same time, a reciprocal stimulation of cholesterol ester synthesis takes place.
Mutations in the LDL receptor (LDLR) gene cause the autosomal dominant disorder, familial hypercholesterolemia.

**ATP-binding cassette, sub-family B (MDR/TAP), member 1**

The membrane-associated protein encoded by this gene is a member of the superfamily of ATP-binding cassette (ABC) transporters. ABC proteins transport various molecules across extra- and intra-cellular membranes. ABC genes are divided into seven distinct subfamilies (ABC1, MDR/TAP, MRP, ALD, OABP, GCN20, White). This protein is a member of the MDR/TAP subfamily. Members of the MDR/TAP subfamily are involved in multidrug resistance. The protein encoded by this gene is an ATP-dependent drug efflux pump for xenobiotic compounds with broad substrate specificity. It is responsible for decreased drug accumulation in multidrug-resistant cells and often mediates the development of resistance to anticancer drugs. This protein also functions as a transporter in the blood-brain barrier.

**Annexin VI (Calphobindin II)**

Annexin VI belongs to a family of calcium-dependent membrane and phospholipid binding proteins. Although their functions are still not clearly defined, several members of the annexin family have been implicated in membrane-related events along exocytotic and endocytotic pathways. The annexin VI gene is approximately 60 kbp long and contains 26 exons. It encodes a protein of about 68 kDa that consists of eight 68-amino acid repeats separated by linking sequences of variable lengths. It is highly similar to human annexins I and II sequences, each of which contain four such repeats. Exon 21 of annexin VI is alternatively spliced, giving rise to two isoforms that differ by a 6-amino acid insertion at the start of the seventh repeat. Annexin VI has been implicated in mediating the endosome aggregation and vesicle fusion in secreting epithelia during exocytosis.

**Protein C inhibitor (PCI-B)**

Marlar and Griffin (J. Clin. Invest. 1980, 66: 1186-1189) identified in normal plasma a protein inhibitor of activated protein C. They showed, furthermore, that this inhibitor is deficient in combined factor V and VIII deficiency. Activated protein C is a potent anticoagulant.

Protein C inhibitor (plasminogen activator inhibitor III); may be a serine protease inhibitor; member of the serpin family of serine protease inhibitors.

**NADH dehydrogenase 5**

Subunit of NADH-ubiquinone oxidoreductase (complex I); transports electrons from NADH to ubiquinone.

**Lysosomal alpha-glucosidase (acid maltase)**

This gene encodes acid alpha-glucosidase, which is essential for the degradation of glycogen to glucose in lysosomes. Different forms of acid alpha-glucosidase are obtained by proteolytic processing. Defects in this gene are the cause of glycogen storage disease II, also known as Pompe's disease, which is an autosomal recessive disorder with a broad clinical spectrum.

In classic cases of Pompe disease, affected children are prostrate and markedly hypotonic with large hearts. The tongue may be enlarged. The liver is rarely enlarged (except as a result of heart failure), and hypoglycemia and acidosis do not occur as they do in type I glycogen storage disease. Death usually occurs in the first year of life in the classic form of the disorder and cardiac involvement is striking.

**Interleukin 6**

Interleukin 6 (interferon-beta 2); induces the maturation of B cells into immunoglobulin-secreting cells.

**Fibrinogen gamma chain/fibrinogen alpha chain genes**

The protein encoded by these genes is fibrinogen, a blood-borne glycoprotein comprised of three pairs of nonidentical polypeptide chains. Following vascular injury, fibrinogen is cleaved by thrombin to form fibrin which is the most abundant component of blood clots. In addition, various cleavage products of fibrinogen and fibrin regulate cell adhesion and spreading, display vasoconstrictor and chemotactic activities, and are mitogens for several cell types. Mutations in this gene lead to several disorders, including dysfibrinogenemia, hypofibrinogenemia and thrombophilia. Alternative splicing results in two isoforms, varying in the carboxy-terminus.

**Paraoxonase 2 (PON2)**

Paraoxonase/arylesterase 2; possibly functions in protecting low density lipoprotein against oxidative modification; member of a family that hydrolyzes toxic organophosphates.

**Defensin, alpha 6**

Defensins are a family of microbicidal and cytotoxic peptides thought to be involved in host defense. They are abundant in the granules of neutrophils and also found in the epithelia of mucosal surfaces such as those of the intestine, respiratory tract, urinary tract, and vagina. Members of the defensin family are highly similar in protein sequence and distinguished by a conserved cysteine motif. Several alpha defensin genes appear to be clustered on chromosome 8. The protein encoded by this gene, defensin, alpha 6, is highly expressed in the secretory granules of Paneth cells of the small intestine, and likely plays a role in host defense of human bowel.

**Tetracycline transporter-like protein**

**Cardiac beta myosin heavy chain, myosin, heavy polypeptide 7**

MYH7 encodes the cardiac muscle beta (or slow) isoform of myosin. Changes in the relative abundance of MYH7 and MYH6 (the alpha, or fast, isoform of cardiac myosin heavy chain) correlate with the contractile velocity of cardiac muscle. Mutations in MYH7 are associated with familial hypertrophic cardiomyopathy.

**Endothelial leukocyte adhesion molecule 1 (ELAM-1)**

The endothelial leukocyte adhesion molecule-1 is expressed by cytokine-stimulated endothelial cells. It is thought to be responsible for the accumulation of blood leukocytes at sites of inflammation by mediating the adhesion of cells to the vascular lining. It exhibits structural features such as the presence of lectin- and EGF-like domains followed by short consensus repeat (SCR) domains that contain 6 conserved cysteine residues. These proteins are part of the selectin family of cell adhesion molecules. This gene is present in single copy in the human genome and contains 14 exons spanning about 13 kb of DNA. Adhesion molecules participate in the interaction between leukocytes and the endothelium and appear to be involved in the pathogenesis of atherosclerosis.

**Lamin B2 (LAMB2)**

Lamin B2 is a member of a family of structural nuclear envelope proteins.

**Estrogen receptor 2 (ER beta)**

Estrogen receptor beta 2 is a transcriptional activator involved in regulation of reproduction; exists in five isoforms.

**Tissue plasminogen activator**

This gene encodes tissue-type plasminogen activator, a secreted serine protease which converts the proenzyme plasminogen to plasmin, a fibrinolytic enzyme. Tissue-type plasminogen activator is synthesized as a single chain which is cleaved by plasmin to a two chain disulfide linked protein. This enzyme plays a role in cell migration and tissue remodeling. Increased enzymatic activity causes hyperfibrinolysis, which manifests as excessive bleeding; decreased activity leads to hypofibrinolysis which can result in thrombosis or embolism. Alternative splicing of this gene produces three transcripts.

**Laminin receptor 1**

Laminins, a family of extracellular matrix glycoproteins, are the major non-collagenous constituent of basement membranes. They have been implicated in a wide variety of biological processes including cell adhesion, differentiation, migration, signaling, neurite outgrowth and metastasis. Many of the effects of laminin are mediated through interactions with cell surface receptors. These receptors include members of the integrin family, as well as non-integrin laminin-binding proteins. This gene encodes a high-affinity, non-integrin family, laminin receptor 1. This receptor has been variously called 67 kD laminin receptor, 37 kD laminin receptor precursor (37LRP) and p40 ribosome-associated protein. The amino acid sequence of laminin receptor 1 is highly conserved through evolution, suggesting a key biological function. It has been observed that the level of the laminin receptor transcript is higher in colon carcinoma tissue and lung cancer cell line than their normal counterparts. Also, there is a correlation between the upregulation of this polypeptide in cancer cells and their invasive and metastatic phenotype. Multiple copies of this gene exist, however, most of them are pseudogenes thought to have arisen from retropositional events.

**Integrin, beta 3 (platelet glycoprotein IIIa, antigen CD61)**

The ITGB3 protein product is the integrin beta chain beta 3. Integrins are integral cell-surface proteins composed of an alpha chain and a beta chain. A given chain may combine with multiple partners resulting in different integrins. Integrin beta 3 is found along with the alpha IIb chain in platelets. Integrins are known to participate in cell adhesion as well as cell-surface mediated signalling.

**Myeloid cell differentiation protein (MCL1)**

Rinkenberger et al. (Genes Dev. 2000, 14: 23-27) disrupted the Mel1 locus in murine ES cells to determine the developmental roles of this Bcl2 family member. Deletion of Mcl1 resulted in periimplantation embryonic lethality. Homozygous Mcl1-deficient embryos did not implant in utero, but could be recovered at E3.5 to E4.0. Null blastocysts failed to hatch or attach in vitro, indicating a trophectoderm defect, although the inner cell mass could grow in culture. Of note, homozygous Mcl1-deficient blastocysts showed no evidence of increased apoptosis, but exhibited a delay in maturation beyond the precompaction stage. This model indicates that Mcl1 is essential for preimplantation development and implantation, and suggests that it has a function beyond regulating apoptosis.

**Tumor necrosis factor type 1 receptor associated protein (TRAP1)**

TRAP is a highly conserved member of a class of molecular chaperones with multiple functions. It is involved in the maturation of a subset of proteins involved in signal transduction.

**Xanthine dehydrogenase/oxidase**

Xanthine dehydrogenase belongs to the group of molybdenum-containing hydroxylases involved in the oxidative metabolism of purines. The enzyme is a homodimer. Xanthine dehydrogenase can be converted to xanthine oxidase by reversible sulfhydryl oxidation or by irreversible proteolytic modification. Defects in xanthine dehydrogenase cause xanthinuria, may contribute to adult respiratory stress syndrome, and may potentiate influenza infection through an oxygen metabolite-dependent mechanism.

**Stromal cell-derived factor 1 (SDF 1)**

Stromal cell-derived factor 1 is a lymphocyte chemoattractant that signals through the receptor CXCR4.

**Diacylglycerol kinase delta**

Diacylglycerol kinase delta phosphorylates the arachidonoyl type of diacylglycerol; contains a pleckstrin homology domain and an EPH domain.

**Smooth muscle myosin heavy chain, MYH11**

The protein encoded by this gene is a smooth muscle myosin belonging to the myosin heavy chain family. The gene product is a subunit of a hexameric protein that consists of 2 heavy chain subunits and 2 pairs of non-identical light chain subunits. It functions as a major contractile protein, converting chemical energy into mechanical energy through the hydrolysis of ATP. The gene encoding a human ortholog of rat NUDE1 is transcribed from the reverse strand of MYH11 gene, and its 3' end overlaps with that of the latter. The pericentric inversion of chromosome 16 [inv(16)(p13q22)] produces a chimeric transcript consisting of the first 165 residues from the N terminus of core-binding factor beta in a fusion with the C-terminal portion of the smooth muscle myosin heavy chain. This chromosomal rearrangement is associated with acute myeloid leukemia of the M4Eo subtype. Alternative splicing generates isoforms that are differentially expressed, with ratios changing during muscle cell maturation. Additional splice variants have been described but their full-length nature has not been determined.

**Caveolin 1**

The scaffolding protein encoded by this gene is the main component of the caveolae plasma membranes found in most cell types. The protein links integrin subunits to the tyrosine kinase FYN, an initiating step in coupling integrins to the Ras-ERK pathway and promoting cell cycle progression. The gene is a tumor suppressor gene candidate and a negative regulator of the Ras-p42/44 MAP kinase cascade. CAV1 and CAV2 are located next to each other on chromosome 7 and express colocalizing proteins that form a stable hetero-oligomeric complex. By using alternative initiation codons in the same reading frame, two isoforms (alpha and beta) are encoded by a single transcript from this gene.

**AP-2 beta**

Expression of AP-2 transcription factors has been detected previously in embryonic renal tissues. It was shown that AP-2beta -/- mice complete embryonic development and die at postnatal days 1 and 2 because of polycystic kidney disease. Analyses of kidney development revealed that induction of epithelial conversion, mesenchyme condensation, and further glomerular and tubular differentiation occur normally in AP-2beta-deficient mice. At the end of embryonic development expression of bcl-X(L), bcl-w, and bcl-2 is down-regulated in parallel to massive apoptotic death of collecting duct and distal tubular epithelia. Addressing the molecular mechanism it was shown that transfection of AP-2 into cell lines in vitro strongly suppresses c-myc-induced apoptosis pointing to a function of AP-2 in programming cell survival during embryogenesis. The position of the human AP-2beta gene was identified at chromosome 6p12-p21.1, within a region that has been mapped for autosomal recessive polycystic kidney disease (ARPKD). Sequence analyses of ARPKD patients and linkage analyses using intragenic polymorphic markers indicate that the AP-2beta gene is located in close proximity to but distinct from the ARPKD gene (Moser et al., Genes Dev 1997, 11:1938-1948).

**Transforming growth factor beta 2 (TGFB2)**

Transforming growth factor-beta 2 (glioblastoma-derived T cell suppressor factor); suppresses IL2 - dependent growth of T cells and is a member of a family of cytokines that transmits signals through transmembrane serine/threonine kinases.

**Tumor necrosis factor, alpha-induced protein 2 (TNFAIP2, B94)**

Secreted by vascular endothelium, expression is induced by tumor necrosis factor alpha, interleukin-1 beta, and lipopolysaccharide.

**Protein phosphatase 1A (formerly 2C), magnesium-dependent, alpha isoform**

Magnesium- or manganese-dependent alpha protein phosphatase 1A; regulates cell stress responses.

**ATPase, Ca++ transporting, plasma membrane 3 (PMCA3)**

**Osteonidogen (nidogen 2)**

**ATP-binding cassette, sub-family A (ABC1), member 1**

The membrane-associated protein encoded by this gene is a member of the superfamily of ATP-binding cassette (ABC) transporters. ABC proteins transport various molecules across extra- and intracellular membranes. ABC genes are divided into seven distinct subfamilies (ABC1, MDR/TAP, MRP, ALD, OABP, GCN20, White). This protein is a member of the ABC1 subfamily. Members of the ABC1 subfamily comprise the only major ABC subfamily found exclusively in multicellular eukaryotes. With cholesterol as its substrate, this protein functions as a cholesteral efflux pump in the cellular lipid removal pathway. Mutations in this gene have been associated with Tangier's disease and familial high-density lipoprotein deficiency.

**Bromodomain-containing 3 (BRD3)**

This gene was identified based on its homology to the gene encoding the RING3 protein, a serine/threonine kinase. The gene localizes to 9q34, a region which contains several major histocompatibility complex (MHC) genes. The function of the encoded protein is not known.

**Peroxisome proliferator activated receptor-gamma (PPAR gamma)**

Peroxisome prolifetator activated receptor-gamma (PPAR gamma) regulates adipocyte and macrophage gene expression and differentiation.

**Tumor necrosis factor beta (Lymphotoxin alpha, LTA)**

Lymphotoxin alpha, a member of the tumor necrosis factor family, is a cytokine produced by lymphocytes. LTA is highly inducible, secreted, and exists as homotrimeric molecule. LTA forms heterotrimers with lymphotoxin-beta which anchors lymphotoxin-alpha to the cell surface. LTA mediates a large variety of inflammatory, immunostimulatory, and antiviral responses. LTA is also involved in the formation of secondary lymphoid organs during development and plays a role in apoptosis.

**DEK oncogene**

Site-specific DNA binding protein; involved in transcriptional regulation and signal transduction.

**S-adenosyl methionine transferase**

S-adenosyl methionine transferase catalyzes the formation of S-adenosylmethionine from methionine and ATP. Both the beta and alpha isoforms may be encoded by one gene. Methionine adenosyltransferase deficiency is known to be caused by recessive as well as dominant mutations, the latter identified in autosomal dominant persistant hypermethioninemia.

**Coagulation factor III (thromboplastin, tissue factor)**

This gene encodes coagulation factor III which is a cell surface glycoprotein. This factor enables cells to initiate the blood coagulation cascades, and it functions as the high-affinity receptor for the coagulation factor VII. The resulting complex provides a catalytic event that is responsible for initiation of the coagulation protease cascades by specific limited proteolysis. Unlike the other cofactors of these protease cascades, which circulate as nonfunctional precursors, this factor is a potent initiator that is fully functional when expressed on cell surfaces. There are 3 distinct domains of this factor: extracellular, transmembrane, and cytoplasmic. This protein is the only one in the coagulation pathway for which a congenital deficiency has not been described.

**Membrane protein palmitoylated 2 (MPP2)**

Palmitoylated membrane protein 2 is a member of a family of membrane-associated proteins termed MAGUKs (membrane-associated guanylate kinase homologs). MAGUKs interact with the cytoskeleton and regulate cell proliferation, signaling pathways, and intracellular junctions. Palmitoylated membrane protein 2 contains a conserved sequence, called the SH3 (src homology 3) motif, found in several other proteins that associate with the cytoskeleton and are suspected to play important roles in signal transduction.

**Granulocyte-macrophage colony stimulating factor 2 receptor, beta chain (CSF2RB)**

CSF2RB is a common beta chain of the high affinity receptor for IL-3, IL-5 and CSF. Defective CSF2RB has been reported to be associated with protein alveolar proteinosis

**Coagulation factor II (Prothrombin, F2)**

Coagulation factor II is proteolytically cleaved to form thrombin in the first step of the coagulation cascade which ultimately results in the stemming of blood loss. F2 also plays a role in maintaining vascular integrity during development and postnatal life. Mutations in F2 leads to various forms of thrombosis and dysprothrombinemia.

**Lipoprotein lipase (LPL)**

LPL encodes lipoprotein lipase, which is expressed in heart, muscle, and adipose tissue. LPL functions as a homodimer, and has the dual functions of triglyceride hydrolase and ligand/bridging factor for receptor-mediated lipoprotein uptake. Severe mutations that cause LPL deficiency result in type I hyperlipoproteinemia, while less extreme mutations in LPL are linked to many disorders of lipoprotein metabolism.

**activin beta-C chain (Inhibin beta C)**

This gene encodes the beta C chain of inhibin, a member of the TGF-beta superfamily. This subunit forms heterodimers with beta A and beta B subunits. Inhibins and activins, also members of the TGF-beta superfamily, are hormones with opposing actions and are involved in hypothalamic, pituitary, and gonadal hormone secretion, as well as growth and differentiation of various cell types.

**Methylenetetrahydrofolate dehydrogenase, NADP+ dependent (MTHFD)**

This gene encodes a protein that possesses three distinct enzymatic activities, 5,10-methylenetetrahydrofolate dehydrogenase, 5,10-methenyltetrahydrofolate cyclohydrolase and 10-formyltetrahydrofolate synthetase. Each of these activities catalyzes one of three sequential reactions in the interconversion of 1-carbon derivatives of tetrahydrofolate, which are substrates for methionine, thymidylate, and de novo purine syntheses. The trifunctional enzymatic activities are conferred by two major domains, an aminoterminal portion containing the dehydrogenase and cyclohydrolase activities and a larger synthetase domain.

**PACAP gene for pituitary adenylate cyclase activating polypeptide**

This gene encodes adenylate cyclase activating polypeptide 1. Mediated by adenylate cyclase activating polypeptide 1 receptors, this polypeptide stimulates adenylate cyclase and subsequently increases the cAMP level in target cells. Adenylate cyclase activating polypeptide 1 is not only a hypophysiotropic hormone, but also functions as a neurotransmitter and neuromodulator. In addition, it plays a role in paracrine and autocrine regulation of certain types of cells. This gene is composed of five exons. Exons 1 and 2 encode the 5' UTR and signal peptide, respectively; exon 4 encodes an adenylate cyclase activating polypeptide 1-related peptide; and exon 5 encodes the mature peptide and 3' UTR. This gene encodes three different mature peptides, including two isotypes: a shorter form and a longer form.

**Cytochrome P450, subfamily IIC (mephenytoin 4-hydroxylase), polypeptide 8**

This gene encodes a member of the cytochrome P450 superfamily of enzymes. The cytochrome P450 proteins are monooxygenases which catalyze many reactions involved in drug metabolism and synthesis of cholesterol, steroids and other lipids. This protein localizes to the endoplasmic reticulum and its expression is induced by phenobarbital. The enzyme is known to metabolize many xenobiotics, including the anticonvulsive drug mephenytoin, benzo(a)pyrene, 7-ethyoxycoumarin, and the anticancer drug taxol. Two transcript variants for this gene have been described; it is thought that the longer form does not encode an active cytochrome P450 since its protein product lacks the heme binding site. This gene is located within a cluster of cytochrome P450 genes on chromosome 10q24.

**Leucocyte adhesion receptor, L-selectin (SELL)**

SELL is a cell surface component that is a member of a family of adhesion/homing receptors which play important roles in leukocyte-endothelial cell interactions. The molecule is composed of multiple domains: one homologous to lectins, one to epidermal growth factor, and two to the consensus repeat units found in C3/C4 binding proteins.

**Mitochondrial ATP synthase c subunit (P1 form)**

Isoform 1 (P1) of subunit c, H+-translocating subunit of F0 ATP synthase; catalyzes the synthesis of ATP during oxidative phosphorylation.

**Calmodulin**

Calmodulin binds Ca2+, regulates proteins and enzymes in a Ca2+-dependent manner

**WNT1 inducible signaling pathway protein 1 (WISP1) gene**

WISP1 is a member of the connective tissue growth factor family

**Ribophorin I**

Ribophorin I; subunit of oligosaccharyltransferase that binds ribosomes.

**Nonsyndromic hearing impairment protein (DFNA5)**

Hearing impairment is a heterogeneous condition with over 40 loci described. The protein encoded by this gene is expressed in fetal cochlea, however, its function is not known. Nonsyndromic hearing impairment is associated with a mutation in this gene.

**Cytochrome P450 2E1 (CYP2E1)**

This gene encodes a member of the cytochrome P450 superfamily of enzymes. The cytochrome P450 proteins are monooxygenases which catalyze many reactions involved in drug metabolism and synthesis of cholesterol, steroids and other lipids.

This protein localizes to the endoplasmic reticulum and is induced by ethanol, the diabetic state, and starvation. The enzyme metabolizes both endogenous substrates, such as ethanol, acetone, and acetal, as well as exogenous substrates including benzene, carbon tetrachloride, ethylene glycol, and nitrosamines which are premutagens found in cigarette smoke. Due to its many substrates, this enzyme may be involved in such varied processes as gluconeogenesis, hepatic cirrhosis, diabetes, and cancer.

**Vacuolar H+ ATPase E subunit (ATP6E)**

Subunit E of the vacuolar H+-ATPase proton pump; regulates ATP binding and hydrolysis by the A and B subunits

**Retinoid X receptor, alpha**

Retinoid X receptors (RXRs) and retinoic acid receptors (RARs), are nuclear receptors that mediate the biological effects of retinoids by their involvement in retinoic acid-mediated gene activation. These receptors exert their action by binding, as homodimers or heterodimers, to specific sequences in the promoters of target genes and regulating their transcription. The protein encoded by this gene is a member of the steroid and thyroid hormone receptor superfamily of transcriptional regulators.

**Peroxisome proliferative activated receptor, delta (PPARD)**

Peroxisome proliferator-activated receptor delta is a member of the steroid hormone receptor superfamily

**Ataxin (SCA1)**

The autosomal dominant cerebellar ataxias (ADCA) are a heterogeneous group of neurodegenerative disorders characterized by progressive degeneration of the cerebellum, brain stem and spinal cord. Clinically, ADCA has been divided into three groups: ADCA types I-III. ADCAI is genetically heterogeneous, with five genetic loci, designated spinocerebellar ataxia (SCA) 1, 2, 3, 4 and 6, being assigned to five different chromosomes. ADCAII, which always presents with retinal degeneration (SCA7), and ADCAIII often referred to as the 'pure' cerebellar syndrome (SCA5), are most likely homogeneous disorders. Several SCA genes have been cloned and shown to contain CAG repeats in their coding regions. ADCA is caused by the expansion of the CAG repeats, producing an elongated polyglutamine tract in the corresponding protein. The expanded repeats are variable in size and unstable, usually increasing in size when transmitted to successive generations. The function of the ataxins is not known. The SCA1 locus has been mapped to chromosome 6, and it has been determined that the diseased allele contains 41-81 CAG repeats, compared to 6-39 in the normal allele. Several transcript variants of SCA1 in the 5' UTR have been described; however, their full-length nature is not known.

**Adducin 1 (alpha)**

Adducins are a family of cytoskeleton proteins encoded by three genes (alpha, beta, gamma). Alpha- and gamma-adducins are ubiquitously expressed. In contrast, beta-adducin is expressed at high levels in brain and hematopoietic tissues. Adducin is a heterodimeric protein that consists of related subunits, alpha and beta, which are produced from distinct genes but share a similar structure. Alpha- and beta-adducin include a protease-resistant N-terminal region and a protease-sensitive, hydrophilic C-terminal region. Adducin binds with high affinity to Ca(2+)/calmodulin and is a substrate for protein kinases A and C.

**Zinc finger protein 202 (ZNF202)**

Zinc-finger protein 202; may repress genes involved in lipid metabolism; contains zinc fingers

**Mevalonate pyrophosphate decarboxylase**

The enzyme mevalonate pyrophosphate decarboxylase catalyzes the conversion of mevalonate pyrophosphate into isopentenyl pyrophosphate in one of the early steps in cholesterol biosynthesis. It decarboxylates and dehydrates its substrate while hydrolyzing ATP.

**Integrin, beta 2 (ITGB2, lymphocyte function-associated antigen 1, LFA1)**

The ITGB2 protein product is the integrin beta chain beta 2. Integrins are integral cell-surface proteins composed of an alpha chain and a beta chain. A given chain may combine with multiple partners resulting in different integrins. For example, beta 2 combines with the alpha L chain to form the integrin LFA-1, and combines with the alpha M chain to form the integrin Mac-1. Integrins are known to participate in cell adhesion as well as cell-surface mediated signalling.

**Adrenergic beta-3- receptor (ADRB3)**

The ADRB3 gene product, beta-3-adrenergic receptor, is located mainly in adipose tissue and is involved in the regulation of lipolysis and thermogenesis. Beta adrenergic receptors are involved in the epenephrine and norepinephrine-induced activation of adenylate cyclase through the action of G proteins.

**Cytochrome P450, subfamily IVF, polypeptide 8**

This gene, CYP4F8, encodes a member of the cytochrome P450 superfamily of enzymes. The cytochrome P450 proteins are monooxygenases which catalyze many reactions involved in drug metabolism and synthesis of cholesterol, steroids and other lipids. This protein localizes to the endoplasmic reticulum and functions as a 19-hydroxylase of prostaglandins in seminal vesicles. This gene is part of a cluster of cytochrome P450 genes on chromosome 19. Another member of this family, CYP4F3, is approximately 18 kb away.

**Coagulation factor XIII, A subunit**

This gene encodes the coagulation factor XIII A subunit. Coagulation factor XIII is the last zymogen to become activated in the blood coagulation cascade. Plasma factor XIII is a heterotetramer composed of 2 A subunits and 2 B subunits. The A subunits have catalytic function, and the B subunits do not have enzymatic activity and may serve as a plasma carrier molecules. Platelet factor XIII is comprised only of 2 A subunits, which are identical to those of plasma origin. Upon activation by the cleavage of the activation peptide by thrombin and in the presence of calcium ion, the plasma factor XIII dissociates its B subunits and yields the same active enzyme, factor XIIIa, as platelet factor XIII. This enzyme acts as a transglutaminase to catalyze the formation of gamma-glutamyl-epsilon-lysine crosslinking between fibrin molecules, thus stabilizing the fibrin clot. It also crosslinks alpha-2-plasmin inhibitor, or fibronectin, to the alpha chains of fibrin. Factor XIII deficiency is classified into two categories: type I deficiency, characterized by the lack of both the A and B subunits; and type II deficiency, characterized by the lack of the A subunit alone. These defects can result in a lifelong bleeding tendency, defective wound healing, and habitual abortion.

**Beta adaptin**

The beta adaptin subunit is part of the clathrin coat assembly complex which links clathrin to receptors in coated pits and vesicles. These vesicles are involved in endocytosis and Golgi processing. The beta 1 subunit is one of the assembly proteins which binds to clathrin and initiates coat formation.

**Transforming growth factor-beta 1 (TGFB1)**

Transforming growth factor-beta 1; regulates cell proliferation, differentiation, and apoptosis

**Transforming growth factor-beta 3 (TGFB3)**

Transforming growth factor-beta 3; transmits signals through transmembrane serine/threonine kinases, may be required for normal development of the lung and palate; member of family of cytokines, very strongly similar to murine Tgfb3.

**Flavin containing monooxygenase 1 (FMO1)**

Metabolic N-oxidation of the diet-derived amino-trimethylamine (TMA) is mediated by flavin-containing monooxygenase and is subject to an inherited FMO3 polymorphism in man resulting in a small subpopulation with reduced TMA N-oxidation capacity resulting in fish odor syndrome Trimethylaminuria. Three forms of the enzyme, FMO1 found in fetal liver, FMO2 found in adult liver, and FMO3 are encoded by genes clustered in the 1q23-q25 region. Flavin-containing monooxygenases are NADPH-dependent flavoenzymes that catalyzes the oxidation of soft nucleophilic heteroatom centers in drugs, pesticides, and xenobiotics.

**Coagulation factor IX (F9)**

This gene encodes vitamin K-dependent coagulation factor IX that circulates in the blood as an inactive zymogen. This factor is converted to an active form by factor XIa, which excises the activation peptide and thus generates a heavy chain and a light chain held together by one or more disulfide bonds. The role of this activated factor IX in the blood coagulation cascade is to activate factor X to its active form through interactions with Ca+2 ions, membrane phospholipids, and factor VIII. Alterations of this gene, including point mutations, insertions and deletions, cause factor IX deficiency, which is a recessive X-linked disorder, also called hemophilia B or Christmas disease.

**Apical protein, Xenopus laevis-like (APXL)**

May be an amiloride-sensitive sodium channel; similar to Xenopus laevis Apical Protein

As SNPs are linked to other SNPs in neighboring genes on a chromosome (Linkage Disequilibrium) those SNPs could also be used as marker SNPs. In a recent publication it was shown that SNPs are linked over 100 kb in some cases more than 150 kb (Reich D.E. et al. Nature 411, 199-204, 2001). Hence SNPs lying in regions neighbouring CA SNPs could be linked to the latter and by this being a CVD marker. These associations could be performed as described for the gene polymorphism in methods.

**Definitions**

For convenience, the meaning of certain terms and phrases employed in the specification, examples, and appended claims are provided below. Moreover, the definitions by itself are intended to explain a further background of the invention.

The term "allele", which is used interchangeably herein with "allelic variant" refers to alternative forms of a gene or portions thereof. Alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides. An allele of a gene can also be a form of a gene containing a mutation.

The term "allelic variant of a polymorphic region of a gene" refers to a region of a gene having one of several nucleotide sequences found in that region of the gene in other individuals.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, though preferably less than 25% identity, with one of the sequences of the present invention.

The term "a homologue of a nucleic acid" refers to a nucleic acid having a nucleotide sequence having a certain degree of homology with the nucleotide sequence of the nucleic acid or complement thereof. A homologue of a double stranded nucleic acid having SEQ ID NO. X is intended to include nucleic acids having a nucleotide sequence which has a certain degree of homology with SEQ ID NO. X or with the complement thereof. Preferred homologous of nucleic acids are capable of hybridizing to the nucleic acid or complement thereof.

The term "interact" as used herein is meant to include detectable interactions between molecules, such as can be detected using, for example, a hybridization assay.

The term interact is also meant to include "binding" interactions between molecules. Interactions may be, for example, protein-protein, protein-nucleic acid, protein-small molecule or small molecule-nucleic acid in nature.

The term "intronic sequence" or "intronic nucleotide sequence" refers to the nucleotide sequence of an intron or portion thereof.

The term "isolated" as used herein with respect to nucleic acids, such as DNA or RNA, refers to molecules separated from other DNAs or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized.

Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

The term "lipid" shall refer to a fat or fat-like substance that is insoluble in polar solvents such as water. The term "lipid" is intended to include true fats (e.g. esters of fatty acids and glycerol); lipids (phospholipids, cerebrosides, waxes); sterols (cholesterol, ergosterol) and lipoproteins (e.g. HDL, LDL and VLDL).

The term "locus" refers to a specific position in a chromosome. For example, a locus of a gene refers to the chromosomal position of the gene.

The term "modulation" as used herein refers to both up-regulation, (i.e., activation or stimulation), for example by agonizing, and down-regulation (i.e. inhibition or suppression), for example by antagonizing of a bioactivity (e.g. expression of a gene).

The term "molecular structure" of a gene or a portion thereof refers to the structure as defined by the nucleotide content (including deletions, substitutions, additions of one or more nucleotides), the nucleotide sequence, the state of methylation, and/or any other modification of the gene or portion thereof.

The term "mutated gene" refers to an allelic form of a gene, which is capable of altering the phenotype of a subject having the mutated gene relative to a subject which does not have the mutated gene. If a subject must be homozygous for this mutation to have an altered phenotype, the mutation is said to be recessive. If one copy of the mutated gene is sufficient to alter the genotype of the subject, the mutation is said to be dominant. If a subject has one copy of the mutated gene and has a phenotype that is intermediate between that of a homozygous and that of a heterozygous (for that gene) subject, the mutation is said to be co-dominant.

As used herein, the term "nucleic acid" refers to polynucleotides such as deoxyribonucleic acid (DNA), and, where appropriate, ribonucleic acid (RNA). The term should also be understood to include, as equivalents, derivatives, variants and analogs of either RNA or DNA made from nucleotide analogs, including peptide nucleic acids (PNA), morpholino oligonucleotides (J. Summerton and D. Weller, Antisense and Nucleic Acid Drug Development 7:187 (1997)) and, as applicable to the embodiment being described, single (sense or antisense) and double-stranded polynucleotides. Deoxyribonucleotides include deoxyadenosine, deoxycytidine, deoxyguanosine, and deoxythymidine. For purposes of clarity, when referring herein to a nucleotide of a nucleic acid, which can be DNA or an RNA, the term "adenosine", "cytidine", "guanosine", and "thymidine" are used. It is understood that if the nucleic acid is RNA, a nucleotide having a uracil base is uridine.

The term "nucleotide sequence complementary to the nucleotide sequence set forth in SEQ ID NO. x" refers to the nucleotide sequence of the complementary strand of a nucleic acid strand having SEQ ID NO. x. The term "complementary strand" is used herein interchangeably with the term "complement". The complement of a nucleic acid strand can be the complement of a coding strand or the complement of a non-coding strand. When referring to double stranded nucleic acids, the complement of a nucleic acid having SEQ ID NO. x refers to the complementary strand of the strand having SEQ ID NO. x or to any nucleic acid having the nucleotide sequence of the complementary strand of SEQ ID NO. x. When referring to a single stranded nucleic acid having the nucleotide sequence SEQ ID NO. x, the complement of this nucleic acid is a nucleic acid having a nucleotide sequence which is complementary to that of SEQ ID NO. x. The nucleotide sequences and complementary sequences thereof are always given in the 5' to 3' direction. The term "complement" and "reverse complement" are used interchangeably herein.

The term "operably linked" is intended to mean that the promoter is associated with the nucleic acid in such a manner as to facilitate transcription of the nucleic acid.

The term "polymorphism" refers to the coexistence of more than one form of a gene or portion thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

To describe a "polymorphic site" in a nucleotide sequence often there is used an "ambiguity code" that stands for the possible variations of nucleotides in one site. The list of ambiguity codes is summarized in the following table:

| Ambiguity Codes (IUPAC Nomenclature) | |
|---|---|
| B | c/g/t |
| D | a/g/t |
| H | a/c/t |
| K | g/t |
| M | a/c |
| N | a/c/g/t |
| R | a/g |

(continued)

| Ambiguity Codes (IUPAC Nomenclature) | |
|---|---|
| S | c/g |
| V | a/c/g |
| W | a/t |
| Y | c/t |

So, for example, a "R" in a nucleotide sequence means that either an "a" or a "g" could be at that position.

The terms "protein", "polypeptide" and "peptide" are used interchangeably herein when referring to a gene product.

A "regulatory element", also termed herein "regulatory sequence is intended to include elements which are capable of modulating transcription from a basic promoter and include elements such as enhancers and silencers. The term "enhancer", also referred to herein as "enhancer element", is intended to include regulatory elements capable of increasing, stimulating, or enhancing transcription from a basic promoter. The term "silencer", also referred to herein as "silencer element" is intended to include regulatory elements capable of decreasing, inhibiting, or repressing transcription from a basic promoter. Regulatory elements are typically present in 5' flanking regions of genes. However, regulatory elements have also been shown to be present in other regions of a gene, in particular in introns. Thus, it is possible that genes have regulatory elements located in introns, exons, coding regions, and 3' flanking sequences. Such regulatory elements are also intended to be encompassed by the present invention and can be identified by any of the assays that can be used to identify regulatory elements in 5' flanking regions of genes.

The term "regulatory element" further encompasses "tissue specific" regulatory elements, i.e., regulatory elements which effect expression of the selected DNA sequence preferentially in specific cells (e.g., cells of a specific tissue). gene expression occurs preferentially in a specific cell if expression in this cell type is significantly higher than expression in other cell types. The term "regulatory element" also encompasses non-tissue specific regulatory elements, i.e., regulatory elements which are active in most cell types. Furthermore, a regulatory element can be a constitutive regulatory element, i.e., a regulatory element which constitutively regulates transcription, as opposed to a regulatory element which is inducible, i.e., a regulatory element which is active primarily in response to a stimulus. A stimulus can be, e.g., a molecule, such as a hormone, cytokine, heavy metal, phorbol ester, cyclic AMP (cAMP), or retinoic acid.

Regulatory elements are typically bound by proteins, e.g., transcription factors. The term "transcription factor" is intended to include proteins or modified forms thereof, which interact preferentially with specific nucleic acid sequences, i.e., regulatory elements, and which in appropriate conditions stimulate or repress transcription. Some transcription factors are active when they are in the form of a monomer. Alternatively, other transcription factors are active in the form of a dimer consisting of two identical proteins or different proteins (heterodimer). Modified forms of transcription factors are intended to refer to transcription factors having a post-translational modification, such as the attachment of a phosphate group. The activity of a transcription factor is frequently modulated by a post-translational modification. For example, certain transcription factors are active only if they are phosphorylated on specific residues. Alternatively, transcription factors can be active in the absence of phosphorylated residues and become inactivated by phosphorylation. A list of known transcription factors and their DNA binding site can be found, e.g., in public databases, e.g., TFMATRIX Transcription Factor Binding Site Profile database.

As used herein, the term "specifically hybridizes" or "specifically detects" refers to the ability of a nucleic acid molecule of the invention to hybridize to at least approximately 6, 12, 20, 30, 40, 50, 60, 70, 80, 90, 100, 110, 120, 130 or 140 consecutive nucleotides of either strand of a gene.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

"Candidate gene" as used herein includes genes that can be assigned to either normal cardiovascular function or to metabolic pathways that are related to onset and/or progression of cardiovascular diseases. As the development of cardiovascular diseases is not completely understood, the term "candidate gene" may also comprise genes with presently unknown function.

"CA SNP" (cardiovascular disease associated SNP) refers to a polymorphic site which shows a significant association with the risk to develop cardiovascular diseases.

"CA gene" (cardiovascular disease associated gene) refers to a genomic locus harbouring a CA SNP, irrespective of the actual function of this gene locus.

CA gene polypeptide refers to a polypeptide encoded at least in part by a CA gene

Methods for Assessing Cardiovascular Status

The present invention provides diagnostic methods for assessing cardiovascular status in a human individual. Cardiovascular status as used herein refers to the physiological status of an individual's cardiovascular system as reflected in one or more markers or indicators. Status markers include without limitation clinical measurements such as, e.g., blood pressure, electrocardiographic profile, and differentiated blood flow analysis as well as measurements of LDL- and HDL-Cholesterol levels, other lipids and other well established clinical parameters that are standard in the art. Status markers according to the invention include diagnoses of one or more cardiovascular syndromes, such as, e.g., hypertension, acute myocardial infarction, silent myocardial infarction, stroke, and atherosclerosis. It will be understood that a diagnosis of a cardiovascular syndrome made by a medical practitioner encompasses clinical measurements and medical judgement. Status markers according to the invention are assessed using conventional methods well known in the art. Also included in the evaluation of cardiovascular status are quantitative or qualitative changes in status markers with time, such as would be used, e.g., in the determination of an individual's response to a particular therapeutic regimen.

The methods are carried out by the steps of:

(i) determining the sequence of one or more polymorphic positions within one, sseveral or all of the genes listed in Examples or other genes mentioned in this file in the individual to establish a polymorphic pattern for the individual; and

(ii) comparing the polymorphic pattern established in (i) with the polymorphic patterns of humans exhibiting different markers of cardiovascular status. The polymorphic pattern of the individual is, preferably, highly similar and, most preferably, identical to the polymorphic pattern of individuals who exhibit particular status markers, cardiovascular syndromes, and/or particular patterns of response to therapeutic interventions. Polymorphic patterns may also include polymorphic positions in other genes which are shown, in combination with one or more polymorphic positions in the genes listed in the Examples, to correlate with the presence of particular status markers. In one embodiment, the method involves comparing an individual's polymorphic pattern with polymorphic patterns of individuals who have been shown to respond positively or negatively to a particular therapeutic regimen. Therapeutic regimen as used herein refers to treatments aimed at the elimination or amelioration of symptoms and events associated cardiovascular disease. Such treatments include without limitation one or more of alteration in diet, lifestyle, and exercise regimen; invasive and noninvasive surgical techniques such as atherectomy, angioplasty, and coronary bypass surgery; and pharmaceutical interventions, such as administration of ACE inhibitors; angiotensin II receptor antagonists, diuretics, alpha-adrenoreceptor antagonists, cardiac glycosides, phosphodiesterase inhibitors, beta-adrenoreceptor antagonists, calcium channel blockers, HMG-CoA reductase inhibitors, imidazoline receptor blockers, endothelin receptor blockers, organic nitrites, and modulators of protein function of genes listed in the Examples. Interventions with pharmaceutical agents not yet known whose activity correlates with particular polymorphic patterns associated with cardiovascular disease are also encompassed. It is contemplated, for example, that patients who are candidates for a particular therapeutic regimen will be screened for polymorphic patterns that correlate with responsivity to that particular regimen.

In a preferred embodiment, the method involves comparing an individual's polymorphic pattern with polymorphic patterns of individuals who exhibit or have exhibited one or more markers of cardiovascular disease, such as, e.g., elevated LDL-Cholesterol levels, high blood pressure, abnormal electrocardiographic profile, myocardial infarction, stroke, or atherosclerosis.

In practicing the methods of the invention, an individual's polymorphic pattern can be established by obtaining DNA from the individual and determining the sequence at predetermined polymorphic positions in the genes such as those described in this file.

The DNA may be obtained from any cell source. Non-limiting examples of cell sources available in clinical practice include blood cells, buccal cells, cervicovaginal cells, epithelial cells from urine, fetal cells, or any cells present in tissue obtained by biopsy. Cells may also be obtained from body fluids, including without limitation blood, saliva, sweat, urine, cerebrospinal fluid, feces, and tissue exudates at the site of infection or inflammation. DNA is extracted from the cell source or body fluid using any of the numerous methods that are standard in the art. It will be understood that the particular method used to extract DNA will depend on the nature of the source.

**Diagnostic and Prognostic Assays**

The present invention provides methods for determining the molecular structure of at least one polymorphic region of a gene, specific allelic variants of said polymorphic region being associated with cardiovascular disease. In one embodiment, determining the molecular structure of a polymorphic region of a gene comprises determining the identity of the allelic variant. A polymorphic region of a gene, of which specific alleles are associated with cardiovascular disease can be located in an exon, an intron, at an intron/exon border, or in the promoter of the gene.

The invention provides methods for determining whether a subject has, or is at risk, of developing a cardiovascular disease. Such disorders can be associated with an aberrant gene activity, e.g., abnormal binding to a form of a lipid, or an aberrant gene protein level. An aberrant gene protein level can result from an aberrant transcription or post-transcriptional regulation. Thus, allelic differences in specific regions of a gene can result in differences of gene protein due to differences in regulation of expression. In particular, some of the identified polymorphisms in the human gene may be associated with differences in the level of transcription, RNA maturation, splicing, or translation of the gene or transcription product.

In preferred embodiments, the methods of the invention can be characterized as comprising detecting, in a sample of cells from the subject, the presence or absence of a specific allelic variant of one or more polymorphic regions of a gene. The allelic differences can be: (i) a difference in the identity of at least one nucleotide or (ii) a difference in the number of nucleotides, which difference can be a single nucleotide or several nucleotides.

A preferred detection method is allele specific hybridization using probes overlapping the polymorphic site and having about 5, 10, 20, 25, or 30 nucleotides around the polymorphic region. Examples of probes for detecting specific allelic variants of the polymorphic region located in intron X are probes comprising a nucleotide sequence set forth in any of SEQ ID NO. X. In a preferred embodiment of the invention, several probes capable of hybridizing specifically to allelic variants are attached to a solid phase support, e.g., a "chip". Oligonucleotides can be bound to a solid support by a variety of processes, including lithography. For example a chip can hold up to 250,000 oligonucleotides (GeneChip, Affymetrix). Mutation detection analysis using these chips comprising oligonucleotides, also termed "DNA probe arrays" is described e.g., in Cronin et al. (1996) Human Mutation 7:244 and in Kozal et al. (1996) Nature Medicine 2:753. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment. For example, the identity of the allelic variant of the nucleotide polymorphism of nucleotide T or G at position 140 of Seq ID 1 (baySNP10948) and that of other possible polymorphic regions can be determined in a single hybridization experiment.

In other detection methods, it is necessary to first amplify at least a portion of a gene prior to identifying the allelic variant. Amplification can be performed, e.g., by PCR and/or LCR, according to methods known in the art. In one embodiment, genomic DNA of a cell is exposed to two PCR primers and amplification for a number of cycles sufficient to produce the required amount of amplified DNA. In preferred embodiments, the primers are located between 40 and 350 base pairs apart. Preferred primers for amplifying gene fragments of genes of this file are listed in Table 2 in the Examples.

Alternative amplification methods include: self sustained sequence replication (Guatelli, J. C. et al., 1990, Proc. Natl. Acad. Sci. U.S.A. 87:1874-1878), transcriptional amplification system (Kwoh, D. Y. et al., 1989, Proc. Natl. Acad. Sci. U.S.A. 86:1173-1177), Q-Beta Replicase (Lizardi, P. M. et al., 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In one embodiment, any of a variety of sequencing reactions known in the art can be used to directly sequence at least a portion of a gene and detect allelic variants, e.g., mutations, by comparing the sequence of the sample sequence with the corresponding wild-type (control) sequence. Exemplary sequencing reactions include those based on techniques developed by Maxam and Gilbert (Proc. Natl Acad Sci USA (1977) 74:560) or Sanger (Sanger et al (1977) Proc. Nat. Acad. Sci 74:5463). It is also contemplated that any of a variety of automated sequencing procedures may be utilized when performing the subject assays (Biotechniques (1995) 19:448), including sequencing by mass spectrometry (see, for example, U.S. Pat. No. 5,547,835 and international patent application Publication Number WO 94/16101, entitled DNA Sequencing by Mass Spectrometry by H. Koster; U.S. Pat. No. 5,547,835 and international patent application Publication Number WO 94/21822 entitled "DNA Sequencing by Mass Spectrometry Via Exonuclease Degradation" by H. Koster), and U.S. Pat. No. 5,605,798 and International Patent Application No. PCT/US96/03651 entitled DNA Diagnostics Based on Mass Spectrometry by H. Koster; Cohen et al. (1996) Adv Chromatogr 36:127-162; and Griffin et al. (1993) Appl Biochem Biotechnol 38:147-159). It will be evident to one skilled in the art that, for certain embodiments, the occurrence of only one, two or three of the nucleic acid bases need be determined in the sequencing reaction. For instance, A-track or the like, e.g., where only one nucleotide is detected, can be carried out.

Yet other sequencing methods are disclosed, e.g., in U.S. Pat. No. 5,580,732 entitled "Method of DNA sequencing employing a mixed DNA-polymer chain probe" and U.S. Pat. No. 5,571,676 entitled "Method for mismatch-directed in vitro DNA sequencing".

In some cases, the presence of a specific allele of a gene in DNA from a subject can be shown by restriction enzyme analysis. For example, a specific nucleotide polymorphism can result in a nucleotide sequence comprising a restriction site which is absent from the nucleotide sequence of another allelic variant.

In other embodiments, alterations in electrophoretic mobility is used to identify the type of gene allelic variant. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control nucleic acids are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In another preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the identity of an allelic variant of a polymorphic region is obtained by analyzing the movement of a nucleic acid comprising the polymorphic region in polyacrylamide gels containing a gradient of denaturant is assayed using denaturing gradient gel electrophoresis (DGGE) (Myers et al (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:1275).

Examples of techniques for detecting differences of at least one nucleotide between 2 nucleic acids include, but are not limited to, selective oligonucleotide hybridization, selective amplification, or selective primer extension. For example, oligonucleotide probes may be prepared in which the known polymorphic nucleotide is placed centrally (allele-specific probes) and then hybridized to target DNA under conditions which permit hybridization only if a perfect match is found (Saiki et al. (1986) Nature 324:163); Saiki et al (1989) Proc. Natl Acad. Sci USA 86:6230; and Wallace et al. (1979) Nucl. Acids Res. 6:3543). Such allele specific oligonucleotide hybridization techniques may be used for the simultaneous detection of several nucleotide changes in different polymorphic regions of gene. For example, oligonucleotides having nucleotide sequences of specific allelic variants are attached to a hybridizing membrane and this membrane is then hybridized with labeled sample nucleic acid. Analysis of the hybridization signal will then reveal the identity of the nucleotides of the sample nucleic acid.

Alternatively, allele specific amplification technology which depends on selective PCR amplification may be used. Oligonucleotides used as primers for specific amplification may carry the allelic variant of interest in the center of the molecule (so that amplification depends on differential hybridization) (Gibbs et al (1989) Nucleic Acids Res. 17: 2437-2448) or at the extreme 3' end of one primer where, under appropriate conditions, mismatch can prevent, or reduce polymerase extension (Prossner (1993) Tibtech 11:238; Newton et al. (1989) Nucl. Acids Res. 17:2503). This technique is also termed "PROBE" for Probe Oligo Base Extension. In addition it may be desirable to introduce a novel restriction site in the region of the mutation to create cleavage-based detection (Gasparini et al (1992) Mol. Cell Probes 6:1).

In another embodiment, identification of the allelic variant is carried out using an oligonucleotide ligation assay (OLA), as described, e.g., in U.S. Pat. No. 4,998,617 and in Landegren, U. et al., Science 241:1077-1080 (1988). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al., Proc. Natl. Acad. Sci. (U.S.A.) 87:8923-8927 (1990). In this method, PCR is used to achieve the exponential amplification of target DNA, which is then detected using OLA.

Several techniques based on this OLA method have been developed and can be used to detect specific allelic variants of a polymorphic region of a gene. For example, U.S. Pat. No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996)Nucleic Acids Res 24: 3728), OLA combined with PCR permits typing of two alleles in a single microtiter well. By marking each of the allele-specific primers with a unique hapten, i.e. digoxigenin and fluorescein, each LA reaction can be detected by using hapten specific antibodies that are labeled with different enzyme reporters, alkaline phosphatase or horseradish peroxidase. This system permits the detection of the two alleles using a high throughput format that leads to the production of two different colors.

The invention further provides methods for detecting single nucleotide polymorphisms in a gene. Because single nucleotide polymorphisms constitute sites of variation flanked by regions of invariant sequence, their analysis requires no more than the determination of the identity of the single nucleotide present at the site of variation and it is unnecessary to determine a complete gene sequence for each patient. Several methods have been developed to facilitate the analysis of such single nucleotide polymorphisms.

In one embodiment, the single base polymorphism can be detected by using a specialized exonuclease-resistant nucleotide, as disclosed, e.g., in Mundy, C. R. (U.S. Pat. No. 4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA TM is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18: 3671 (1990); Syvanen, A. -C., et al., Genomics 8:684-692 (1990), Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA TM in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A.-C., et al., Amer. J. Hum. Genet. 52:46-59 (1993)).

For determining the identity of the allelic variant of a polymorphic region located in the coding region of a gene, yet other methods than those described above can be used. For example, identification of an allelic variant which encodes a mutated gene protein can be performed by using an antibody specifically recognizing the mutant protein in, e.g., immunohistochemistry or immunoprecipitation. Antibodies to wild-type gene protein are described, e.g., in Acton et al. (1999) Science 271:518 (antimouse gene antibody cross-reactive with human gene). Other antibodies to wild-type gene or mutated forms of gene proteins can be prepared according to methods known in the art. Alternatively, one can also measure an activity of an gene protein, such as binding to a lipid or lipoprotein. Binding assays are known in the art and involve, e.g., obtaining cells from a subject, and performing binding experiments with a labeled lipid, to determine whether binding to the mutated form of the receptor differs from binding to the wild-type of the receptor.

If a polymorphic region is located in an exon, either in a coding or non-coding region of the gene, the identity of the allelic variant can be determined by determining the molecular structure of the mRNA, pre-mRNA, or cDNA. The molecular structure can be determined using any of the above described methods for determining the molecular structure of the genomic DNA, e.g., sequencing and SSCP.

The methods described herein may be performed, for example, by utilizing prepackaged diagnostic kits, such as those described above, comprising at least one probe or primer nucleic acid described herein, which may be conveniently used, e.g., to determine whether a subject has or is at risk of developing a disease associated with a specific gene allelic variant.

Sample nucleic acid for using in the above-described diagnostic and prognostic methods can be obtained from any cell type or tissue of a subject. For example, a subject's bodily fluid (e.g. blood) can be obtained by known techniques (e.g. venipuncture) or from human tissues like heart (biopsies, transplanted organs). Alternatively, nucleic acid tests can be performed on dry samples (e.g. hair or skin). Fetal nucleic acid samples for prenatal diagnostics can be obtained from maternal blood as described in International Patent Application No.WO91/07660 to Bianchi. Alternatively, amniocytes or chorionic villi may be obtained for performing prenatal testing.

Diagnostic procedures may also be performed in situ directly upon tissue sections (fixed and/or frozen) of patient tissue obtained from biopsies or resections, such that no nucleic acid purification is necessary. Nucleic acid reagents may be used as probes and/or primers for such in situ procedures (see, for example, Nuovo, G. J., 1992, PCR in situ hybridization: protocols and applications, Raven Press, New York).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

In practicing the present invention, the distribution of polymorphic patterns in a large number of individuals exhibiting particular markers of cardiovascular status is determined by any of the methods described above, and compared with the distribution of polymorphic patterns in patients that have been matched for age, ethnic origin, and/or any other statistically or medically relevant parameters, who exhibit quantitatively or qualitatively different status markers. Correlations are achieved using any method known in the art, including nominal logistic regression, chi square tests or standard least squares regression analysis. In this manner, it is possible to establish statistically significant correlations between particular polymorphic patterns and particular cardiovascular statuses (given in p values). It is further possible to establish statistically significant correlations between particular polymorphic patterns and changes in cardiovascular status such as, would result, e.g., from particular treatment regimens. In this manner, it is possible to correlate polymorphic patterns with responsivity to particular treatments.

Isolated Polymorphic Nucleic Acids, Probes, and Vectors

The present invention provides isolated nucleic acids comprising the polymorphic positions described herein for human genes; vectors comprising the nucleic acids; and transformed host cells comprising the vectors. The invention also provides probes which are useful for detecting these polymorphisms.

In practicing the present invention, many conventional techniques in molecular biology, microbiology, and recombinant DNA, are used. Such techniques are well known and are explained fully in, for example, Sambrook et al., 1989, Molecular Cloning: A Laboratory Manual, Second Edition, Cold Spring Harbor Laboratory Press, Cold Spring Harbor, New York; DNA Cloning: A Practical Approach, Volumes I and II, 1985 (D. N. Glover ed.); Oligonucleotide Synthesis, 1984, (M. L.Gait ed.); Nucleic Acid Hybridization, 1985, (Hames and Higgins); Ausubel et al., Current Protocols in Molecular Biology, 1997, (John Wiley and Sons); and Methods in Enzymology Vol. 154 and Vol. 155 (Wu and Grossman, and Wu, eds., respectively).

Insertion of nucleic acids (typically DNAs) comprising the sequences in a functional surrounding like full length cDNA of the present invention into a vector is easily accomplished when the termini of both the DNAs and the vector comprise compatible restriction sites. If this cannot be done, it may be necessary to modify the termini of the DNAs and/or vector by digesting back single-stranded DNA overhangs generated by restriction endonuclease cleavage to produce blunt ends, or to achieve the same result by filling in the single-stranded termini with an appropriate DNA polymerase.

Alternatively, any site desired may be produced, e.g., by ligating nucleotide sequences (linkers) onto the termini. Such linkers may comprise specific oligonucleotide sequences that define desired restriction sites. Restriction sites can also be generated by the use of the polymerase chain reaction (PCR). See, e.g., Saiki et al., 1988, Science 239:48. The cleaved vector and the DNA fragments may also be modified if required by homopolymeric tailing.

The nucleic acids may be isolated directly from cells or may be chemically synthesized using known methods. Alternatively, the polymerase chain reaction (PCR) method can be used to produce the nucleic acids of the invention, using either chemically synthesized strands or genomic material as templates. Primers used for PCR can be synthesized using the sequence information provided herein and can further be designed to introduce appropriate new restriction sites, if desirable, to facilitate incorporation into a given vector for recombinant expression.

The nucleic acids of the present invention may be flanked by native gene sequences, or may be associated with heterologous sequences, including promoters, enhancers, response elements, signal sequences, polyadenylation sequences, introns, 5'- and 3'-noncoding regions, and the like. The nucleic acids may also be modified by many means known in the art. Non-limiting examples of such modifications include methylation, "caps", substitution of one or more of the naturally occurring nucleotides with an analog, internucleotide modifications such as, for example, those with uncharged linkages (e.g., methyl phosphonates, phosphotriesters, phosphoroamidates, carbamates, morpholines etc.) and with charged linkages (e.g., phosphorothioates, phosphorodithioates, etc.). Nucleic acids may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. PNAs are also included. The nucleic acid may be derivatized by formation of a methyl or ethyl phosphotriester or an alkyl phosphoramidate linkage. Furthermore, the nucleic acid sequences of the present invention may also be modified with a label capable of providing a detectable signal, either directly or indirectly. Exemplary labels include radioisotopes, fluorescent molecules, biotin, and the like.

The invention also provides nucleic acid vectors comprising the gene sequences or derivatives or fragments thereof of genes described in the Examles. A large number of vectors, including plasmid and fungal vectors, have been described for replication and/or expression in a variety of eukaryotic and prokaryotic hosts, and may be used for gene therapy as well as for simple cloning or protein expression. Non-limiting examples of suitable vectors include without limitation pUC plasmids, pET plasmids (Novagen, Inc., Madison, Wis.), or pRSET or pREP (Invitrogen, San Diego, Calif.), and many appropriate host cells, using methods disclosed or cited herein or otherwise known to those skilled in the relevant art. The particular choice of vector/host is not critical to the practice of the invention.

Suitable host cells may be transformed/transfected/infected as appropriate by any suitable method including electroporation, $CaCl_2$ mediated DNA uptake, fungal or viral infection, microinjection, microprojectile, or other established methods. Appropriate host cells included bacteria, archebacteria, fungi, especially yeast, and plant and animal cells, especially mammalian cells. A large number of transcription initiation and termination regulatory regions have been isolated and shown to be effective in the transcription and translation of heterologous proteins in the various hosts. Examples of these regions, methods of isolation, manner of manipulation, etc. are known in the art. Under appropriate expression conditions, host cells can be used as a source of recombinantly produced peptides and polypeptides encoded by genes of the Examples. Nucleic acids encoding peptides or polypeptides from gene sequences of the Examples may also be introduced into cells by recombination events. For example, such a sequence can be introduced into a cell and thereby effect homologous recombination at the site of an endogenous gene or a sequence with substantial identity to the gene. Other recombination-based methods such as non-homologous recombinations or deletion of endogenous genes by homologous recombination may also be used.

In case of proteins that form heterodimers or other multimers, both or all subunits have to be expressed in one system or cell.

The nucleic acids of the present invention find use as probes for the detection of genetic polymorphisms and as templates for the recombinant production of normal or variant peptides or polypeptides encoded by genes listed in the Examples.

Probes in accordance with the present invention comprise without limitation isolated nucleic acids of about 10-100 bp, preferably 15-75 bp and most preferably 17-25 bp in length, which hybridize at high stringency to one or more of the polymorphic sequences disclosed herein or to a sequence immediately adjacent to a polymorphic position. Furthermore, in some embodiments a full-length gene sequence may be used as a probe. In one series of embodiments, the probes span the polymorphic positions in genes disclosed herein. In another series of embodiments, the probes correspond to sequences immediately adjacent to the polymorphic positions.

## Polymorphic Polypeptides and Polymorphism-Specific Antibodies

The present invention encompasses isolated peptides and polypeptides encoded by genes listed in the Examples comprising polymorphic positions disclosed herein. In one preferred embodiment, the peptides and polypeptides are useful screening targets to identify cardiovascular drugs. In another preferred embodiments, the peptides and polypeptides are capable of eliciting antibodies in a suitable host animal that react specifically with a polypeptide comprising the polymorphic position and distinguish it from other polypeptides having a different sequence at that position.

Polypeptides according to the invention are preferably at least five or more residues in length, preferably at least fifteen residues. Methods for obtaining these polypeptides are described below. Many conventional techniques in protein biochemistry and immunology are used. Such techniques are well known and are explained in Immunochemical Methods in Cell and Molecular Biology, 1987 (Mayer and Waler, eds; Academic Press, London); Scopes, 1987, Protein Purification: Principles and Practice, Second Edition (Springer-Verlag, N.Y.) and Handbook of Experimental Immunology, 1986, Volumes I-IV (Weir and Blackwell eds.).

Nucleic acids comprising protein-coding sequences can be used to direct the ITT recombinant expression of polypeptides encoded by genes disclosed herein in intact cells or in cell-free translation systems. The known genetic code, tailored if desired for more efficient expression in a given host organism, can be used to synthesize oligonucleotides encoding the desired amino acid sequences. The polypeptides may be isolated from human cells, or from heterologous organisms or cells (including, but not limited to, bacteria, fungi, insect, plant, and mammalian cells) into which an appropriate protein-coding sequence has been introduced and expressed. Furthermore, the polypeptides may be part of recombinant fusion proteins.

Peptides and polypeptides may be chemically synthesized by commercially available automated procedures, including, without limitation, exclusive solid phase synthesis, partial solid phase methods, fragment condensation or classical solution synthesis. The polypeptides are preferably prepared by solid phase peptide synthesis as described by Merrifield, 1963, J. Am. Chem. Soc. 85:2149.

Methods for polypeptide purification are well-known in the art, including, without limitation, preparative disc-gel electrophoresis, isoelectric focusing, HPLC, reversed-phase HPLC, gel filtration, ion exchange and partition chromatography, and countercurrent distribution. For some purposes, it is preferable to produce the polypeptide in a recombinant system in which the protein contains an additional sequence tag that facilitates purification, such as, but not limited to, a polyhistidine sequence. The polypeptide can then be purified from a crude lysate of the host cell by chromatography on an appropriate solid-phase matrix. Alternatively, antibodies produced against peptides encoded by genes disclosed herein, can be used as purification reagents. Other purification methods are possible.

The present invention also encompasses derivatives and homologues of the polypeptides. For some purposes, nucleic acid sequences encoding the peptides may be altered by substitutions, additions, or deletions that provide for functionally equivalent molecules, i.e., function-conservative variants. For example, one or more amino acid residues within the sequence can be substituted by another amino acid of similar properties, such as, for example, positively charged amino acids (arginine, lysine, and histidine); negatively charged amino acids (aspartate and glutamate); polar neutral amino acids; and non-polar amino acids.

The isolated polypeptides may be modified by, for example, phosphorylation, sulfation, acylation, or other protein modifications. They may also be modified with a label capable of providing a detectable signal, either directly or indirectly, including, but not limited to, radioisotopes and fluorescent compounds.

The present invention also encompasses antibodies that specifically recognize the polymorphic positions of the invention and distinguish a peptide or polypeptide containing a particular polymorphism from one that contains a different sequence at that position. Such polymorphic position-specific antibodies according to the present invention include polyclonal and monoclonal antibodies. The antibodies may be elicited in an animal host by immunization with peptides encoded by genes disclosed herein or may be formed by in vitro immunization of immune cells. The immunogenic components used to elicit the antibodies may be isolated from human cells or produced in recombinant systems. The antibodies may also be produced in recombinant systems programmed with appropriate antibody-encoding DNA. Alternatively, the antibodies may be constructed by biochemical reconstitution of purified heavy and light chains. The antibodies include hybrid antibodies (i.e., containing two sets of heavy chain/light chain combinations, each of which recognizes a different antigen), chimeric antibodies (i.e., in which either the heavy chains, light chains, or both, are fusion proteins), and univalent antibodies (i.e., comprised of a heavy chain/light chain complex bound to the constant region of a second heavy chain). Also included are Fab fragments, including Fab' and F(ab).sub.2 fragments of antibodies. Methods for the production of all of the above types of antibodies and derivatives are well-known in the art and are discussed in more detail below. For example, techniques for producing and processing polyclonal antisera are disclosed in Mayer and Walker, 1987, Immunochemical Methods in Cell and Molecular Biology, (Academic Press, London). The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. See, e.g., Schreier et al., 1980, Hybridoma Techniques; U.S. Pat. Nos. 4,341,761; 4,399,121; 4,427,783; 4,444,887; 4,466,917; 4,472,500; 4,491,632; and 4,493,890. Panels of monoclonal antibodies produced against peptides encoded by genes disclosed herein can be screened for various properties; i.e. for isotype, epitope affinity, etc.

The antibodies of this invention can be purified by standard methods, including but not limited to preparative disc-gel electrophoresis, isoelectric focusing, HPLC, reversed-phase HPLC, gel filtration, ion exchange and partition chromatography, and countercurrent distribution. Purification methods for antibodies are disclosed, e.g., in The Art of Antibody Purification, 1989, Amicon Division, W. R. Grace & Co. General protein purification methods are described in Protein Purification: Principles and Practice, R. K. Scopes, Ed., 1987, Springer-Verlag, New York, N.Y.

Methods for determining the immunogenic capability of the disclosed sequences and the characteristics of the resulting sequence-specific antibodies and immune cells are well-known in the art. For example, antibodies elicited in response to a peptide comprising a particular polymorphic sequence can be tested for their ability to specifically recognize that polymorphic sequence, i.e., to bind differentially to a peptide or polypeptide comprising the polymorphic sequence and thus distinguish it from a similar peptide or polypeptide containing a different sequence at the same position.

## Kits

As set forth herein, the invention provides diagnostic methods, e.g., for determining the identity of the allelic variants of polymorphic regions present in the gene loci of genes disclosed herein, wherein specific allelic variants of the polymorphic region are associated with cardiovascular diseases. In a preferred embodiment, the diagnostic kit can be used to determine whether a subject is at risk of developing a cardiovascular disease. This information could then be used, e.g., to optimize treatment of such individuals.

In preferred embodiments, the kit comprises a probe or primer which is capable of hybridizing to a gene and thereby identifying whether the gene contains an allelic variant of a polymorphic region which is associated with a risk for cardiovascular disease. The kit preferably further comprises instructions for use in diagnosing a subject as having, or having a predisposition, towards developing a cardiovascular disease. The probe or primers of the kit can be any of the probes or primers described in this file.

Preferred kits for amplifying a region of a gene comprising a polymorphic region of interest comprise one, two or more primers.

## Antibody-based diagnostic methods and kits

The invention also provides antibody-based methods for detecting polymorphic patterns in a biological sample. The methods comprise the steps of: (i) contacting a sample with one or more antibody preparations, wherein each of the antibody preparations is specific for a particular polymorphic form of the proteins encoded by genes disclosed herein, under conditions in which a stable antigen-antibody complex can form between the antibody and antigenic components in the sample; and (ii) detecting any antigen-antibody complex formed in step (i) using any suitable means known in the art, wherein the detection of a complex indicates the presence of the particular polymorphic form in the sample.

Typically, immunoassays use either a labelled antibody or a labelled antigenic component (e.g., that competes with the antigen in the sample for binding to the antibody). Suitable labels include without limitation enzyme-based, fluorescent, chemiluminescent, radioactive, or dye molecules. Assays that amplify the signals from the probe are also known, such as, for example, those that utilize biotin and avidin, and enzyme-labelled immunoassays, such as ELISA assays.

The present invention also provides kits suitable for antibody-based diagnostic applications. Diagnostic kits typically include one or more of the following components:

(i) Polymorphism-specific antibodies. The antibodies may be pre-labelled; alternatively, the antibody may be unlabelled and the ingredients for labelling may be included in the kit in separate containers, or a secondary, labelled antibody is provided; and

(ii) Reaction components: The kit may also contain other suitably packaged reagents and materials needed for the particular immunoassay protocol, including solid-phase matrices, if applicable, and standards.

The kits referred to above may include instructions for conducting the test. Furthermore, in preferred embodiments, the diagnostic kits are adaptable to high-throughput and/or automated operation.

## Drug Targets and Screening Methods

According to the present invention, nucleotide sequences derived from genes disclosed herein and peptide sequences encoded by genes disclosed herein, particularly those that contain one or more polymorphic sequences, comprise useful targets to identify cardiovascular drugs, i.e., compounds that are effective in treating one or more clinical symptoms of cardiovascular disease. Furthermore, especially when a protein is a multimeric protein that are build of two or more subunits, is a combination of different polymorphic subunits very useful.

Drug targets include without limitation (i) isolated nucleic acids derived from the genes disclosed herein, and (ii) isolated peptides and polypeptides encoded by genes disclosed herein, each of which comprises one or more polymorphic positions.

### In vitro screening methods

In one series of embodiments, an isolated nucleic acid comprising one or more polymorphic positions is tested in vitro for its ability to bind test compounds in a sequence-specific manner. The methods comprise:

(i) providing a first nucleic acid containing a particular sequence at a polymorphic position and a second nucleic acid whose sequence is identical to that of the first nucleic acid except for a different sequence at the same polymorphic position;

(ii) contacting the nucleic acids with a multiplicity of test compounds under conditions appropriate for binding; and

(iii) identifying those compounds that bind selectively to either the first or second nucleic acid sequence.

Selective binding as used herein refers to any measurable difference in any parameter of binding, such as, e.g., binding affinity, binding capacity, etc.

In another series of embodiments, an isolated peptide or polypeptide comprising one or more polymorphic positions is tested in vitro for its ability to bind test compounds in a sequence-specific manner. The screening methods involve:

(i) providing a first peptide or polypeptide containing a particular sequence at a polymorphic position and a second peptide or polypeptide whose sequence is identical to the first peptide or polypeptide except for a different sequence at the same polymorphic position;

(ii) contacting the polypeptides with a multiplicity of test compounds under conditions appropriate for binding; and

(iii) identifying those compounds that bind selectively to one of the nucleic acid sequences.

In preferred embodiments, high-throughput screening protocols are used to survey a large number of test compounds for their ability to bind the genes or peptides disclosed above in a sequence-specific manner.

Test compounds are screened from large libraries of synthetic or natural compounds. Numerous means are currently used for random and directed synthesis of saccharide, peptide, and nucleic acid based compounds. Synthetic compound libraries are commercially available from Maybridge Chemical Co. (Trevillet, Cornwall, UK), Comgenex (Princeton, N.J.), Brandon Associates (Merrimack, N.H.), and Micro-source (New Milford, Conn.). A rare chemical library is available from Aldrich (Milwaukee, Wis.). Alternatively, libraries of natural compounds in the form of bacterial, fungal, plant and animal extracts are available from e.g. Pan Laboratories (Bothell, Wash.) or MycoSearch (N.C.), or are readily producible. Additionally, natural and synthetically produced libraries and compounds are readily modified through conventional chemical, physical, and biochemical means.

**In vivo screening methods**

Intact cells or whole animals expressing polymorphic variants of genes disclosed herein can be used in screening methods to identify candidate cardiovascular drugs.

In one series of embodiments, a permanent cell line is established from an individual exhibiting a particular polymorphic pattern. Alternatively, cells (including without limitation mammalian, insect, yeast, or bacterial cells) are programmed to express a gene comprising one or more polymorphic sequences by introduction of appropriate DNA. Identification of candidate compounds can be achieved using any suitable assay, including without limitation (i) assays that measure selective binding of test compounds to particular polymorphic variants of proteins encoded by genes disclosed herein; (ii) assays that measure the ability of a test compound to modify (i.e., inhibit or enhance) a measurable activity or function of proteins encoded by genes disclosed herein; and (iii) assays that measure the ability of a compound to modify (i.e., inhibit or enhance) the transcriptional activity of sequences derived from the promoter (i.e., regulatory) regions of genes disclosed herein.

In another series of embodiments, transgenic animals are created in which (i) one or more human genes disclosed herein, having different sequences at particular polymorphic positions are stably inserted into the genome of the transgenic animal; and/or (ii) the endogenous genes disclosed herein are inactivated and replaced with human genes disclosed herein, having different sequences at particular polymorphic positions. See, e.g., Coffman, Semin. Nephrol. 17:404, 1997; Esther et al., Lab. Invest. 74:953, 1996; Murakami et al., Blood Press. Suppl. 2:36, 1996. Such animals can be treated with candidate compounds and monitored for one or more clinical markers of cardiovascular status.

The following are intended as non-limiting examples of the invention.

**Material and Methods**

Genotyping of patient DNA with the Pyrosequencing™ Method as described in the patent application WO 9813523:

First a PCR is set up to amplify the flanking regions around a SNP. Therefor 2 ng of genomic DNA (patient sample) are mixed with a primerset (20 - 40 pmol) producing a 75 to 320 bp PCR fragment with 0,3 to 1 U Qiagens Hot Star Taq Polymerase™ in a total volume of 20 $\mu$L. One primer is biotinylated depending on the direction of the sequencing primer. To force the biotinylated primer to be incorporated it is used 0,8 fold.

For primer design, programms like Oligo 6™ (Molecular Biology Insights) or Primer Select™ (DNAStar) are used. PCR setup is performed by a BioRobot 3000™ from Qiagen. PCR takes place in T1 or Tgradient Thermocyclers ™ from Biometra.

The whole PCR reaction is transferred into a PSQ plate ™ (Pyrosequencing) and prepared using the Sample Prep Tool ™ and SNP Reagent Kit ™ from Pyrosequencing according to their instructions.

<u>**Preparation of template for Pyrosequencing**™:</u>

Sample preparation using PSQ 96 Sample Prep Tool

1. Mount the PSQ 96 Sample Prep Tool Cover onto the PSQ 96 Sample Prep Tool as follows: Place the cover on the desk, retract the 4 attachment rods by separating the handle from the magnetic rod holder, fit the magnetic rods into the holes of the cover plate, push the handle downward until a click is heard. The PSQ 96 Sample Prep Tool is now ready for use.

2. To transfer beads from one plate to another, place the covered tool into the PSQ 96 Plate containing the samples and lower the magnetic rods by separating the handle from the magnetic rod holder. Move the tool up and down a few times then wait for 30-60 seconds. Transfer the beads into a new PSQ 96 plate containing the solution of choice.

3. Release the beads by lifting the magnetic rod holder, bringing it together with the handle. Move the tool up and down a few times to make sure that the beads are released.

All steps are performed at room temperature unless otherwise stated.

Immobilization of PCR product:

Biotinylated PCR products are immobilized on streptavidin-coated Dynabeads™ M-280 Streptavidin. Parallel immobilization of several samples are performed in the PSQ 96 Plate.

1. Mix PCR product, 20 μl of a well optimized PCR, with 25 μl 2X BW-buffer II. Add 60-150 μg Dynabeads. It is also possible to add a mix of Dynabeads and 2X BW-buffer II to the PCR product yielding a final BW-buffer II concentration of approximately 1x.

2. Incubate at 65°C for 15 min agitation constantly to keep the beads dispersed. For optimal immobilization of fragments longer than 300 bp use 30 min incubation time.

*Strand separation*

4. For strand separation, use the PSQ 96 Sample Prep Tool to transfer the beads with the immobilized sample to a PSQ 96 Plate containing 50 μl 0.50 M NaOH per well. Release the beads.

5. After approximately 1 min, transfer the beads with the immobilized strand to a PSQ 96 Plate containing 99 μl 1x Annealing buffer per well and mix thoroughly.

6. Transfer the beads to a PSQ 96 Plate containing 45 μl of a mix of 1x Annealing buffer and 3-15 pmoles sequencing primer per well.

7. Heat at 80°C for 2 minutes in the PSQ 96 Sample Prep Thermoplate and move to room temperature.

8. After reaching room temperature, continue with the sequencing reaction.

Sequencing reaction

1. Choose the method to be used ("SNP Method") and enter relevant information in the PSQ 96 Instrument Control software.

2. Place the cartridge and PSQ 96 Plate in the PSQ 96 Instrument.

3. Start the run.

Genotyping with a service contractor

Qiagen Genomics, formerly Rapigene, is a service contractor for genotyping SNPs in patient samples. Their method is based on a primer extension method where two complementary primers are designed for each genotype that are labeled with different tags. Depending on the genotype only one primer will be elongated together with a certain tag. This tag can be detected with mass spectrometry and is a measure for the respective genotype. The method is described in the following patent: "Detection and identification of nucleic acid molecules - using tags which may be detected by non-fluorescent spectrometry or potentiometry" (WO 9727325).

## Examples

**Table 1:** Definition of "good" and "bad" serum lipid levels

| | "Good" | "Bad" |
|---|---|---|
| LDL-Cholesterol [mg/dL] | 125 - 150 | 170 - 200 |
| Cholesterol [mg/dL] | 190 - 240 | 265 - 315 |
| HDL-Cholesterol [mg/dL] | 60 -105 | 30 - 55 |
| Triglycerides [mg/dL] | 45 - 115 | 170 - 450 |
| Number of Patients | 146 | 132 |

An informed consent was signed by the patients and control people. Blood was taken by a physician according to medical standard procedures.
Samples were collected anonymous and labeled with a patient number.
DNA was extracted using kits from Qiagen.

**Table 2a: Oligonucleotide primers used for genotyping using mass spectrometry**

The baySNP number refers to an internal numbering of the CA SNPs. Primer sequences are listed for preamplification of the genomic fragments (primers EF and ER) and for subsequent allele specific PCR of the SNP.

| Primer Name | Primer Sequence | baySNP |
|---|---|---|
| 11558_A251C_EF | AAAGAAAGTCAGGATAAAAA | 11558 |
| 11558_A251C_ER | TTTGGTGAGAGTAAGGAG | |
| 11558_A251C_AR | gggacggtcggtagatTTAGACAGGGGTCTTATT | |
| 11558_A251C_CR | gctggctcggtcaagaTTAGACAGGGGTCTTATG | |
| | | |
| 1657_C236T_CF_ | gggacggtcggtagatGCTCTGCTTCTTGGTGCC | 1657 |
| 1657_C236T_EF_ | CAGAAATACTCTGCTGTCTG | |
| 1657_C236T_ER_U | GACGATGCCTTCAGCACAGTGAAAACCATCAAGG AA | |
| 1657_C236T_TF_ | gctggctcggtcaagaGCTCTGCTTCTTGGTGCT | |
| | | |
| 11652_C251T_EF | CACGAGGTCAGGAAGATG | 11652 |
| 11652_C251T_ER | GACGGTGTCATAGGAAGAGA | |
| 11652_C251T_CF | gggacggtcggtagatGAGATGAGGGCTCCTGGC | |
| 11652_C251T_TF | gctggctcggtcaagaGAGATGAGGGCTCCTGGT | |
| | | |
| 6734_A251C_EF | AAGGAATGTTGTAGAGGGA | 6734 |
| 6734_A251C_ER | AGAAAAAGAAAAGAAAAGGAG | |
| 6734_A251C_AF | gggacggtcggtagatCTTCCCAGTAGTAAATAA | |
| 6734_A251C_CF | gctggctcggtcaagaCTTCCCAGTAGTAAATAC | |
| | | |

(continued)

| Primer Name | Primer Sequence | baySNP |
|---|---|---|
| 1504_C180T_CF_ | gggacggtcggtagatGTGACTTTTGGTTCCCAC | 1504 |
| 1504_C180T_EF_ | AACTCGGGGTCACTGGTCT | |
| 1504_C180T_ER_U | GACGATGCCTTCAGCACACAGCGGGTATGGAGGA TG | |
| 1504_C180T_TF_ | gctggctcggtcaagaGTGACTTTTGGTTCCCAT | |
| | | |
| 11536_C50G_EF | GACCCACCTCCCCCTCCC | 11536 |
| 11536_C50G_ER | TTGTTCAGACACTGAGAAGAGCTGTAT | |
| 11536_C50G_CF | gggacggtcggtagatTGCCGCGTCCCTGCCCCC | |
| 11536_C50G_GF | gctggctcggtcaagaTGCCGCGTCCCTGCCCCG | |
| | | |
| 7363_A94G_EF | TGGTGTTAGGAGAAAAAGTAG | 7363 |
| 7363_A94G_ER | AAGGGAAGAGGAGAGAAA | |
| 7363_A94G_AF | gggacggtcggtagatAGGCTGGTTTCCCACACA | |
| 7363_A94G_GF | gctggctcggtcaagaAGGCTGGTTTCCCACACG | |
| | | |
| 11001_C286T_EF | TTCCCAAAGACCCACA | 11001 |
| 11001_C286T_ER | CCTCCACCGCTATCAC | |
| 11001_C286T_CR | gggacggtcggtagatTGGCTGCAGGACGTCCAG | |
| 11001_C286T_TR | gctggctcggtcaagaTGGCTGCAGGACGTCCAA | |
| | | |
| 10948_G140T_EF | AAGGACAGGGTCAGGAAAG | 10948 |
| 10948_G140T_ER | CAGAGGGAGGAAGGAGGT | |
| 10948_G140T_GF | gggacggtcggtagatATGGAGGAGGGTGTCTGG | |
| 10948_G140T_TF | gctggctcggtcaagaATGGAGGAGGGTGTCTGT | |
| | | |
| 2353_A194G_AF_ | gggacggtcggtagatATGAATCCTTTCCCTTTA | 2353 |
| 2353_A194G_EF_ | GAGCAAAGTCAGCCATCT | |
| 2353_A194G_ER_U | GACGATGCCTTCAGCACACCCTCATTTATCCCTC AAC | |
| 2353_A194G_GF_ | gctggctcggtcaagaATGAATCCTTTCCCTTTG | |
| | | |
| 10785_C113T_EF | ATACTTCAACCGCATACACA | 10785 |
| 10785_C113T_ER | GGAGGGGAAATCAGATAAA | |
| 10785_C113T_CR | gggacggtcggtagatAATTGAACCTGTAAATCG | |
| 10785_C113T_TR | gctggctcggtcaagaAATTGAACCTGTAAATCA | |
| | | |

(continued)

| Primer Name | Primer Sequence | baySNP |
|---|---|---|
| 4018_C251T_EF | CGAGTTAGAGCCAGTCAAG | 4018 |
| 4018_C251T_ER | GGAGGGAAGAAATCAACA | |
| 4018_C251T_CR | gggacggtcggtagatCATGGGGGCTCTGTGGCG | |
| 4018_C251T_TR | gctggctcggtcaagaCATGGGGGCTCTGTGGCA | |
| | | |
| 11654_A251G_EF | CGTATCTCTTGCCTTTCTT | 11654 |
| 11654_A251G_ER | CTTCTCTTATGCCTTCCC | |
| 11654_A251G_AF | gggacggtcggtagatTTACTTGAAAGGACACCA | |
| 11654_A251G_GF | gctggctcggtcaagaTTACTTGAAAGGACACCG | |
| | | |
| 11655_A251C_EF | CGTATCTCTTGCCTTTCTT | 11655 |
| 11655_A251C_ER | CTTCTCTTATGCCTTCCC | |
| 11655_A251C_AF | gggacggtcggtagatTTCTGCACTAAAGCTGTA | |
| 11655_A251C_CF | gctggctcggtcaagaTTCTGCACTAAAGCTGTC | |
| | | |
| 11637_A162C_EF | AAAGACCCTGAAAACACA | 11637 |
| 11637_A162C_ER | AACTGACAAAGAACCATTTAC | |
| 11637_A162C_AF | gggacggtcggtagatGTCCAAAAACTAAAAAGA | |
| 11637_A162C_CF | gctggctcggtcaagaGTCCAAAAACTAAAAAGC | |
| | | |
| 11627_C251T_EF | TTTATCACTACACCCCCTACTC | 11627 |
| 11627_C251T_ER | GACAGACCGACCAATCAC | |
| 11627_C251T_CR | gggacggtcggtagatCCCTGGGAAGGTTGAGAG | |
| 11627_C251T_TR | gctggctcggtcaagaCCCTGGGAAGGTTGAGAA | |
| | | |
| 2463_C479T_EF | GGCGATGGCCAAATAATAAA | 2463 |
| 2463_C479T_ER | AACTCTCCCGCACAGTCG | |
| 2463_C479T_CR | gggacggtcggtagatCTCATCCTTCGGATGCTG | |
| 2463_C479T_TR | gctggctcggtcaagaCTCATCCTTCGGATGCTA | |
| | | |
| 11456_A251G_EF | GGTTTAGGGGTGGGGTAG | 11456 |
| 11456_A251G_ER | CTGAGACTGTGGGTTCTGG | |
| 11456_A251G_AR | gggacggtcggtagatCCATGCCCTTGTTACTAT | |
| 11456_A251G_GR | gctggctcggtcaagaCCATGCCCTTGTTACTAC | |
| | | |
| 4966_A251G_EF | CATTGCTCTTCCTCTCTGT | 4966 |
| 4966_A251G_ER | GTGTCATCATTCCTTTCTTG | |
| 4966_A251G_AR | gggacggtcggtagatTCAGAGACATGAGTCCAT | |

(continued)

| Primer Name | Primer Sequence | baySNP |
|---|---|---|
| 4966_A251G_GR | gctggctcggtcaagaTCAGAGACATGAGTCCAC | |
| | | |
| 11531_A251G_EF | CTTTGACAGGTGGGGGTG | 11531 |
| 11531_A251G_ER | TGCACTGAAATAATACTAGGCAGG | |
| 11531_A251G_AR | gggacggtcggtagatGTGCACCGGGGCCAAGGT | |
| 11531_A251G_GR | gctggctcggtcaagaGTGCACCGGGGCCAAGGC | |
| | | |
| 7075_C182G_EF | TGGCCAAATAATAAACTAACA | 7075 |
| 7075_C182G_ER | CCATCCCCATAAACTCTC | |
| 7075_C182G_CR | gggacggtcggtagatCTCAGTGCGCCTCGCTCG | |
| 7075_C182G_GR | gctggctcggtcaagaCTCAGTGCGCCTCGCTCC | |
| | | |
| 6396_C199T_EF | CAGGGCAAAGGGAAGTAG | 6396 |
| 6396_C199T_ER | GAAAGGTCAAGGCAGGAG | |
| 6396_C199T_CR | gggacggtcggtagatCAAGGAGCAGAGCAAGGG | |
| 6396_C199T_TR | gctggctcggtcaagaCAAGGAGCAGAGCAAGGA | |
| | | |
| 8480_C71G_EF | CCTAAAAGAGGGTGATAG | 8480 |
| 8480_C71G_ER | GGAGAAAAGTCCATAAAG | |
| 8480_C71G_CR | gggacggtcggtagatTTTTGAGAATAGTGTAAG | |
| 8480_C71G_GR | gctggctcggtcaagaTTTTGAGAATAGTGTAAC | |
| | | |
| 4912_A74G_EF | CTTCACTGAGCGTCCGCAGAG | 4912 |
| 4912_A74G_ER | CCGTCGGCCCGATTCA | |
| 4912_A74G_AR | CAGGCGAGCCTCAGCCCT | |
| 4912_A74G_GR | CAGGCGAGCCTCAGCCCC | |
| | | |
| 6872_A254G_EF | CATCAAGGCAGACCAA | 6872 |
| 6872_A254G_ER | GAAGGAGAGCAAAGGG | |
| 6872_A254G_AF | gggacggtcggtagatAAGATACCTAAATAACAA | |
| 6872_A254G_GF | gctggctcggtcaagaAAGATACCTAAATAACAG | |
| | | |
| 5704_C61T_EF | ACAGCCATAACAGGAGTG | 5704 |
| 5704_C61T_ER | GGGTTACTCAACCTAAGAGA | |
| 5704_C61T_CR | gggacggtcggtagatGTTCTCTTTGGGAAAACG | |
| 5704_C61T_TR | gctggctcggtcaagaGTTCTCTTTGGGAAAACA | |
| | | |
| 8138_C251T_EF | TCCTGTTCCTGTCTCTCTCT | 8138 |

(continued)

| Primer Name | Primer Sequence | baySNP |
|---|---|---|
| 8138_C251T_ER | CATCATCTCTGACTCCTGTG | |
| 8138_C251T_CR | gggacggtcggtagatACTTAACTTTACAGGCGG | |
| 8138_C251T_TR | gctggctcggtcaagaACTTAACTTTACAGGCGA | |
| | | |
| 10830_A194G_EF | AGAGTTGATAGTTCCGAGAGA | 10830 |
| 10830_A194G_ER | CAGACATTTTGGTAGTGATTG | |
| 10830_A194G_AR | gggacggtcggtagatAAAGTTAGTGAAAGAAGT | |
| 10830_A194G_GR | gctggctcggtcaagaAAAGTTAGTGAAAGAAGC | |
| | | |
| 8168_A251C_EF | GAAGAGAAGCCAGGAATG | 8168 |
| 8168_A251C_ER | CCTAGACCCATCCAGAAA | |
| 8168_A251C_AF | gggacggtcggtagatTAATGGAGAGGGGGCCTA | |
| 8168_A251C_CF | gctggctcggtcaagaTAATGGAGAGGGGGCCTC | |
| | | |
| 9883_A249G_EF | TCCACAACCTCAAAACCAC | 9883 |
| 9883_A249G_ER | CACAGTCCTGCAAGCTCA | |
| 9883_A249G_AR | gggacggtcggtagatCCGTGGCCGTGGCTCACT | |
| 9883_A249G_GR | gctggctcggtcaagaCCGTGGCCGTGGCTCACC | |
| | | |
| 11462_G251T_EF | GAGAACAATAAAAGACCAAAAA | 11462 |
| 11462_G25IT_ER | TAGCACAATCAACCCAGA | |
| 11462_G251T_GF | gggacggtcggtagatCTGAAAGCTACTGGAAAG | |
| 11462_G251T_TF | gctggctcggtcaagaCTGAAAGCTACTGGAAAT | |
| | | |
| 8241_A251G_EF | TTTTCTAACCCAGTCTTATTTT. | 8241 |
| 8241_A251G_ER | TGAAATCTGAAGTCTTACACC | |
| 8241_A251G_AF | gggacggtcggtagatTTCTTCCACTTTTTCCAA | |
| 8241_A251G_GF | gctggctcggtcaagaTTCTTCCACTTTTTCCAG | |
| | | |
| | | |
| 11248-C225T-EF | TGAGTTGAACAGCACTTGG | 11248 |
| 11248_C225T_ER | AGGGTAAGGGAGGGAAAA | |
| 11248_C225T_CR | gggacggtcggtagatTGATTCTTTCGCTTGGCG | |
| 11248_C225T_TR | gctggctcggtcaagaTGATTCTTTCGCTTGGCA | |
| | | |
| 11448_A251G_EF | ACAGAAGAACAACAACAAAAC | 11448 |
| 11448_A251G_ER | TGCGTATGAGGTAAAGAGA | |
| 11448_A251G_AF | gggacggtcggtagatGAAGCTGGGAGTGGTGAA | |

(continued)

| Primer Name | Primer Sequence | baySNP |
|---|---|---|
| 11448_A251G_GF | gctggctcggtcaagaGAAGCTGGGAGTGGTGAG | |
| | | |
| 11449_C251G_EF | ACAGAAGAACAACAACAAAAC | 11449 |
| 11449_C251G_ER | TGCGTATGAGGTAAAGAGA | |
| 11449_C251G_CF | gggacggtcggtagatATGAGTGAAGCCTGTCTC | |
| 11449_C251G_GF | gctggctcggtcaagaATGAGTGAAGCCTGTCTG | |
| | | |
| 11450_A251T_EF | ACAGAAGAACAACAACAAAAC | 11450 |
| 11450_A251T_ER | TGCGTATGAGGTAAAGAGA | |
| 11450_A251T_AR | gggacggtcggtagatGGACCATAATCTTGAAGT | |
| 11450_A251T_TR | gctggctcggtcaagaGGACCATAATCTTGAAGA | |
| | | |
| 9940_C93T_EF | CATCTCCTGGAAGAACAA | 9940 |
| 9940_C93T_ER | CAAACATAAGTGCATGAAAG | |
| 9940_C93T_CR | gggacggtcggtagatGTCAGGGCCCAATAGGTG | |
| 9940_C93T_TR | gctggctcggtcaagaGTCAGGGCCCAATAGGTA | |
| | | |
| | | |
| 11624_C251T_EF | TCGGGAGGTGTAAGTAAG . | 11624 |
| 11624_C251T_ER | CCACAGTCAGAAGAGACAA | |
| 11624_C251T_CR | gggacggtcggtagatAGAGACCCTGGTCCCAAG | |
| 11624_C251T_TR | gctggctcggtcaagaAGAGACCCTGGTCCCAAA | |
| | | |
| 10388_A251G_EF | CACCCCTATACACATATCC | 10388 |
| 10388_A251G_ER | GTTTGGAATACATCATTGTC | |
| 10388_A251G_AR | gggacggtcggtagatTATTTTTATTGGCTATAT | |
| 10388_A251G_GR | gctggctcggtcaagaTATTTTTATTGGCTATAC | |
| | | |
| 10877_A251C_EF | CCTGTTTCTCAACCTTCTC | 10877 |
| 10877_A251C_ER | ATGGTCTATGGAACCTAATCT | |
| 10877_A251C_AF | gggacggtcggtagatGCACTGATTCTGCTTCCA | |
| 10877_A251C_CF | gctggctcggtcaagaGCACTGATTCTGCTTCCC | |
| | | |
| 52_C397G_CR_ | gggacggtcggtagatTATTTTATAATGCAAAAG | 52 |
| 52_C397G_EF_U | GACGATGCCTTCAGCACAGTGAATTGCCAGATTA GTG | |
| 52_C397G_ER_ | TCTAAAGTGCTGGGATTG | |

(continued)

| Primer Name | Primer Sequence | baySNP |
|---|---|---|
| 52_C397G_GR_ | gctggctcggtcaagaTATTTTATAATGCAAAAC | |
| | | |
| 57_C290T_CR_ | gggacggtcggtagatAGCAGCAGGTGGCAAGAG | 57 |
| 57_C290T_EF_U | GACGATGCCTTCAGCACATAACATTAGGGCACAA CC | |
| 57_C290T_ER_ | CCCACAAGTAAGGACAGA | |
| 57_C290T_TR_ | gctggctcggtcaagaAGCAGCAGGTGGCAAGAA | |
| | | |
| 118_C675T_CF_ | gggacggtcggtagatGTGCTGCTATCTGCTTTC | 118 |
| 118_C675T_EF_ | TACCTGTTTCTCCCACAC | |
| 118_C675T_ER_U | GACGATGCCTTCAGCACAAAAGACAGCCAAAAGA GA. | |
| 118_C675T_TF_ | gctggctcggtcaagaGTGCTGCTATCTGCTTTT | |
| | | |
| 152_A587G_AF_ | gggacggtcggtagatGGTGGGAGGTTCCAGCCA | 152 |
| 152_A587G_EF_ | GCAGGAAGAAAGCTAGAA | |
| 152_A587G_ER_U | GACGATGCCTTCAGCACAAGGCAGGATAATGACA AC | |
| 152_A587G_GF_ | gctggctcggtcaagaGGTGGGAGGTTCCAGCCG | |
| | | |
| 288_C541G_CR_ | gggacggtccgtagatGTGCCCTTCCTCCCGCCG | 288 |
| 288_C541G_EF_U | GACGATGCCTTCAGCACATAGGTTTCTGTTCTAA CCTTG | |
| 288_C541G_ER_ | GGACTTTTCTCCTGACACT | |
| 288_C541G_GR_ | gctggctcggtcaagaGTGCCCTTCCTCCCGCCC | |
| | | |
| 307_C215T_CR_ | gggacggtcggtagatGAGTGGGTGCTGTTCCCG | 307 |
| 307_C215T_EF_U | GACGATGCCTTCAGCACAGTTACTGCCTCTCTGA CC | |
| 307_C215T_ER_ | AGTGTGACCTGCTCTCTT | |
| 307_C215T_TR_ | gctggctcggtcaagaGAGTGGGTGCTGTTCCCA | |
| | | |
| 384_C375G_CR_ | gggacggtcggtagatCTCATGTTGCCACTCACG | 384 |
| 384_C375G_EF_U | GACGATGCCTTCAGCACATCCGTTTCCAGGCCAT CT | |

(continued)

| Primer Name | Primer Sequence | baySNP |
|---|---|---|
| 384_C375G_ER_ | GTCCGTCTTATAGTGCAGGGTGT | |
| 384_C375G_GR_ | gctggctcggtcaagaCTCATGTTGCCACTCACC | |
| | | |
| 533_A289G_AF_ | gggacggtcggtagatCCTGGCCTGGACCCTACA | 533 |
| 533_A289G_EF_ | CTCCTGGAGACGCACGACTCT | |
| 533_A289G_ER_U | GACGATGCCTTCAGCACAACAGCATCGGGAGGTA CA | |
| 533_A289G_GF_ | gctggctcggtcaagaCCTGGCCTGGACCCTACG | |
| | | |
| 542_A402G_AR_ | gggacggtcggtagatAGAAATTCCCTCCCAACT | 542 |
| 542_A402G_EF_U | GACGATGCCTTCAGCACATGATTGAGCCAGTTGT TT | |
| 542_A402G_ER_ | GGGGTGTATTTTGAGAGTG | |
| 542_A402G_GR_ | gctggctcggtcaagaAGAAATTCCCTCCCAACC | |
| | | |
| 555_A202G_AF_ | gggacggtcggtagatGTGAGAGGGGTTCCTGGA | 555 |
| 555_A202G_EF_ | GACTATGCGGAGAAGATG | |
| 555_A202G_ER_U | GACGATGCCTTCAGCACACTTGGCTTGGAATAGA GA | |
| 555_A202G_GF_ | gctggctcggtcaagaGTGAGAGGGGTTCCTGGG | |
| | | |
| 576_C639T_CF_ | gggacggtcggtagatCATCCCTTTTGCAGGAGC | 576 |
| 576_C639T_EF_ | ACCCTTAGAATGAAGTTGAGAG | |
| 576_C639T_ER_U | GACGATGCCTTCAGCACAGCTTCGTAGACACGTT GAG | |
| 576_C639T_TF_ | gctggctcggtcaagaCATCCCTTTTGCAGGAGT | |
| | | |
| 608_A182G_AR_ | gggacggtcggtagatCCTTGGCCTCTGTGAACT | 608 |
| 608_A182G_EF_U | GACGATGCCTTCAGCACACTTATCCAGGGTGATG GT | |
| 608_A182G_ER_ | TGTCAGATCCCAGTTCAG | |
| 608_A182G_GR_ | gctggctcggtcaagaCCTTGGCCTCTGTGAACC | |
| | | |
| 614_A150G_AF_ | gggacggtcggtagatTCGCAACATCCATGCCAA | 614 |
| 614_A150G_EF_ | ACACACTTGTCCTTGTCTC | |

(continued)

| Primer Name | Primer Sequence | baySNP |
|---|---|---|
| 614_A150G_ER_U | GACGATGCCTTCAGCACAGCTCATCGTTCCTACT AAA | |
| 614_A150G_GF_ | gctggctcggtcaagaTCGCAACATCCATGCCAG | |
| | | |
| 738_A120C_AR_ | gggacggtcggtagatGAGGTGAAGAGGAATGTT | 738 |
| 738_A120C_CR_ | gctggctcggtcaagaGAGGTGAAGAGGAATGTG | |
| 738_A120C_EF_U | GACGATGCCTTCAGCACACTCCTTCTCAACGCCA CTTC | |
| 738_A120C_ER_ | CCTTCCCCCACCAACTCT | |
| | | |
| 777_C266T_CR_ | gggacggtcggtagatTCCACAGGGCCTCTCCG | 777 |
| 777_C266T_EF_U | GACGATGCCTTCAGCACAGGGGCAGGCCAGGAGG ATGTA | |
| 777_C266T_ER_ | CCTGAGACGCGGACCGT | |
| 777_C266T_TR_ | gctggctcggtcaagaTCCACAGGGCCTCTCCA | |
| | | |
| 1056_A354G_AR_ | gggacggtcggtagatGCGGCTGCCCCGTCCTGT | 1056 |
| 1056_A354G_EF_U | GACGATGCCTTCAGCACAGTGTGTCTATGTGTCT GTGTG | |
| 1056_A354G_ER_ | CGGACTTCTCCTTCTTGT | |
| 1056_A354G_GR_ | gctggctcggtcaagaGCGGCTGCCCCGTCCTGC | |
| | | |
| 1275_C307G_CF_ | gggacggtcggtagatCCTCCCGCCCTGGGAGAC | 1275 |
| 1275_C307G_EF_ | GAGAAAGAGAGAGAGAGAGAGAGAG | |
| 1275_C307G_ER_U | GACGATGCCTTCAGCACAGTGGGTTTGGTTTTGG TT | |
| 1275_C307G_GF_ | gctggctcggtcaagaCCTCCCGCCCTGGGAGAG | |
| | | |
| 1524_A284C_AF_ | gggacggtcggtagatCTCTCAAAGCCCACACAA | 1524 |
| 1524_A284C_CF_ | gctggctcggtcaagaCTCTCAAAGCCCACACAC | |
| 1524_A284C_EF_ | AGAAAAAGAAAAGGAAAAAGA | |
| 1524_A284C_ER_U | GACGATGCCTTCAGCACAGGAAAGTTACAAGGCT ATGA | |
| | | |

(continued)

| Primer Name | Primer Sequence | baySNP |
|---|---|---|
| 1583_C491T_CR_ | gggacggtcggtagatAGTTGATGGCAATGTATG | 1583 |
| 1583_C491T_EF_U | GACGATGCCTTCAGCACAGGGATGAGAACAGAGA GAA | |
| 1583_C491T_ER_ | CCAGAAAAGCACAAACAC | |
| 1583_C491T_TR_ | gctggctcggtcaagaAGTTGATGGCAATGTATA | |
| | | |
| 1669_C317T_CR_ | gggacggtcggtagatTGATAGGCGCTCAATAAG | 1669 |
| 1669_C317T_EF_U | GACGATGCCTTCAGCACATCAGATAGCAGCAAGA GG | |
| 1669_C317T_ER_ | GTGCTTCATTTTCTTCACA | |
| 1669_C317T_TR_ | gctggctcggtcaagaTGATAGGCGCTCAATAAA | |
| | | |
| 1722_C89T_CF_ | gggacggtcggtagatACCCCAGGATGCCCACAC | 1722 |
| 1722_C89T_EF_ | GTTTATCCTCCTCATGTCC | |
| 1722_C89T_ER_U | GACGATGCCTTCAGCACAGTTACCTTTTCCACCT CTC | |
| 1722_C89T_TF_ | gctggctcggtcaagaACCCCAGGATGCCCACAT | |
| | | |
| 1757_A210G_AF_ | gggacggtcggtagatGGAAACAAACCAAAATGA | 1757 |
| 1757_A210G_EF_ | CCAGCACCCAAAATAAGA | |
| 1757_A210G_ER_U | GACGATGCCTTCAGCACAATAAGTTGAAGCCCTC CC | |
| 1757_A210G_GF_ | gctggctcggtcaagaGGAAACAAACCAAAATGG | |
| | | |
| 1765_A240G_AF_ | gggacggtcggtagatGGCTTCACGGAGGAAGAA | 1765 |
| 1765_A240G_EF_ | TTAGGAGCTGTGAGGTATG | |
| 1765_A240G_ER_U | GACGATGCCTTCAGCACATAAGATGGAGCAGGGTAG | |
| 1765_A240G_GF_ | gctggctcggtcaagaGGCTTCACGGAGGAAGAG | |
| | | |
| 1837_C413T_CF_ | gggacggtcggtagatCTCAGCTTCATGCAGGGC | 1837 |
| 1837_C413T_EF_ | CCCACTCAGCCCTGCTCTT | |
| 1837_C413T_ER_U | GACGATGCCTTCAGCACAGCATCCTTGGCGGTCT TG | |
| 1837_C413T_TF_ | gctggctcggtcaagaCTCAGCTTCATGCAGGGT | |

(continued)

| Primer Name | Primer Sequence | baySNP |
|---|---|---|
| | | |
| 1862_C109T_CF_ | gggacggtcggtagatCTGGATGTGACGTCTAAC | 1862 |
| 1862_C109T_EF_ | TTGGGGGATGAAGAGGGA | |
| 1862_C109T_ER_U | GACGATGCCTTCAGCACAAATGGTGGAATGGAAG ACAGAAC | |
| 1862_C109T_TF_ | gctggctcggtcaagaCTGGATGTGACGTCTAAT | |
| | | |
| 2000_C349T_CR_ | gggacggtcggtagatAGTATGGTAATTAGGAAG | 2000 |
| 2000_C349T_EF_U | GACGATGCCTTCAGCACACTGACACTGAGCCACA AC | |
| 2000_C349T_ER_ | AACTGATGAGCAAGAAGGA | |
| 2000_C349T_TR_ | gctggctcggtcaagaAGTATGGTAATTAGGAAA | |
| | | |
| 2085_G415T_EF_ | GCTTTTTCTTTTCATTACATC | 2085 |
| 2085_G415T_ER_U | GACGATGCCTTCAGCACACCTCTTTTAGAATCAG AGACA | |
| 2085_G415T_GF_ | gggacggtcggtagatGGTAGTGTTACCAGAAAG | |
| 2085_G415T_TF_ | gctggctcggtcaagaGGTAGTGTTACCAGAAAT | |
| | | |
| 2093_C229T_CR_ | gggacggtcggtagatGGCTCTCAGCAATCAAAG | 2093 |
| 2093_C229T_EF_U | GACGATGCCTTCAGCACACTTTTGTGTTTGTGTG GG | |
| 2093_C229T_ER_ | CTGGTGTCAGGTGTAGTTATG | |
| 2093_C229T_TR_ | gctggctcggtcaagaGGCTCTCAGCAATCAAAA | |
| | | |
| 2109_A543G_AR_ | gggacggtcggtagatGCAATTCTGCAGTGACAT | 2109 |
| 2109_A543G_EF_U | GACGATGCCTTCAGCACAGCATAAAACATACCTT GAGTG | |
| 2109_A543G_ER_ | CTTCCCTACATAATAACAACAGA | |
| 2109_A543G_GR_ | gctggctcggtcaagaGCAATTCTGCAGTGACAC | |
| | | |
| 2187_C99T_CR_ | gggacggtcggtagatGAGTCCCTAAAGTTGGTG | 2187 |
| 2187_C99T_EF_U | GACGATGCCTTCAGCACACGTAAGTGCCATGAAG AC | |

(continued)

| Primer Name | Primer Sequence | baySNP |
|---|---|---|
| 2187_C99T_ER_ | AGAAAAGCCAAAGAAAGTG | |
| 2187_C99T_TR_ | gctggctcggtcaagaGAGTCCCTAAAGTTGGTA | |
| | | |
| 2203_C745T_CF_ | gggacggtcggtagatGAAGTAAATTATAGTGAC | 2203 |
| 2203_C745T_EF_ | GTGGGTTTTTGTTTTGTTT | |
| 2203_C745T_ER_U | GACGATGCCTTCAGCACATTTTTGTTGATGGGTT TG | |
| 2203_C745T_TF_ | gctggctcggtcaagaGAAGTAAATTATAGTGAT | |
| | | |
| 2217_T281G_EF_U | GACGATGCCTTCAGCACATAACCTCACCAACCTC AA | 2217 |
| 2217_T281G_ER_ | TGTCACCAATTAACCAGAA | |
| 2217_T281G_GR_ | gctggctcggtcaagaAAAATGACAGCCATGGCC | |
| 2217_T281G_TR_ | gggacggtcggtagatAAAATGACAGCCATGGCA | |
| | | |
| 2284_A220G_AF_ | gggacggtcggtagatGGAGGGCAGATCTAGGGA | 2284 |
| 2284_A220G_EF_ | TTGTAAAGGAAACCCTCCC | |
| 2284_A220G_ER_U | GACGATGCCTTCAGCACATTTTCTCCTCCCCCTT CT | |
| 2284_A220G_GF_ | gctggctcggtcaagaGGAGGGCAGATCTAGGGG | |
| | | |
| 2290_A404G_AR_ | gggacggtcggtagatCACATCAGGAAAAACAGT | 2290 |
| 2290_A404G_EF_U | GACGATGCCTTCAGCACACAGAAGTGACAAGGAT GG | |
| 2290_A404G_ER_ | GGTGCTTTGAGTGATGTT | |
| 2290_A404G_GR_ | gctggctcggtcaagaCACATCAGGAAAAACAGC | |
| | | |
| 2297_C272T_CF_ | gggacggtcggtagatAAGAAAAAGCCAATGCAC | 2297 |
| 2297_C272T_EF | TGCAGTAAGCTGAGATGG | |
| 2297_ C272T_ER_U | GACGATGCCTTCAGCACAGGAGGAGTAGGTTTTG TTTAG | |
| 2297_C272T_TF_ | gctggctcggtcaagaAAGAAAAAGCCAATGCAT | |
| | | |

(continued)

| Primer Name | Primer Sequence | baySNP |
|---|---|---|
| 2321_G516T_EF_U | GACGATGCCTTCAGCACAGGCTCGACGGGTAGGT AAC | 2321 |
| 2321_G516T_ER_ | GGGTCTTGGGTGAGGAACGAC | |
| 2321_G516T_GR_ | gggacggtcggtagatAGCCGCCGGTCCCTCTGC | |
| 2321_G516T_TR_ | gctggctcggtcaagaAGCCGCCGGTCCCTCTGA | |
| | | |
| 2354_C145T_CF_ | gggacggtcggtagatAACTAGAGAGTACTTGGC | 2354 |
| 2354_C145T_EF_ | CAGAAGCACTAGGAAGACAA | |
| 2354_C145T_ER_U | GACGATGCCTTCAGCACATCAAAGAAAACACAGC CA | |
| 23S4_C14ST_TF_ | gctggctcggtcaagaAACTAGAGAGTACTTGGT | |
| | | |
| 2371_A72C_AR_ | gggacggtcggtagatCACCTTTGTTTAAAATCT | 2371 |
| 2371_A72C_CR_ | gctggctcggtcaagaCACCTTTGTTTAAAATCG | |
| 2371_A72C_EF_U | GACGATGCCTTCAGCACACTGCGGTGTAACGGTG TC | |
| 2371_A72C_ER_ | CCCTCTCAAATTCCAGTGTTC | |
| | | |
| 2376_C302T_CR_ | gggacggtcggtagatCTGGTGGGACAGGATGTG | 2376 |
| 2376_C302T_EF_U | GACGATGCCTTCAGCACAAGAGAAAAGGAAGAAA GAA | |
| 2376_C302T_ER_ | CAGGTAACAGGAATGTATG | |
| 2376_C302T_TR_ | gctggctcggtcaagaCTGGTGGGACAGGATGTA | |
| | | |
| | | |
| 87148_A251C_EF | AAGGAATGTTGTAGAGGGA | 6734 |
| 87148_A251C_ER | AGAAAAAGAAAAGAAAAGGAG | |
| 87148_A251C_AF | gggacggtcggtagatCTTCCCAGTAGTAAATAA | |
| 87148_A251C_CF | gctggctcggtcaagaCTTCCCAGTAGTAAATAC | |

**Table 2b: Oligonucleotide primers used for genotyping using Pyrosequencing**

The baySNP number refers to an internal numbering of the CA SNPs. Primer sequences are listed for preamplification of the genomic fragments and for sequencing of the SNP using the pyrosequencing method.

(continued)

| Primer Name | Primer Sequence | baySNP |
|---|---|---|
| SP-1755FPy BIO | CCTCCATGTAGGTGCCACGG | 1755 |
| SP-1755 R Py | GGCCCTCCAGGATCTGC | |
| SP-1755 seq Py | GATGCTCACAGGGGAGAAGA | |
| | | |
| SP-8816 F Py | AGGGAGCCCACGGTGATA | 8816 |
| SP-8816 R Py BIO | AGGGCACTCTGCTTGTTATTAGA | |
| SP-8816 seq Py F | GCCCCTCTGCTCCAAGCG | |
| | | |
| SP-1653FPy: BIO | AAGTGAGGGATTTATTCATGAC | 1653 |
| SP-1653RPy: | TAAGATGCTAACTGTAGGGA | |
| SP-1653seqPy | GGAAGGCCAACTGAGGAG | |
| | | |
| 5564 FW | ACTCCCACAGGTTTAATTAGT | 5564 |
| 5564 REbio | AGACAGTGCAGGTTATCTCA | |
| 5564 SQ | ATCCTGGGAAAGGTACT | |
| | | |
| 5320 FW | TGCTAGGTGCTCGGGATA | 5320 |
| 5320 REbio | GCACTGGCCAACTTAATCACT | |
| 5320 SQ | GTAAGAGAGGTTAAGGAAAGC | |
| | | |
| 1062 FW | GAATGAGGTCCTGTGCGTTT . | 1062 |
| 1062 REbio | ACCACACTTCTCCTCGACCTG | |
| 1062 SQ | CGTTTTGACACAGAAGAT | |
| | | |
| SP10811Fint seq | TTCAGTTCCAGCTCTACCAT | 10811 |
| SP10811F | AGATTTACTGAAGTGCGAGGTG | |
| SP10811RBio | GATCAGAGGCTCACAAAGGTT | |
| | | |
| SP4383FPy | CCAGAACAAGAGTCCCTAAACCCAC | 4383 |
| SP4383RPy BIO | GGTTCAGGCACTTTTCGTTGAGTT | |
| SP4383seqPy | CTTACACAGAACGCTGCTGA | |
| | | |
| SP-3843FPy | ATTGTTTGAGGCCAGGAGGTAGAAGT | 3843 |
| SP-3843RPy BIO | GGGGGAAATTTTGAATGTAGCA | |
| SP-3843seqPy | TTGCTGTTTTCTATTTTTGGTA | |
| | | |
| SNP4527-FW | AAGCAGATAAAGCATATGATG | 4527 |

(continued)

| Primer Name | Primer Sequence | baySNP |
|---|---|---|
| SNP4527-REbio | GGGTTTCCACAAGCTGAC | |
| SNP4527-SQ | CAGAAGAAATAGATGCAAAGG | |
| | | |
| SNP5019-FWbio | TCCTTGGGTCTCACCGTGTAT | 5019 |
| SNP5019-RE | AGCGCAGAAGGGTATAG | |
| SNP5019-SQrev | CAGTCCTGTGCTGGGGAGAAC | |
| | | |
| SNP5093-FW | ATCTGGCATCTTGGCAAACCT | 5093 |
| SNP5093-REbio | CCCCACCTTCGTAACAGC | |
| SNP5093-SQ | CAGAGGCTGCCCTGAAGACAT | |
| | | |
| SNP5717-FW | AATGGCAGAAATCCGGAGA | 5717 |
| SNP5717-REbio | CCGCAAAGAGCTTGACAATGT | |
| SNP5717-SQ | GGATATGATCTAAAAAGGGA | |
| | | |
| SP5245Rint | CAAGATTGTAAAAGAATAGC | 5245 |
| SP5245FBio | TGGGGGCTGCTTAGATGCT | |
| SP5245R | GATGACGTTTCAATTTTAGGAG | |
| | | |
| SP1574F | CAATGCAGGCCAGGAAAACACT | 1574 |
| SP1574RBio | ACCCCCAAAAGACACCAAAAACA | |
| SP1574Fint | CCCTGGGCAATGAAAAAGT | |
| | | |
| SP4838Rint | TGACTAAGAATGTAATGGGGAAGA | 4838 |
| SP4838FBio | CAAAGATGACCTTATGGCTCTGA | |
| SP4838R | GTCTCGGAACATGACCTTTAGT | |
| | | |
| SP4856Fint | CTGCAGAAGCTGAGTTACC | 4856 |
| SP4856F | CGTGGTCCGGGCTCTTTTTGCT | |
| SP4856RBio | GGTGGGGGTCAGGCAGTGG | |

**Table 3: CA SNPs and putative CA genes**

The baySNP number refers to an internal numbering of the CA SNPs. Listed are the different polymorphisms found in our association study. Also accession numbers and descriptions of those gene loci are given that are most homologous to the CA genes as listed in the sequences section (see below). Homologous genes and their accession numbers could be found by those skilled in the art in the Genbank database.

(continued)

| baySNP | GT 11 | GT 12 | GT 22 | Accession | DESCRIPTION of putative CA genes |
|---|---|---|---|---|---|
| 10948 | GG | GT | TT | M10065 | Apolipoprotein E |
| 7363 | AA | AG | GG | M15887 | Endozepine (putative ligand of benzodiazepine receptor) |
| 11377 | CC | CT | TT | Z82215 | DNA from clone RP1-6802 on chromosome 22. MYH9 nonmuscle type myosin heavy chain 9 |
| 1837 | CC | CT | TT | J00098 | Apolipoprotein A-I and C-III genes |
| 10830 | AA | AG | GG | M14162 | Apolipoprotein B-100 |
| 777 | CC | CT | TT | U63721 | elastin (ELN) gene, partial cds, and LIM-kinase (LIMK1) gene |
| 555 | AA | AG | GG | U34804 | Thermostable phenol sulfotransferase (STP2), HAST |
| 11652 | CC | CT | TT | AH002776 | LDL receptor |
| 8138 | CC | CT | TT | AC002457 BAC | clone CTB-60P12 from 7q21, ABCB1 (MDR/TAP) |
| 1765 | AA | AG | GG | J05096 | Na,K-ATPase subunit alpha 2 (ATP1A2) gene |
| 5019 | AA | AT | TT | D00510 | mRNA for calphobindin II |
| 2187 | CC | CT | TT | U35464 | Protein C inhibitor (PCI-B) mRNA |
| 2000 | CC | TT | - | P03915 | NADH-UBIQUINONE OXIDOREDUCTASE CHAIN 5 (EC 1.6.5.3). |
| 11000 | CC | CT | TT | Y00839 | GAA mRNA for lysosomal alpha-glucosidase (acid maltase) |
| 5564 | GG | GT | TT | M14584 | Interleukin 6 mRNA |
| 2297 | CC | CT | - | U36478 | Fibrinogen gamma chain/ fibrinogen alpha chain genes, intergenic region. |
| 10388 | AA | AG | GG | AC005021 BAC | clone GS1-293C5 from 7q21-q22, PON2 |
| 1583 | CC | CT | TT | U33317 | Defensin 6 (HD-6) gene |
| 11001 | CC | CT | TT | Y00839 | GAA mRNA for lysosomal alpha-glucosidase (acid maltase) |
| 4856 | AG | GG | - | L11669 | Tetracycline transporter-like protein |

(continued)

| baySNP | GT 11 | GT 12 | GT 22 | Accession | DESCRIPTION of putative CA genes |
|---|---|---|---|---|---|
| 1574 | CC | CT | TT | X52889 | Gene for cardiac beta myosin heavy chain |
| 2354 | CC | CT | TT | M24736 | Endothelial leukocyte adhesion molecule 1 (ELAM-1) |
| 608 | AA | AG | GG | M94363 | Lamin B2 (LAMB2) gene and ppv 1 gene sequence. |
| 11456 | AA | AG | GG | AF051427 | Estrogen receptor beta |
| 6734 | AA | AC | - | K03021 | Tissue plasminogen activator (PLAT) gene, (elastin, LIM-kinase) |
| 5320 | AA | AG | GG | J03799 | Colin carcinoma laminin-binding protein |
| 152 | AA | AG | GG | M32670 | ITGB3 gene, intron 2, fragment C,Integrin beta 3 (GpIIIa) |
| 1755 | AA | AG | GG | U35464 | Protein C inhibitor (PCI-B) |
| 4838 | AA | AG | GG | L08246 | Myeloid cell differentiation protein (MCL1) |
| 3843 | AA | AT | TT | U12595 | Tumor necrosis factor type 1 receptor associated protein (TRAP 1) |
| 10749 | CC | CG | GG | U39487 | Xanthine dehydrogenase/ oxidase |
| 2203 | CC | CT | TT | U16752 | Cytokine SDF-1-beta |
| 1722 | CC | CT | TT | D73409 | mRNA for diacylglycerol kinase delta |
| 576 | CC | CT | - | D10667 | mRNA for smooth muscle myosin heavy chain. |
| 8168 | AA | AC | CC | Z18951 | mRNA for caveolin |
| 2109 | AA | AG | GG | Y09912 | AP-2 beta gene (transcription factor) |
| 11637 | AA | AC | CC | M19154 | transforming growth factor-beta-2 |
| 1862 | CC | CT | TT | M92357 | B94 protein mRNA |
| 11073 | CC | CG | GG | S87759 | Protein phosphatase 2C alpha |
| 1056 | AA | AG | GG | Q16720 | CALCIUM-TRANSPORTING ATPASE PLASMA MEMBRANE, (CALCIUM PUMP) |
| 5245 | AA | AG | GG | D86425 | mRNA for osteonidogen |

(continued)

| baySNP | GT 11 | GT 12 | GT 22 | Accession | DESCRIPTION of putative CA genes |
|---|---|---|---|---|---|
| 8241 | AA | AG | GG | AF165281 | ATP cassette binding transporter 1 (ABC1) |
| 5093 | AA | AG | GG | D26362 | bromodomain-containing 3, BRD3 |
| 8480 | CC | CG | GG | D83233 | PPAR gamma2 |
| 1275 | CC | CG | GG | M55913 | Tumor necrosis factor-beta (TNFB) gene |
| 118 | CC | CT | TT | X64229 | dek mRNA (oncogene) |
| 738 | AA | AC | CC | X68836 | S-adenosylmethionine synthetase |
| 8148 | AA | AG | GG | U63721 | Elastin (ELN) gene, partial cds, and LIM-kinase (LIMK1) gene |
| 1657 | CC | CT | TT | J02846 | Tissue factor gene |
| 533 | AA | AG | GG | X82895 | DLG2 (membrane protein palmitoylated 2, MPP2) |
| 11462 | GG | GT | TT | AF051427 | Estrogen receptor beta mRNA |
| 5717 | AA | AG | GG | M59941 | GM-CSF receptor beta chain mRNA |
| 1669 | CC | CT | TT | M17262 | Prothrombin (F2) gene |
| 2376 | CC | CT | - | X54516 | lipoprotein lipase |
| 11558 | AA | AC | CC | AC006312 | chromosome 9, clone hRPK.401_G_18 |
| 10785 | CC | CT | TT | D50678 | Apolipoprotein E receptor 2 |
| 2085 | GG | GT | TT | X82540 | Activin beta-C chain |
| 614 | AA | AG | GG | J04031 | Methylenetetrahydrofolate dehydrogenase-cyclohydrolase-formyltetrahydrofolate synthetas |
| 11248 | CC | CT | - | X60435 | Gene PACAP for pituitary adenylate cyclase activating polypeptide |
| 6396 | CC | CT | TT | X54807 | CYP2C8 gene for cytochrome P-450 |
| 9940 | CC | CT | TT | X54807 | CYP2C8 gene for cytochrome P-450 |
| 2353 | AA | AG | GG | AJ246000 | Leucocyte adhesion receptor, L-selectin |
| 52 | CC | CG | GG | X69907 | Gene for mitochondrial ATP synthase c subunit (P1 form) |

(continued)

| baySNP | GT 11 | GT 12 | GT 22 | Accession | DESCRIPTION of putative CA genes |
|---|---|---|---|---|---|
| 1757 | AA | AG | GG | J04046 | Calmodulin |
| 1524 | AA | AC | CC | AF223404 | WNT1 inducible signaling pathway protein 1 (WISP1) gene |
| 4912 | AA | AG | GG | AF022375 | Vascular endothelial growth factor |
| 6957 | CC | CT | TT | Y00281 | Ribophorin I |
| 8816 | CC | CG | GG | U16752 | Cytokine SDF-1-beta mRNA |
| 1062 | AA | AG | GG | AF073308 | Nonsyndromic hearing impairment protein (DFNA5), ICERE-1 |
| 2463 | CC | CT | TT | AF084225 | Cytochrome P450 2E1 (CYP2E1) mRNA, partial cds. |
| 4527 | AA | AG | GG | X76228 | Vacuolar H+ ATPase E subunit |
| 11531 | AA | AG | GG | X52773 | Retinoic acid receptor-like protein |
| 11536 | CC | CG | GG | AL022721 DNA | sequence from clone 109F14 on chromosome 6p21.2-21.3. Contains the PPARD |
| 10811 | AA | AG | GG | D86425 | Osteonidogen |
| 288 | CC | CG | GG | X79204 | SCA1 mRNA for ataxin |
| 2371 | AA | AC | CC | Q92679 | BETA-MYOSIN HEAVY CHAIN. |
| 4383 | AA | AG | GG | X58141 | mRNA for erythrocyte adducin alpha subunit |
| 11654 | AA | AG | GG | AJ276180 | ZNF202 |
| 11655 | AA | AC | CC | AJ276180 | ZNF202 |
| 11450 | AA | AT | TT | AF050163 | Lipoprotein lipase precursor, gene cds. |
| 11448 | AA | AG | GG | AF050163 | Lipoprotein lipase precursor, gene |
| 4018 | CC | CT | TT | U49260 | Mevalonate pyrophosphate decarboxylase (MPD) |
| 2217 | GG | GT | TT | M15395 | Leukocyte adhesion protein (LFA-1/Mac-1/p150,95 family) beta subunit mRNA. |
| 2321 | GG | GT | TT | M29932 | Beta-3-adrenergic receptor gene. |

(continued)

| baySNP | GT 11 | GT 12 | GT 22 | Accession | DESCRIPTION of putative CA genes |
|---|---|---|---|---|---|
| 4966 | AA | AG | GG | AF133298 | Cytochrome P450 (CYP4F8) mRNA |
| 2284 | AA | AG | GG | AB021744 | XIIIA gene for coagulation factor XIII A subunit, promoter sequence. |
| 57 | CC | CT | TT | M34175 | Beta adaptin mRNA |
| 11614 | CC | CT | TT | AF107885 | Chromosome 14q24.3 clone BAC270M14 (TGF-beta 3) gene |
| 11645 | AA | AG | GG | X05839 | Transforming growth factor-beta precursor gene exon 1 and 5'flanking region (and joined CDS) |
| 384 | CC | CG | GG | U12595 | Tumor necrosis factor type 1 receptor associated protein (TRAP1) mRNA, partial cds. |
| 542 | AA | AG | GG | M64082 | Flavin-containing monooxygenase (FMO1) mRNA |
| 2290 | AA | AG | GG | M35672 | Coagulation factor IX mRNA, partial cds. |
| 2093 | CC | CT | TT | X83543 | APXL mRNA |
| 11585 | GG | GT | TT | AC073593 | 12 BAC RP11-13J12 (Roswell Park Cancer Institute BAC Library) complete sequence. |

**Table 4: Cohorts**

| Given are names (as used in table 5) and formations of the various cohorts that were used for genotyping | |
|---|---|
| **COHORT** | **Definition** |
| HELD_ALL_GOOD/BAD | Healthy elderly individuals of both genders with good or bad serum lipid profiles (as defined in table 1) |
| HELD_FEM_GOOD/BAD | Healthy elderly individuals (female) with good or bad serum lipid profiles (as defined in table 1) |
| HELD_MAL_GOOD/BAD | Healthy elderly individuals (male) with good or bad serum lipid profiles (as defined in table 1) |
| CVD_ALL_CASE/CTRL | Individuals with diagnosis of cardiovascular disease and healthy controls (both genders) |
| CVD_FEM_CASE/CTRL | Individuals with diagnosis of cardiovascular disease and healthy controls (female) |
| CVD_MAL_CASE/CTRL | Individuals with diagnosis of cardiovascular disease and healthy controls (male) |

**Table 5: Cohort sizes and p-values of CA SNPs**

The baySNP number refers to an internal numbering of the CA SNPs. GTYPE P and A PVAL provide the p values obtained through chi square tests when comparing COHORTs A and B. For GTYPE P (p value of genotypes) the number of patients in cohort A carrying genotypes 11, 12 or 22 (FQ11 A, FQ 12 A, FQ 22 A) were compared with the respective patients in cohort B (FQ11 B, FQ 12 B, FQ 22 B) resulting in a chi square test with a 3×2 matrix. For A PVAL (p value of alleles) we compared the allele count of alleles 1 and 2 in cohorts A and B, respectively (chi square test with a 2×2 matrix). SIZE A and B: Number of patients in cohorts A and B, respectively. See table 4 for definition of COHORTs A and B.

| BAYSNP | GTYPE P | A PVAL | COHORT A | SIZE A | FQ11 A | FQ12 A | FQ22 A | COHORT B | SIZE B | FQ11 B | FQ12 B | FQ22 B |
|---|---|---|---|---|---|---|---|---|---|---|---|---|
| 10948 | 0,02 | 0,019 | HELD_ALL_BAD | 107 | 23 | 60 | 24 | HELD_ALL_GOOD | 125 | 48 | 56 | 21 |
| 7363 | 0,04 | 0,0149 | HELD_ALL_BAD | 107 | 8 | 44 | 55 | HELD_ALL_GOOD | 125 | 3 | 40 | 82 |
| 11377 | 0,0577 | 0,1103 | HELD_ALL_BAD | 107 | 12 | 62 | 33 | HELD_ALL_GOOD | 124 | 29 | 61 | 34 |
| 1837 | 0,0591 | 0,3602 | HELD_ALL_BAD | 106 | 60 | 33 | 13 | HELD_ALL_GOOD | 125 | 56 | 58 | 11 |
| 10830 | 0,0159 | 0,0036 | HELD_ALL_BAD | 105 | 22 | 47 | 36 | HELD_ALL_GOOD | 124 | 42 | 58 | 24 |
| 777 | 0,0631 | 0,0154 | HELD_ALL_BAD | 105 | 68 | 31 | 6 | HELD_ALL_GOOD | 124 | 96 | 26 | 2 |
| 555 | 0,0316 | 0,2062 | HELD_ALL_BAD | 103 | 48 | 40 | 15 | HELD_ALL_GOOD | 125 | 40 | 70 | 15 |
| 11652 | 0,0407 | 0,5098 | HELD_ALL_BAD | 104 | 24 | 59 | 21 | HELD_ALL_GOOD | 124 | 43 | 50 | 31 |
| 8138 | 0,0458 | 0,0782 | HELD_ALL_BAD | 100 | 21 | 39 | 40 | HELD_ALL_GOOD | 124 | 31 | 63 | 30 |
| 1765 | 0,093 | 0,5287 | HELD_ALL_BAD | 106 | 6 | 21 | 79 | HELD_ALL_GOOD | 118 | 3 | 37 | 78 |
| 5019 | 0,0205 | 0,0069 | HELD_ALL_BAD | 92 | 31 | 40 | 21 | HELD_ALL_GOOD | 109 | 27 | 37 | 45 |
| 2187 | 0,0113 | 0,8219 | CVD_ALL_CASE | 105 | 50 | 36 | 19 | CVD_ALL_CTRL | 74 | 29 | 40 | 5 |
| 2000 | 0,0425 | 0,0035 | CVD_ALL_CASE | 105 | 101 | 4 | 0 | CVD_ALL_CTRL | 74 | 65 | 9 | 0 |
| 11000 | 0,0041 | 1 | CVD_ALL_CASE | 104 | 3 | 38 | 63 | CVD_ALL_CTRL | 74 | 9 | 14 | 51 |
| 5564 | 0,0048 | 0,0519 | CVD_ALL_CASE | 104 | 14 | 76 | 14 | CVD_ALL_CTRL | 74 | 25 | 40 | 9 |
| 2297 | 0,0211 | 0,0222 | CVD_ALL_CASE | 104 | 103 | 1 | 0 | CVD_ALL_CTRL | 74 | 68 | 6 | 0 |
| 10388 | 0,0263 | 0,0398 | CVD_ALL_CASE | 104 | 11 | 61 | 32 | CVD_ALL_CTRL | 74 | 19 | 38 | 17 |
| 1583 | 0,0343 | 0,0398 | CVD_ALL_CASE | 104 | 3 | 20 | 81 | CVD_ALL_CTRL | 74 | 2 | 27 | 45 |

(continued)

| BAYSNP | GTYPE P | A PVAL | COHORT A | SIZE A | FQ11 A | FQ12 A | FQ22 A | COHORT B | SIZE B | FQ11 B | FQ12 B | FQ22 B |
|---|---|---|---|---|---|---|---|---|---|---|---|---|
| 11001 | 0,0116 | 0,795 | CVD_ALL_CASE | 103 | 3 | 38 | 62 | CVD_ALL_CTRL | 74 | 9 | 16 | 49 |
| 4856 | 0,0118 | 0,0123 | CVD_ALL_CASE | 103 | 0 | 103 | 0 | CVD_ALL_CTRL | 74 | 5 | 69 | 0 |
| 1574 | 0,0193 | 0,224 | CVD_ALL_CASE | 103 | 3 | 20 | 80 | CVD_ALL_CTRL | 74 | 0 | 26 | 48 |
| 2354 | 0,0406 | 0,0436 | CVD_ALL_CASE | 103 | 74 | 28 | 1 | CVD_ALL_CTRL | 74 | 64 | 9 | 1 |
| 608 | 0,0094 | 0,0027 | CVD_ALL_CASE | 103 | 2 | 21 | 80 | CVD_ALL_CTRL | 73 | 9 | 18 | 46 |
| 11456 | 0,0016 | 0,0239 | CVD_ALL_CASE | 99 | 81 | 18 | 0 | CVD_ALL_CTRL | 74 | 71 | 2 | 1 |
| 6734 | 0,0404 | 0,0462 | CVD_ALL_CASE | 102 | 87 | 15 | 0 | CVD_ALL_CTRL | 71 | 68 | 3 | 0 |
| 5320 | 0,0405 | 0,0222 | CVD_ALL_CASE | 101 | 35 | 48 | 18 | CVD_ALL_CTRL | 72 | 19 | 28 | 25 |
| 152 | 0,0552 | 0,0373 | CVD_ALL_CASE | 101 | 59 | 37 | 5 | CVD_ALL_CTRL | 71 | 34 | 26 | 11 |
| 1755 | 0,0104 | 0,8179 | CVD_ALL_CASE | 100 | 48 | 33 | 19 | CVD_ALL_CTRL | 71 | 28 | 38 | 5 |
| 4838 | 0,0432 | 0,7409 | CVD_ALL_CASE | 102 | 19 | 58 | 25 | CVD_ALL_CTRL | 69 | 21 | 26 | 22 |
| 3843 | 0,0167 | 0,0355 | CVD_ALL_CASE | 99 | 44 | 34 | 21 | CVD_ALL_CTRL | 71 | 17 | 37 | 17 |
| 10749 | 0,0647 | 0,0249 | HELD_FEM_BAD | 86 | 48 | 33 | 5 | HELD_FEM_GOOD | 83 | 33 | 39 | 11 |
| 2203 | 0,0074 | 0,0097 | HELD_FEM_BAD | 85 | 6 | 45 | 34 | HELD_FEM_GOOD | 83 | 4 | 26 | 53 |
| 1722 | 0,0308 | 0,5747 | CVD_ALL_CASE | 95 | 22 | 29 | 44 | CVD_ALL_CTRL | 73 | 12 | 37 | 24 |
| 576 | 0,0574 | 0,0585 | HELD_FEM_BAD | 85 | 85 | 0 | 0 | HELD_FEM_GOOD | 83 | 79 | 4 | 0 |
| 8168 | 0,0201 | 0,1107 | HELD_FEM_BAD | 84 | 4 | 15 | 65 | HELD_FEM_GOOD | 83 | 2 | 30 | 51 |
| 2109 | 0,0498 | 0,0224 | HELD_FEM_BAD | 85 | 64 | 20 | 1 | HELD_FEM_GOOD | 82 | 48 | 31 | 3 |
| 11637 | 0,0098 | 0,0208 | HELD_FEM_BAD | 82 | 38 | 31 | 13 | HELD_FEM_GOOD | 83 | 47 | 34 | 2 |
| 1862 | 0,0553 | 0,0288 | HELD_FEM_BAD | 85 | 48 | 34 | 3 | HELD_FEM_GOOD | 80 | 32 | 40 | 8 |
| 11073 | 0,0644 | 0,0172 | HELD_FEM_BAD | 83 | 17 | 39 | 27 | HELD_FEM_GOOD | 82 | 9 | 33 | 40 |
| 1056 | 0,0472 | 0,0613 | HELD_FEM_BAD | 83 | 33 | 36 | 14 | HELD_FEM_GOOD | 81 | 39 | 38 | 4 |
| 5245 | 0,0698 | 0,0601 | CVD_ALL_CASE | 96 | 2 | 41 | 53 | CVD_ALL_CTRL | 68 | 6 | 33 | 29 |
| 8241 | 0,0113 | 0,0082 | HELD_FEM_BAD | 81 | 64 | 17 | 0 | HELD_FEM_GOOD | 81 | 51 | 26 | 4 |
| 5093 | 0,0357 | 0,0667 | CVD_ALL_CASE | 90 | 23 | 33 | 34 | CVD_ALL_CTRL | 72 | 7 | 34 | 31 |

(continued)

| BAYSNP | GTYPE P | A PVAL | COHORT A | SIZE A | FQ11 A | FQ12 A | FQ22 A | COHORT B | SIZE B | FQ11 B | FQ12 B | FQ22 B |
|---|---|---|---|---|---|---|---|---|---|---|---|---|
| 8480 | 0,0014 | 0 | CVD_ALL_CASE | 86 | 71 | 10 | 5 | CVD_ALL_CTRL | 74 | 73 | 1 | 0 |
| 1275 | 0,0105 | 0,0004 | CVD_ALL_CASE | 81 | 27 | 23 | 31 | CVD_ALL_CTRL | 34 | 21 | 8 | 5 |
| 118 | 0,0462 | 0,0509 | CVD_MAL_CASE | 70 | 51 | 19 | 0 | CVD_MAL_CTRL | 34 | 19 | 13 | 2 |
| 738 | 0,0062 | 0,001 | CVD_MAL_CASE | 69 | 17 | 32 | 20 | CVD_MAL_CTRL | 34 | 17 | 15 | 2 |
| 8148 | 0,0126 | 0,182 | CVD_MAL_CASE | 69 | 17 | 42 | 10 | CVD_MAL_CTRL | 34 | 9 | 12 | 13 |
| 1657 | 0,0382 | 0,8798 | CVD_MAL_CASE | 70 | 22 | 39 | 9 | CVD_MAL_CTRL | 33 | 14 | 10 | 9 |
| 533 | 0,0095 | 0,0038 | CVD_MAL_CASE | 68 | 3 | 27 | 38 | CVD_MAL_CTRL | 34 | 0 | 5 | 29 |
| 11462 | 0,0488 | 0,06 | CVD_MAL_CASE | 68 | 53 | 15 | 0 | CVD_MAL_CTRL | 34 | 32 | 2 | 0 |
| 5717 | 0,0245 | 0,0646 | CVD_MAL_CASE | 67 | 3 | 40 | 24 | CVD_MAL_CTRL | 33 | 7 | 18 | 8 |
| 1669 | 0,0068 | 0,236 | CVD_MAL_CASE | 64 | 4 | 11 | 49 | CVD_MAL_CTRL | 34 | 0 | 15 | 19 |
| 2376 | 0,0473 | 0,0511 | CVD_MAL_CASE | 69 | 66 | 3 | 0 | CVD_MAL_CTRL | 29 | 24 | 5 | 0 |
| 11558 | 0,0168 | 0,084 | HELD_ALL_CASE | 49 | 42 | 5 | 2 | HELD_ALL_CTRL | 43 | 28 | 14 | 1 |
| 10785 | 0,0344 | 0,0442 | HELD_ALL_CASE | 49 | 0 | 5 | 44 | HELD_ALL_CTRL | 43 | 0 | 12 | 31 |
| 2085 | 0,0518 | 0,0503 | HELD_ALL_CASE | 49 | 19 | 27 | 3 | HELD_ALL_CTRL | 43 | 11 | 22 | 10 |
| 614 | 0,0045 | 0,0083 | HELD_ALL_CASE | 48 | 9 | 17 | 22 | HELD_ALL_CTRL | 43 | 6 | 4 | 33 |
| 11248 | 0,0089 | 0,0351 | HELD_ALL_CASE | 48 | 30 | 18 | 0 | HELD_ALL_CTRL | 43 | 15 | 28 | 0 |
| 6396 | 0,0309 | 0,0387 | HELD_ALL_CASE | 49 | 1 | 5 | 43 | HELD_ALL_CTRL | 42 | 1 | 13 | 28 |
| 9940 | 0,0337 | 0,0419 | HELD_ALL_CASE | 48 | 42 | 5 | 1 | HELD_ALL_CTRL | 43 | 29 | 13 | 1 |
| 2353 | 0,0065 | 0,1234 | HELD_ALL_CASE | 49 | 43 | 3 | 3 | HELD_ALL_CTRL | 41 | 28 | 12 | 1 |
| 52 | 0,0207 | 0,0039 | HELD_ALL_CASE | 48 | 13 | 22 | 13 | HELD_ALL_CTRL | 42 | 23 | 14 | 5 |
| 1757 | 0,0223 | 0,5028 | HELD_ALL_CASE | 49 | 7 | 16 | 26 | HELD_ALL_CTRL | 40 | 0 | 20 | 20 |
| 1524 | 0,0509 | 0,0235 | HELD_ALL_CASE | 48 | 1 | 15 | 32 | HELD_ALL_CTRL | 41 | 6 | 15 | 20 |
| 4912 | 0,0153 | 0,0285 | CVD_MAL_CASE | 58 | 3 | 25 | 30 | CVD_MAL_CTRL | 30 | 8 | 10 | 12 |
| 6957 | 0,0613 | 0,0218 | HELD_ALL_CASE | 45 | 5 | 20 | 20 | HELD_ALL_CTRL | 43 | 11 | 22 | 10 |
| 8816 | 0,0418 | 0,0055 | CVD_MAL_CASE | 60 | 9 | 16 | 35 | CVD_MAL_CTRL | 27 | 1 | 3 | 23 |

(continued)

| BAYSNP | GTYPE P | A PVAL | COHORT A | SIZE A | FQ11 A | FQ12 A | FQ22 A | COHORT B | SIZE B | FQ11 B | FQ12 B | FQ22 B |
|---|---|---|---|---|---|---|---|---|---|---|---|---|
| 1062 | 0,0465 | 0,0345 | CVD_MAL_CASE | 56 | 0 | 10 | 46 | CVD_MAL_CTRL | 31 | 1 | 11 | 19 |
| 2463 | 0,0187 | 0,0217 | HELD_ALL_CASE | 49 | 0 | 8 | 41 | HELD_ALL_CTRL | 37 | 0 | 0 | 37 |
| 4527 | 0,001 | 0,4255 | CVD_MAL_CASE | 51 | 0 | 23 | 28 | CVD_MAL_CTRL | 25 | 5 | 5 | 15 |
| 11531 | 0,0345 | 0,0622 | CVD_FEM_CASE | 36 | 0 | 18 | 18 | CVD_FEM_CTRL | 39 | 0 | 10 | 29 |
| 11536 | 0,0455 | 0,0272 | CVD_FEM_CASE | 35 | 20 | 13 | 2 | CVD_FEM_CTRL | 40 | 32 | 8 | 0 |
| 10811 | 0,0515 | 0,0354 | CVD_FEM_CASE | 34 | 21 | 11 | 2 | CVD_FEM_CTRL | 40 | 34 | 5 | 1 |
| 288 | 0,04 | 0,0437 | CVD_FEM_CASE | 33 | 1 | 18 | 14 | CVD_FEM_CTRL | 39 | 0 | 12 | 27 |
| 2371 | 0,068 | 0,0432 | CVD_FEM_CASE | 33 | 11 | 19 | 3 | CVD_FEM_CTRL | 39 | 23 | 15 | 1 |
| 4383 | 0,0646 | 0,0594 | CVD_FEM_CASE | 34 | 0 | 9 | 25 | CVD_FEM_CTRL | 36 | 1 | 17 | 18 |
| 11654 | 0,009 | 0,0434 | HELD_MAL_BAD | 21 | 1 | 17 | 3 | HELD_MAL_GOOD | 42 | 2 | 18 | 22 |
| 11655 | 0,009 | 0,0434 | HELD_MAL_BAD | 21 | 3 | 17 | 1 | HELD_MAL_GOOD | 42 | 22 | 18 | 2 |
| 11450 | 0,03 | 0,0046 | HELD_MAL_BAD | 21 | 4 | 9 | 8 | HELD_MAL_GOOD | 42 | 2 | 10 | 30 |
| 11448 | 0,0176 | 0,0076 | HELD_MAL_BAD | 21 | 2 | 9 | 10 | HELD_MAL_GOOD | 41 | 2 | 5 | 34 |
| 4018 | 0,0245 | 0,0071 | HELD_MAL_BAD | 20 | 0 | 6 | 14 | HELD_MAL_GOOD | 42 | 6 | 21 | 15 |
| 2217 | 0,0124 | 0,0211 | HELD_MAL_BAD | 21 | 11 | 10 | 0 | HELD_MAL_GOOD | 40 | 34 | 6 | 0 |
| 2321 | 0,0278 | 0,0335 | HELD_MAL_BAD | 20 | 14 | 6 | 0 | HELD_MAL_GOOD | 41 | 38 | 3 | 0 |
| 4966 | 0,0158 | 0,0114 | HELD_MAL_BAD | 20 | 0 | 11 | 9 | HELD_MAL_GOOD | 40 | 9 | 24 | 7 |
| 2284 | 0,0416 | 0,0114 | HELD_MAL_BAD | 17 | 0 | 2 | 15 | HELD_MAL_GOOD | 41 | 3 | 16 | 22 |
| 57 | 0,0065 | 0,0063 | HELD_FEM_CASE | 33 | 16 | 16 | 1 | HELD_FEM_CTRL | 23 | 20 | 3 | 0 |
| 11614 | 0,0531 | 0,0634 | HELD_FEM_CASE | 33 | 0 | 10 | 23 | HELD_FEM_CTRL | 23 | 4 | 6 | 13 |
| 11645 | 0,0533 | 0,0623 | HELD_FEM_CASE | 33 | 0 | 2 | 31 | HELD_FEM_CTRL | 23 | 0 | 6 | 17 |
| 384 | 0,0618 | 0,0212 | HELD_FEM_CASE | 33 | 13 | 15 | 5 | HELD_FEM_CTRL | 23 | 3 | 12 | 8 |
| 542 | 0,0003 | 0,0001 | HELD_MAL_CASE | 16 | 2 | 8 | 6 | HELD_MAL_CTRL | 20 | 0 | 1 | 19 |
| 2290 | 0,0298 | 0,0007 | HELD_MAL_CASE | 16 | 10 | 0 | 6 | HELD_MAL_CTRL | 20 | 19 | 0 | 1 |
| 2093 | 0,0301 | 0,0043 | HELD_MAL_CASE | 16 | 9 | 1 | 6 | HELD_MAL_CTRL | 20 | 18 | 0 | 2 |

(continued)

| BAYSNP | GTYPE P | A PVAL | COHORT A | SIZE A | FQ11 A | FQ12 A | FQ22 A | COHORT B | SIZE B | FQ11 B | FQ12 B | FQ22 B |
|---|---|---|---|---|---|---|---|---|---|---|---|---|
| 11585 | 0,0533 | 0,0508 | HELD_MAL_CASE | 16 | 8 | 8 | 0 | HELD_MAL_CTRL | 20 | 6 | 8 | 6 |

**Table 6: Correlation of genotypes of CA SNPs to relative risk**

| For diagnostic conclusions to be drawn from genotyping a particular patient we calculated the relative risk RR1, RR2, RR3 for the three possible genotypes of each SNP. Given the genotype frequencies as | | | |
|---|---|---|---|
| | gtype1 | gtype2 | gtype3 |
| case | N11 | N12 | N13 |
| control | N21 | N22 | N23 |

we calculate

$$RR1 = \frac{N11}{N21} \bigg/ \frac{N12 + N13}{N22 + N23}$$

$$RR2 = \frac{N12}{N22} \bigg/ \frac{N11 + N13}{N21 + N23}$$

$$RR3 = \frac{N13}{N23} \bigg/ \frac{N11 + N12}{N21 + N22}$$

Here, the *case* and *control* populations represent any case-control-group pair, or bad(case)-good(control)-group pair, respectively. A value RR1>1, RR2>1, and RR3>1 indicates an increased risk for individuals carrying genotype 1, genotype 2, and genotype 3, respectively. For example, RR1=3 indicates a 3-fold risk of an individual carrying genotype 1 as compared to individuals carrying genotype 2 or 3 (a detailed description of relative risk calculation and statistics can be found in (Biostatistics, L. D. Fisher and G. van Belle, Wiley Interscience 1993)). The baySNP number refers to an internal numbering of the CA SNPs and can be found in the sequence listing.

| BAYSNP | GTYPE1 | GTYPE2 | GTYPE3 | RR1 | RR2 | RR3 | FRE01 A | FRE02 A | FRE03 A | FRE01 B | FRE02 B | FRE03 B |
|---|---|---|---|---|---|---|---|---|---|---|---|---|
| 52 | CC | CG | GG | 0,56 | 1,27 | 1,49 | 13 | 22 | 13 | 23 | 14 | 5 |
| 57 | CC | CT | TT | 0,52 | 1,83 | 1,72 | 16 | 16 | 1 | 20 | 3 | 0 |
| 118 | CC | CT | TT | 1,3 | 0,84 | 0 | 51 | 19 | 0 | 19 | 13 | 2 |
| 152 | AA | AG | GG | 1,19 | 1 | 0,51 | 59 | 37 | 5 | 34 | 26 | 11 |
| 288 | CC | CG | GG | 2,22 | 1,68 | 0,56 | 1 | 18 | 14 | 0 | 12 | 27 |
| 384 | CC | CG | GG | 1,63 | 0,9 | 0,59 | 13 | 15 | 5 | 3 | 12 | 8 |
| 533 | AA | AG | GG | 1,52 | 1,44 | 0,66 | 3 | 27 | 38 | 0 | 5 | 29 |
| 542 | AA | AG | GG | 2,43 | 3 | 0,28 | 2 | 8 | 6 | 0 | 1 | 19 |
| 555 | AA | AG | GG | 1,39 | 0,68 | 1,13 | 48 | 40 | 15 | 40 | 70 | 15 |
| 608 | AA | AG | GG | 0,3 | 0,9 | 1,38 | 2 | 21 | 80 | 9 | 18 | 46 |
| 614 | AA | AG | GG | 1,17 | 1,83 | 0,55 | 9 | 17 | 22 | 6 | 4 | 33 |
| 738 | AA | AC | CC | 0,66 | 1,03 | 1,5 | 17 | 32 | 20 | 17 | 15 | 2 |
| 777 | CC | CT | TT | 0,73 | 1,26 | 1,67 | 68 | 31 | 6 | 96 | 26 | 2 |
| 1056 | AA | AG | GG | 0,84 | 0,93 | 1.65 | 33 | 36 | 14 | 39 | 38 | 4 |
| 1062 | AA | AG | GG | 0 | 0,68 | 1,56 | 0 | 10 | 46 | 1 | 11 | 19 |
| 1275 | CC | CG | GG | 0,7 | 1,07 | 1,36 | 27 | 23 | 31 | 21 | 8 | 5 |
| 1524 | AA | AC | CC | 0,25 | 0,89 | 1,42 | 1 | 15 | 32 | 6 | 15 | 20 |
| 1574 | CC | CT | TT | 1,74 | 0,69 | 1,33 | 3 | 20 | 80 | 0 | 26 | 48 |
| 1583 | CC | CT | TT | 1,03 | 0.66 | 1,45 | 3 | 20 | 81 | 2 | 27 | 45 |
| 1657 | CC | CT | TT | 0,85 | 1,39 | 0,7 | 22 | 39 | 9 | 14 | 10 | 9 |
| 1669 | CC | CT | TT | 1,57 | 0,57 | 1,44 | 4 | 11 | 49 | 0 | 15 | 19 |
| 1722 | CC | CT | TT | 1,19 | 0,68 | 1,27 | 22 | 29 | 44 | 12 | 37 | 24 |
| 1755 | AA | AG | GG | 1,15 | 0,69 | 1,44 | 48 | 33 | 19 | 28 | 38 | 5 |
| 1757 | AA | AG | GG | 1,95 | 0,71 | 1,06 | 7 | 16 | 26 | 0 | 20 | 20 |
| 1765 | AA | AG | GG | 1.43 | 0,71 | 1,25 | 6 | 21 | 79 | 3 | 37 | 78 |

(continued)

| BAYSNP | GTYPE1 | GTYPE2 | GTYPE3 | RR1 | RR2 | RR3 | FRE01 A | FRE02 A | FRE03 A | FRE01 B | FRE02 B | FRE03 B |
|---|---|---|---|---|---|---|---|---|---|---|---|---|
| 1837 | CC | CT | TT | 1,29 | 0,7 | 1,21 | 60 | 33 13 | | 56 | 58 | 11 |
| 1862 | CC | CT | TT | 1,38 | 0,82 | 0,51 | 48 | 34 | 3 | 32 | 40 | 8 |
| 2000 | CC | TT | | 1,98 | 0,51 | 0 | 101 | 4 | 0 | 65 | 9 | 0 |
| 2085 | GG | GT | TT | 1,31 | 1,08 | 0,4 | 19 | 27 | 3 | 11 | 22 | 10 |
| 2093 | CC | CT | TT | 0,43 | 2,33 | 2,1 | 9 | 1 | 6 | 18 | 0 | 2 |
| 2109 | AA | AG | GG | 1,5 | 0,7 | 0,49 | 64 | 20 | 1 | 48 | 31 | 3 |
| 2187 | CC | CT | TT | 1,15 | 0,71 | 1,43 | 50 | 36 | 19 | 29 | 40 | 5 |
| 2203 | CC | CT | TT | 1,2 | 1,54 | 0,62 | 6 | 45 | 34 | 4 | 26 | 53 |
| 2217 | GG | GT | TT | 0,39 | 2,56 | 0 | 11 | 10 | 0 | 34 | 6 | 0 |
| 2284 | AA | AG | GG | 0 | 0.3 | 4.26 | 0 | 2 | 15 | 3 | 16 | 22 |
| 2290 | AA | AG | GG | 0.4 | 0 | 2,49 | 10 | 0 | 6 | 19 | 0 | 1 |
| 2297 | CC | CT | | 4.22 | 0.24 | 0 | 103 | 1 | 0 | 68 | 6 | 0 |
| 2321 | GG | GT | TT | 0,4 | 2,48 | 0 | 14 | 6 | 0 | 38 | 3 | 0 |
| 2353 | AA | AG | GG | 1,92 | 0,33 | 1,4 | 43 | 3 | 3 | 28 | 12 | 1 |
| 2354 | CC | CT | TT | 0,72 | 1,41 | 0,86 | 74 | 28 | 1 | 64 | 9 | 1 |
| 2371 | AA | AC | CC | 0.56 | 1.52 | 1,7 | 11 | 19 | 3 | 23 | 15 | 1 |
| 2376 | CC | CT | | 1,96 | 0,51 | 0 | 66 | 3 | 0 | 24 | 5 | 0 |
| 2463 | CC | CT | TT | 0 | 1,9 | 0,53 | 0 | 8 | 41 | 0 | 0 | 37 |
| 3843 | AA | AT | TT | 1,43 | 0,73 | 0,94 | 44 | 34 | 21 | 17 | 37 | 17 |
| 4018 | CC | CT | TT | 0 | 0,56 | 2,66 | 0 | 6 | 14 | 6 | 21 | 15 |
| 4383 | AA | AG | GG | 0 | 0,61 | 1,74 | 0 | 9 | 25 | 1 | 17 | 18 |
| 4527 | AA | AG | GG | 0 | 1,41 | 0,93 | 0 | 23 | 28 | 5 | 5 | 15 |
| 4838 | AA | AG | GG | 0,75 | 1,37 | 0,86 | 19 | 58 | 25 | 21 | 26 | 22 |
| 4887 | AA | AC | CC | 0 | 0,36 | 3,1 | 0 | 3 | 13 | 1 | 11 | 8 |
| 4912 | AA | AG | GG | 0,38 | 1,15 | 1,17 | 3 | 25 | 30 | 8 | 10 | 12 |

(continued)

| BAYSNP | GTYPE1 | GTYPE2 | GTYPE3 | RR1 | RR2 | RR3 | FRE01 A | FRE02 A | FRE03 A | FRE01 B | FRE02 B | FRE03 B |
|---|---|---|---|---|---|---|---|---|---|---|---|---|
| 4966 | AA | AG | GG | 0 | 0,87 | 2,25 | 0 | 11 | 9 | 9 | 24 | 7 |
| 5019 | AA | AT | TT | 1,25 | 1,24 | 0,6 | 31 | 40 | 21 | 27 | 37 | 45 |
| 5093 | AA | AG | GG | 1,51 | 0,82 | 0,91 | 23 | 33 | 34 | 7 | 34 | 31 |
| 5245 | AA | AG | GG | 0,41 | 0,91 | 1,23 | 2 | 41 | 53 | 6 | 33 | 29 |
| 5320 | AA | AG | GG | 1,17 | 1,16 | 0,66 | 35 | 48 | 18 | 19 | 28 | 25 |
| 5564 | GG | GT | TT | 0,55 | 1,45 | 1,05 | 14 | 76 | 14 | 25 | 40 | 9 |
| 5717 | AA | AG | GG | 0.42 | 1.07 | 1,19 | 3 | 40 | 24 | 7 | 18 | 8 |
| 6396 | CC | CT | TT | 0,93 | 0,46 | 2.02 | 1 | 5 | 43 | 1 | 13 | 28 |
| 6734 | AA | AC | | 0.67 | 1,48 | 0 | 87 | 15 | 0 | 68 | 3 | 0 |
| 6957 | CC | CT | TT | 0.56 | 0.88 | 1.55 | 5 | 20 | 20 | 11 | 22 | 10 |
| 7363 | AA | AG | GG | 1,62 | 1,23 | 0,73 | 8 | 44 | 55 | 3 | 40 | 82 |
| 8138 | CC | CT | TT | 0.88 | 0.76 | 1,47 | 21 | 39 | 40 | 31 | 63 | 30 |
| 8148 | AA | AG | GG | 0,97 | 1.41 | 0.59 | 17 | 42 | 10 | 9 | 12 | 13 |
| 8168 | AA | AC | CC | 1,34 | 0,59 | 1,5 | 4 | 15 | 65 | 2 | 30 | 51 |
| 8241 | AA | AG | GG | 1,54 | 0,74 | 0 | 64 | 17 | 0 | 51 | 26 | 4 |
| 8480 | CC | CG | GG | 0,53 | 1,78 | 1,91 | 71 | 10 | 5 | 73 | 1 | 0 |
| 8816 | CC | CG | GG | 1,36 | 1,3 | 0,7 | 9 | 16 | 35 | 1 | 3 | 23 |
| 9940 | CC | CT | TT | 1,97 | 0.47 | 0.95 | 42 | 5 | 1 | 29 | 13 | 1 |
| 10388 | AA | AG | GG | 0,58 | 1,13 | 1,17 | 11 | 61 | 32 | 19 | 38 | 17 |
| 10749 | CC | CG | GG | 1,37 | 0,84 | 0,59 | 48 | 33 | 5 | 33 | 39 | 11 |
| 10785 | CC | CT | TT | 0 | 0,5 | 1,99 | 0 | 5 | 44 | 0 | 12 | 31 |
| 10811 | AA | AG | GG | 0,56 | 1,73 | 1,48 | 21 | 11 | 2 | 34 | 5 | 1 |
| 10830 | AA | AG | GG | 0,68 | 0,86 | 1,47 | 22 | 47 | 36 | 42 | 58 | 24 |
| 10948 | GG | GT | TT | 0,62 | 1,28 | 1,2 | 23 | 60 | 24 | 48 | 56 | 21 |
| 11000 | CC | CT | TT | 0,41 | 1,4 | 0,88 | 3 | 38 | 63 | 9 | 14 | 51 |

(continued)

| BAYSNP | GTYPE1 | GTYPE2 | GTYPE3 | RR1 | RR2 | RR3 | FRE01 A | FRE02 A | FRE03 A | FRE01 B | FRE02 B | FRE03 B |
|---|---|---|---|---|---|---|---|---|---|---|---|---|
| 11001 | CC | CT | TT | 0,41 | 1,33 | 0,9 | 3 | 38 | 62 | 9 | 16 | 49 |
| 11073 | CC | CG | GG | 1,38 | 1,14 | 0,71 | 17 | 39 | 27 | 9 | 33 | 40 |
| 11248 | CC | CT | | 1,7 | 0,59 | 0 | 30 | 18 | 0 | 15 | 28 | 0 |
| 11377 | CC | CT | TT | 0,59 | 1,21 | 1,09 | 12 | 62 | 33 | 29 | 61 | 34 |
| 11448 | AA | AG | GG | 1,53 | 2,57 | 0,37 | 2 | 9 | 10 | 2 | 5 | 34 |
| 11450 | AA | AT | TT | 2,24 | 1,74 | 0,4 | 4 | 9 | 8 | 2 | 10 | 30 |
| 11456 | AA | AG | GG | 0.62 | 1,7 | 0 | 81 | 18 | 0 | 71 | 2 | 1 |
| 11462 | GG | GT | TT | 0,74 | 1,35 | 0 | 53 | 16 | 0 | 32 | 3 | 0 |
| 11531 | AA | AG | GG | 0 | 1,68 | 0,8 | 0 | 18 | 18 | 0 | 10 | 29 |
| 11536 | CC | CG | GG | 0,59 | 1,52 | 2,21 | 20 | 13 | 2 | 32 | 8 | 0 |
| 11558 | AA | AC | CC | 1,89 | 0,44 | 1,26 | 42 | 5 | 2 | 28 | 14 | 1 |
| 11585 | GG | GT | TT | 1,57 | 1,25 | 0 | 8 | 8 | 0 | 6 | 8 | 6 |
| 11614 | CC | CT | TT | 0 | 1,09 | 1.28 | 0 | 10 | 23 | 4 | 6 | 13 |
| 11637 | AA | AC | CC | 0,81 | 0,94 | 1.88 | 38 | 31 | 13 | 47 | 34 | 2 |
| 11645 | AA | AG | GG | 0 | 0,39 | 2,58 | 0 | 2 | 31 | 0 | 6 | 17 |
| 11652 | CC | CT | TT | 0.72 | 1.43 | 0.86 | 24 | 59 | 21 | 43 | 50 | 31 |
| 11654 | AA | AG | GG | 1 | 3,4 | 0,25 | 1 | 17 | 3 | 2 | 18 | 22 |
| 11655 | AA | AC | CC | 0,25 | 3.4 | 1 | 3 | 17 | 1 | 22 | 18 | 2 |

## Claims

1. An isolated polynucleotide encoded by a cardiovascular associated (CA) gene; the polynucleotide is selected from the group comprising
SEQ ID 49, 33, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 34, 35, 36, 37, 38, 39, 40, 41, 42, 43, 44, 45, 46, 47, 48, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 with allelic variation as indicated in the sequences section contained in a functional surrounding like full length cDNA for CA gene polypeptide and with or without the CA gene promoter sequence.

2. An expression vector containing one or more of the polynucleotides of claim 1.

3. A host cell containing the expression vector of claim 2.

4. A substantially purified CA gene polypeptide encoded by a polynucleotide of claim 1.

5. A method for producing a CA gene polypeptide, wherein the method comprises the following steps:

    a) culturing the host cell of claim 3 under conditions suitable for the expression of the CA gene polypeptide; and
    b) recovering the CA gene polypeptide from the host cell culture.

6. A method for the detection of a polynucleotide of claim 1 or a CA gene polypeptide of claim 4 comprising the steps of:

    contacting a biological sample with a reagent which specifically interacts with the polynucleotide or the CA gene polypeptide.

7. A method of screening for agents which regulate the activity of a CA gene comprising the steps of:

    contacting a test compound with a CA gene polypeptide encoded by any polynucleotide of claim 1; and detecting CA gene activity of the polypeptide, wherein a test compound which increases the CA gene polypeptide activity is identified as a potential therapeutic agent for increasing the activity of the CA gene polypeptide and wherein a test compound which decreases the CA activity of the polypeptide is identified as a potential therapeutic agent for decreasing the activity of the CA gene polypeptide.

8. A reagent that modulates the activity of a CA polypeptide or a polynucleotide wherein said reagent is identified by the method of the claim 7.

9. A pharmaceutical composition, comprising:

    the expression vector of claim 2 or the reagent of claim 8 and a pharmaceutically acceptable carrier.

10. Use of the reagent according to claim 8 for the preparation of a medicament.

11. A method for determining whether a human subject has, or is at risk of developing a cardiovascular disease, comprising determining the identity of nucleotide variations as indicated in the sequences section of SEQ ID 49,33, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 34, 35, 36, 37, 38, 39, 40, 41, 42, 43, 44, 45, 46, 47, 48, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 of the CA gene locus of the subject; whereas a "risk" genotype has a risk ratio of greater than 1 as can be seen from table 6.

12. A kit for assessing cardiovascular status, said kit comprising

    a) sequence determination primers and
    b) sequence determination reagents,

    wherein said primers are selected from the group comprising primers that hybridize to polymorphic positions in human CA genes according to claim 1; and primers that hybridize immediately adjacent to polymorphic positions in human CA genes according to claim 1.

**13.** A kit as defined in claims 12 detecting a combination of two or more, up to all, polymorphic sites selected from the groups of sequences as defined in claim 1.

**14.** A kit for assessing cardiovascular status, said kit comprising one or more antibodies specific for a polymorphic position defined in claim 1 within the human CA gene polypeptides and combinations of any of the foregoing.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5547835 A
- WO 9416101 A
- WO 9421822 A
- US 5605798 A
- US 9603651 W
- US 5580732 A
- US 5571676 A
- US 4998617 A
- US 5593826 A
- US 4656127 A, Mundy, C. R
- FR 2650840, Cohen, D.
- WO 9102087 A
- GB 9215712 W, Goelet, P.
- WO 9107660 A, Bianchi
- US 4341761 A
- US 4399121 A
- US 4427783 A
- US 4444887 A
- US 4466917 A
- US 4472500 A
- US 4491632 A
- US 4493890 A
- WO 9813523 A
- WO 9727325 A


### Non-patent literature cited in the description


- **ROSS.** *Nature,* 1993, vol. 362, 801-809
- **LUSIS, A. J.** *Nature,* 2000, vol. 407, 233-241
- **HWANG et al.** *Circulation,* 1997, vol. 96, 4146-4203
- **GLASS et al.** *Cell,* 2001, vol. 104, 503-516
- **MELANSON et al.** *Cardiol Rev,* 2001, vol. 9, 202-207
- **DUCKWORTH.** *Curr Atheroscler Rep,* 2001, vol. 3, 383-391
- **SAFAR.** *Curr Opin Cardiol,* 2000, vol. 15, 258-263
- **SOWERS et al.** *Hypertension,* 2001, vol. 37, 1053-1059
- **MARLAR ; GRIFFIN.** *J. Clin. Invest,* 1980, vol. 66, 1186-1189
- **RINKENBERGER et al.** *Genes Dev,* 2000, vol. 14, 23-27
- **MOSER et al.** *Genes Dev,* vol. 11, 1938-1948
- **REICH D.E. et al.** *Nature,* 2001, vol. 411, 199-204
- **CRONIN et al.** *Human Mutation,* 1996, vol. 7, 244
- **KOZAL et al.** *Nature Medicine,* 1996, vol. 2, 753
- **GUATELLI, J. C. et al.** *Proc. Natl. Acad. Sci. U.S.A.,* 1990, vol. 87, 1874-1878
- **KWOH, D. Y. et al.** *Proc. Natl. Acad. Sci. U.S.A.,* 1989, vol. 86, 1173-1177
- **LIZARDI, P. M. et al.** *Bio/Technology,* 1988, vol. 6, 1197
- **MAXAM ; GILBERT.** *Proc. Natl Acad Sci USA,* 1977, vol. 74, 560
- **SANGER et al.** *Proc. Nat. Acad. Sci,* 1977, vol. 74, 5463
- *Biotechniques,* 1995, vol. 19, 448
- **COHEN et al.** *Adv Chromatogr,* 1996, vol. 36, 127-162
- **GRIFFIN et al.** *Appl Biochem Biotechnol,* 1993, vol. 38, 147-159
- **ORITA et al.** *Proc Natl. Acad. Sci USA,* 1989, vol. 86, 2766
- **COTTON.** *Mutat Res,* 1993, vol. 285, 125-144
- **HAYASHI.** *Genet Anal Tech Appl,* 1992, vol. 9, 73-79
- **KEEN et al.** *Trends Genet,* 1991, vol. 7, 5
- **MYERS et al.** *Nature,* 1985, vol. 313, 495
- **ROSENBAUM ; REISSNER.** *Biophys Chem,* 1987, vol. 265, 1275
- **SAIKI et al.** *Nature,* 1986, vol. 324, 163
- **SAIKI et al.** *Proc. Natl Acad. Sci USA,* 1989, vol. 86, 6230
- **WALLACE et al.** *Nucl. Acids Res,* 1979, vol. 6, 3543
- **GIBBS et al.** *Nucleic Acids Res.,* 1989, vol. 17, 2437-2448
- **PROSSNER.** *Tibtech,* 1993, vol. 11, 238
- **NEWTON et al.** *Nucl. Acids Res,* 1989, vol. 17, 2503
- **GASPARINI et al.** *Mol. Cell Probes,* 1992, vol. 6, 1
- **LANDEGREN, U. et al.** *Science,* 1988, vol. 241, 1077-1080
- **NICKERSON, D. A. et al.** *Proc. Natl. Acad. Sci. (U.S.A.,* 1990, vol. 87, 8923-8927

- **TOBE et al.** *Nucleic Acids Res,* 1996, vol. 24, 3728
- **KOMHER, J. S. et al.** *Nucl. Acids. Res,* 1989, vol. 17, 7779-7784
- **SOKOLOV, B. P.** *Nucl. Acids Res,* 1990, vol. 18, 3671
- **SYVANEN, A. -C. et al.** *Genomics,* 1990, vol. 8, 684-692
- **KUPPUSWAMY, M. N. et al.** *Proc. Natl. Acad. Sci. (U.S.A.,* 1991, vol. 88, 1143-1147
- **PREZANT, T. R. et al.** *Hum. Mutat,* 1992, vol. 1, 159-164
- **UGOZZOLI, L. et al.** *GATA,* vol. 9, 107-112
- **NYREN, P. et al.** *Anal. Biochem.,* 1993, vol. 208, 171-175
- **SYVANEN, A.-C. et al.** *Amer. J. Hum. Genet.,* 1993, vol. 52, 46-59
- **ACTON et al.** *Science,* 1999, vol. 271, 518
- **SAMBROOK et al.** Molecular Cloning: A Laboratory Manual. Cold Spring Harbor Laboratory Press, 1989
- DNA Cloning: A Practical Approach. 1985, vol. I-II
- Oligonucleotide Synthesis. 1984
- Nucleic Acid Hybridization. 1985
- **AUSUBEL et al.** Current Protocols in Molecular Biology. John Wiley and Sons, 1997
- Methods in Enzymology. vol. 154-155
- **SAIKI et al.** *Science,* 1988, vol. 239, 48
- Immunochemical Methods in Cell and Molecular Biology. Academic Press, 1987
- **SCOPES.** Protein Purification: Principles and Practice. Springer-Verlag, 1987
- Handbook of Experimental Immunology. 1986, vol. I-IV
- **MERRIFIELD.** *J. Am. Chem. Soc.,* 1963, vol. 85, 2149
- **MAYER ; WALKER.** Immunochemical Methods in Cell and Molecular Biology. Academic Press, 1987
- The Art of Antibody Purification. Amicon Division. W. R. Grace & Co, 1989
- General protein purification methods are described. Protein Purification: Principles and Practice. Springer-Verlag, 1987
- **COFFMAN.** *Semin. Nephrol,* 1997, vol. 17, 404
- **ESTHER et al.** *Lab. Invest,* 1996, vol. 74, 953
- **MURAKAMI et al.** *Blood Press,* 1996, vol. 2, 36
- **L. D. FISHER ; G. VAN BELLE.** Biostatistics. Wiley Interscience, 1993

SEQUENCE LISTING

<110> Bayer AG

<120> Single Nucleotide Polymorphisms predicting Cardiovascular Disease and Medication Efficacy

<130> empty

<160> 94

<170> PatentIn version 3.1

<210> 1

<211> 623

<212> DNA

<213> Homo Sapiens

<220>

<221> variation

<222> (140)..(140)

<223> BaySNP 10948, G140T

<400> 1
gagtccagat ccccagcccc ctctccagat tacattcatc caggcacagg aaaggacagg 60

gtcaggaaag gaggactctg ggcggcagcc tccacattcc ccttccacgc ttggccccca 120

gaatggagga gggtgtctgk attactgggc gaggtgtcct cccttcctgg ggactgtggg 180

gggtggtcaa aagacctcta tgccccacct ccttcctccc tctgccctgc tgtgcctggg 240

gcagggggag aacagcccac ctcgtgactg ggggctggcc cagcccgccc tatccctggg 300

ggagggggcg ggacaggggg agccctataa ttggacaagt ctgggatcct tgagtcctac 360

tcagccccag cggaggtgaa ggacgtcctt ccccaggagc cggtgagaag cgcagtcggg 420

ggcacgggga tgagctcagg ggcctctaga aagagctggg accctgggaa cccctggcct 480

ccaggtagtc tcaggagagc tactcggggt cgggcttggg gagaggagga gcgggggtga 540

ggcaagcagc aggggactgg acctgggaag ggctgggcag cagagacgac ccgacccgct 600

agaaggtggg gtggggagag cag 623

<210> 2

<211> 432

<212> DNA

<213> Homo Sapiens

<220>

<221> variation

<222> (94)..(94)

<223> BaySNP7363, A94G

<400> 2
gtgcttttag aaaaggcata aatggtgtta ggagaaaaag taggtggccc ggtttttttcc 60

cattctggca caggtcaggc tggtttccca cacrtgcccc attgactttg ttttctctcc 120

tcttccctta cacttcataa tgaagactct ttcactactg ggtttcacaa atgagagaaa 180

ttgattagaa caattacctt tcagctcatt ccaggcatcc cacttggcct tgcccgtgaa 240

gtccaacatc ccgggccgtt cttaaacaag ggaagaaaag gcattagatc tggttcaaat 300

tctaatggag aacttgatca accttcttgc cataaatgcc atcttctatt acacctgttc 360

cttctaggct ctgtcatggg gttaaatagg tctagtagag tgggttctgc cctaagaaaa 420

ccaatgtatg cc 432

<210> 3

<211> 1413

<212> DNA

<213> Homo Sapiens

<220>

<221> variation

<222> (146)..(146)

<223> BaySNP 11377, C146T

<400> 3

```
agaagcacca tttcctaggc gaggcagacc ttttgcagac cacagctgct tccacctgaa      60
ctttacagca gaccattgtg cctgccccgg gacacagtcc cacccatccc ccgctgcctg     120
gcaagggcca gcaacacaac gtttaygctc tctcactctc ctggccttgg ccagttcaaa     180
gcctttggtg ccatcacaga gttccgagca acagtccaat ccccaaggca ggaagaccca     240
ggaacggcca tgacaacgaa ttaacaacat gggaaagcca ggggcaggag ggtgaaccga     300
ccatgtcacc caaggccacg ctgggcacgt ctgggatgcc ctgtaactac ttactgactg     360
acacacacca ccgcctttct gcaagatccg acctcgccac acctgacaaa actcccagga     420
cacctgacat tggctcagtc gtgctggcgc aggtgtaggc accaaatgtc ttgttttgta     480
gctagggcca caaatctctg tctataaaca tgcctcgtct ctccacctgg accgccaggg     540
catccagtaa caggataatg tcaaaacctc ctggcattct ggcaccttct gtcacctacc     600
tccacggcct aatccaaggc actacatgct gtgatgcccc acccgtgccc acggcagaca     660
gcactaatcc atctcagaat tcctgcatcg agtctcaaat ccttgccagc acagggctcc     720
aggcagccac tactgatcga tcggagatga cactcaaagt aaaactaatc tgcaatgctt     780
agtccagtag ttcttaactg ggaataatct tgccccccac ggaaacttga tggcaatgtc     840
tgagaatatt tttggttgtc acaactggag agggaaggtg tagctggcac caagtgaata     900
gaggccacga tgctgctaaa aatcctacag tgctcaggaa gatcccccac ccccaacaga     960
gaatcatcca gcctcaaata tcactagtgc caagagtgag aatccctggc ttagtctgac    1020
aataggtgag tgacatataa tttatcctcc aaactgggat gcttctgaaa gtgaaagaga    1080
gtgctattaa taataactgt ggaacaacag gcataaatca ggactgtaca ggcaaaccag    1140
tacactgaac agataaaggc agatggaggt ccccaggaag ggtcaaagag ctcctctcct    1200
acaagtcccc agacagagcc accaactgcc ccgtttggca gaataaatgc tagtatgtca    1260
taaggcacag ccagctcagg aaggaaatct ttgtctagtt cttgtttaaa gaaaaagcac    1320
aaaaggaact ttccggctgt gccttttaag atgtaagtgg cagagaacaa atctgtttcc    1380
ctcaaagcca gggggcagc atgcactaag act                                   1413
```

<210> 4

<211> 527

<212> DNA

<213> Homo Sapiens

<220>

<221> variation

<222> (413)..(413)

<223> BaySNP 1837, C413T

<400> 4
ctcaccagtc cccttctga gagcccgtat gagcagggag ccggccccta ctccttctgg 60

cagacccagc taaggttcta ccttaggggc cacgccacct ccccagggag gggtccacag 120

gcatggggac ctggggtgcc cctcacagga cacttccttg caggaacaga ggtgccatgc 180

agccccgggt actccttgtt gttgccctcc tggcgctcct ggcctctgcc cgtaagcact 240

tggtgggact gggctggggg cagggtggag gcaacttggg gatcccagtc ccaatgggtg 300

gtcaagcagg agcccagggc tcgtccagag gccgatccac cccactcagc cctgctcttt 360

cctcaggagc ttcagaggcc gaggatgcct cccttctcag cttcatgcag ggytacatga 420

agcacgccac caagaccgcc aaggatgcac tgagcagcgt gcaggagtcc caggtggccc 480

agcaggccag gtacacccgc tggcctccct ccccatcccc cctgcca 527

<210> 5

<211> 607

<212> DNA

<213> Homo Sapiens

<220>

<221> variation

<222> (194)..(194)

<223> BaySNP 10830, A194G

<400> 5
aaatccaaag gcagtgaggg tagttttcag catgctttct ttaggaaggt agttatttgg 60

atcaaatata agattccctt ctattttggc tgaggctggg tcaagtgatg gaagagaaac 120

agatttgtag agttgatagt tccgagagaa ttttctgaag tccatgacag ttggaagttg 180

agattctttc agarcttctt tcactaactt tttcagacta gataagaaga agtatatttt 240

gagctgacac accatgttat tatcctttga ctcttgcacc ccaagtaaat atggatttcc 300

aatcactacc aaaatgtctg gatttcattg accctaagtc tttgggtcta gatctgctac 360

acatttgcac aagtgtttgt ttcagaagca agggcagaca gtggctatgc caaaacctag 420

ggttggaatt ccagctcagg gccctcagtg gtatatgggg tgaatagctc ttacttactc 480

ttggatatcc aattcttctg agttcaagat attggcaata tgggaagcca caaagttctt 540

cacttgctca ttctgttccc atggtagaat ttggacaatt ttgttaatat ctgcctgtga 600

aggactc 607

<210> 6

<211> 587

<212> DNA

<213> Homo Sapiens

<220>

<221> variation

<222> (202)..(202)

<223> BaySNP 555, A202G

<400> 6
tggagcctcc ccataggcac tcggggcctc ccctgggatg agactccagc tttgctccct 60

gccttcctcc cccaggcatg gctggggact ggaagaccac cttcaccgtg gcgcagaatg 120

agcgcttcga tgcggactat gcggagaaga tggcaggctg cagcctcagc ttccgctctg 180

agctgtgaga ggggttcctg grgtcactgc agagggagtg tgcgaatcaa gcctgaccaa 240

gaggctccag aataaagtat gatttgtgtt caatgcagag tctctattcc aagccaagag 300

aaaccctgag ctgaaagagt gatcgcccac tggggccaaa tacggccacc tccccgctcc 360

agctcctcaa cttgccctgt ttggagaggg gagagggtct ggagaagtaa aacccaggag 420

acgagtagag gggaatgtg tttaatccca gcacgtcctc tgctgtcctg ccctgtgtcg 480

ttgggggatg gcgagtctgc caggcggcat cactttttct tgggttcctt acaagccacc 540

acgtatctct gagccacatt gaggggaggg gaatagccat ctgcata 587

<210> 7

<211> 2090

<212> DNA

<213> Homo Sapiens

<220>

<221> variation

<222> (1429)..(1429)

<223> BaySNP 11652, C1429T

<400> 7
cgacacctgc agccagctct gcgtgaacct ggagggtggc tacaagtgcc agtgtgagga 60

aggcttccag ctggaccccc acacgaaggc ctgcaaggct gtgggtgagc acgggaaggc 120

ggcgggtggg ggcggcctca ccccttgcag gcagcagtgg tgggggagtt tcatcctctg 180

aactttgcac agactcatat cccctgaccg ggaggctgtt tgctcctgag ggctctggca 240

ggggagtctg ccgccctgtt aggacttggg cttgccaggg ggatgcctgc atatgtccta 300

gtttttggga atatccagtt aacggaaccc tcagccctac tggtggaaca ggaaccggct 360

ttcctttcag ggacaacctg gggagtgact tcaaggggtt aaagaaaaaa aattagctgg 420

gcatggtgcc acacacctgt ggtcccagct actcagaagg ctgaggcggg aggattgctt 480

gagggcagga ggattggttg atcctcccac ctcagcctcc ggagtagctg ggacctcagg 540

tgcatgccac tatgcctggc taattttctt tttccttttt tttttttttt cgagacggag 600

tctcgctctg ttgcccaggc tggagtgcag tggcaggatc tcggctcact gcaagctccg 660

cctcccgggt tcacgccatt ctcctgcctc agcctcccca gtagctggga ctacaggagc 720

ccgccactgc accaggccaa tttttttgta tttttagtag agacggggtt tcactgtgtt 780

agccaggatg gtctcgatct cctgacttcg tgatccgccc acctcggcct tccaaagtgc 840

tcggattaca ggcgtgagcc actgcgccca gccgctaatt ttcatatttt tagtaaaaac 900

agggtttcac catgttggcc aggctagtct tgaactcctg aacccaagtg atcctcctgc 960

cttggcctcc caaagtgctg ggattacaga caccacacct ggctattatt attttttaga 1020

gacagggtgc tgctctatct tccagcctgt agtgcagtgc agcctccatc atagctcgct 1080

gcagccttga cctcctgggt tcacgtgatc gtcccgccta agcctctgga ggagctggga 1140

gtactggcat gtgccaccat gcctggttaa tttttttttt tttttttttt gagacagagt 1200

ctcattctgt cacccaggct ggagtgcggt ggtgcgatct tggcttactg aaacctccac 1260

ctcccaggtt ccagcaattc tcctgcctca cccttctgag tagctgggat tacaggttcc 1320

ggctaccaaa cctggctagt tttgtatgt ttagtagaga cagggtttca ccatgttggt 1380

gaggctggtc tcgattctcc cgcctcagcc tcccaaagtg ctgggattac aggcttgagc 1440

caccgtgcct ggcttttttt ttttttttt ttttttgtgg caataaggtc tcattgtctt 1500

gcccaggcta gccttatgct cctagcctca agtgatcctc ctccctcagc ctcccaaagt 1560

gctgggatta caggtgggcg ccactgtgcc tgttcccgtt gggaggtctt ttccaccctc 1620

tttttctggg tgcctcctct ggctcagccg caccctgcag gatgacacaa ggggatgggg 1680

aggcactctt ggttccatcg acgggtcccc tctgaccccc tgacctcgct ccccggaccc 1740

ccaggctcca tcgcctacct cttcttcacc aaccggcacg aggtcaggaa gatgacgctg 1800

gaccggagcg agtacaccag cctcatcccc aacctgagga acgtggtcgc tctggacacg 1860

gaggtggcca gcaatagaat ctactggtct gacctgtccc agagaatgat ctgcaggtga 1920

gcgtcgcccc tgcctgcagc cttggcccgc aggtgagatg agggctcctg gygctgatgc 1980

ccttctctcc tcctgcctca gcacccagct tgacagagcc cacggcgtct cttcctatga 2040

caccgtcatc agcagggaca tccaggcccc cgacgggctg gctgtggact 2090

<210> 8

<211> 628

<212> DNA

<213> Homo Sapiens

<220>

<221> variation

<222> (487)..(487)

<223> BaySNP 8138, C487T

<400> 8

aatcataaaa acctacctgt gtctgaatct gtttcctcct ccttctttcc agttatgaca 60

gagttttagt cttaagacta atcattcata ctttggctcc tatctcctct gccttctcag 120

ggaccttaca tcattattac cactcattct cttctgtatc ttccatcttt ccctctcaac 180

tggatctgtc ccattggcat tgacacatca aggcactgat gcccttcatg gagccacccc 240

tgccagctcc cttcacacac aaaagtggcc tttgcgttct gtcttgattt cgccattgac 300

ttagttctca acctattgaa gtttggctcc tgttcctgtc tctctctaca ttcagtggac 360

attttaaaat tctatcttga tctctcagca gcacttagac tttctcctat attttttgta 420
tttgcttctg ttatgtcacg gtttgctgat tttcctccag ctctttggct ttttcttgtg 480
ctgcccycgc ctgtaaagtt aagttcctca acccottatc acaggagtca gagatgatgt 540
ggacattcct atagttccac aaagccattt tcaaattttt tttattctca tgcaaactcc 600
tttgtcattt gagtttcatt ctgcataa 628

<210> 9
<211> 1279
<212> DNA
<213> Homo Sapiens

<220>
<221> variation
<222> (240)..(240)
<223> BaySNP 1765, A240G

<400> 9
gtatctgtgt gtctgtgtct gtgtgtgtgt gagtgtgtgt ccttgaccac gtgttctcca 60
taggcgggga gtcctgcagt tactcatgga tgagtacctg tgtttgtgtc tcagcaggca 120
aatttgtaaa tgtacaagcc ttcaagcctg cacaagcata aatgtcactg tgtgggggtg 180
cttgagagtc tgtgagcctg cccttaggag ctgtgaggta tgggcttcac ggaggaagar 240
gtcatagctg caggaagata tactgtcttg ggaccttcag ctgcatcttg acatctctgg 300
ctccccatgg aaagggcatc cctagctgag atgcaggacc tctgagaaga ggcaggggtt 360
aaaggatcag ataagccctc catttccccc atccaagtga agagagaagg aggggaggta 420
gccccctacc ctgctccatc ttagagcaag gtagacccag ctcaggaggt cttgcttggg 480
aagtgatgag accttgactt ttccagtctg tctttttccc ctagcacccc caaactcccc 540
ttaatcacca tcctaagttg ctgtgggtga tgcaatagca agatgaggag cagatctggg 600
ctgtttaaac taagaggctg ggtgaggtgg gggtatttag ggcctggagc ttagagttca 660
acctaccaac gacccctgaa gagggaaggc atctgacacc cacaacctgt tctagggatg 720
atttttcctc caatcccttc tcccctgcat ctccactgca gaggcaggct tcactgtccc 780
cccattaccc agtggctgtg aagggcagcg tgggagttgg gggaagggac gacactggtg 840
ggagggagcc cagcctgctc cagctaccac ggagaggctg agatgggggg agcgttggcg 900

gattcccagc tgccccact ctgtcccagc ctctggcttt ctcaaaaagg actctctgtt 960
ctccttcagc attcaagacc cagagagggg gacttgtggt tgggggaggg aggagtggag 1020
ggaggttggg ggggtccttg cttcctctct ttctttcttg cctgggcagc cgctggcccc 1080
aaatctctgc aggctcctgg ctgcagagcc tgagatcttt gccaggacag gaggaggggg 1140
aaggggcagt gtgtctcaag ctctaagcct gctggagagc agggcgggag cttgggaaaa 1200
ggaggcactg cgtggagctg cttagctcag ccacaatcca gcatgccaaa gtgcatggac 1260
cagcaagttt ttaaaaagc 1279

<210> 10
<211> 1127
<212> DNA
<213> Homo Sapiens

<220>
<221> variation
<222> (210)..(210)
<223> BaySNP 5019, A210T

<400> 10
cggacatatg aatgaatcag gccactcctt ctcactcctc ggcttttcta taggtgattt 60
cctccaccca aacactgtcc ccagaccctc ttcacctggc tcttcccctt cctccttggg 120
tctcaccgtg tatctccaaa gagtctcccc ttggtctggg ctagggcatc tctgtgtgct 180
tttatatatg ctttctatgt ggcacagccw gttctcccca gcacaggact gctccccttct 240
ggaaactggt ctataccctt ctgcgctgtg gccttcatca ggggcttcct cacagggtct 300
gcacccagaa ggcacttaac acaaatatgc tgcataaatg agtaggcaga gcaggaatgt 360
gtcctttttcc catttaacag gtgatgaaac cgagaaatgg ctatgaaata gcagggattg 420
gatgctcata ttctggttcc tagtccacaa ccccagactg actttccaag ttcacaagag 480
cagacatctc catatacagg agaccacgct cacgttcaga cccccacact ctcagacaca 540
cagacccaca agcacagcag ctaccagcaa aagccgcctt tcacttgtgc gacgctcctg 600
gctcagggga atccccagct catccaatcc acccatcagg atgcgagcca ggagagtccc 660
cgcctggcac cccaaggcca tgccctccag ccaggggcca agaagccact tactcatcat 720
ctcccccaga cagcttcagc agagtcttct tgtactcgcc agaggtgtca ttctgaagag 780

aaagaaagaa aggttacctc tcacccagcc ctgggctcct ctcagacaag gaggtgaagg 840
ttctgcacag cctgaaagag gggaaggctg aatgaacact tattcatagc agccctggcc 900
ctcctgtgct ggttaatatt gaataactgg ttctcagcgg gcaggttgga ggaagcccta 960
atttatagca tttgcctatt tctgtggtgt aaatacttct agcaatggac aatttcaagt 1020
taccaatatg actttgctac caatatgcgg agttgagaag agatgctcgc tgccctttca 1080
agccagtgcc ccagcacagc actgccacca gacagtcccc agcccag 1127

<210> 11
<211> 814
<212> DNA
<213> Homo Sapiens

<220>
<221> variation
<222> (99)..(99)
<223> BaySNP 2187, C99T

<400> 11
ctgagcctcg gcaatgccct tttcaccgac ctggtggtag acctgcagga caccttcgta 60
agtgccatga agacgctgta cctggcagac actttcccya ccaactttag ggactctgca 120
ggggccatga agcagatcaa tgattatgtg gcaaagcaaa cgaagggcaa gattgtggac 180
ttgcttaaga acctcgatag caatgcggtc gtgatcatgg tgaattacat cttctttaaa 240
ggtaaggccc ttgggcccaa acctgcactt tctttggctt ttctgctgct tttatctaaa 300
gaataccaa ttccctcaca tacataaaag acggggagta cgttaagttc ttttgggtgc 360
ctgttgagaa aaattaagta aacaagcagc cagagaaggt aagatgaatg ccttcttgct 420
gtggatggga ttagtgaggc tgagatgctg tttcctccac ggaggaagag ctggttgctg 480
tcttcgggcc cctggggaca tctgaagccc cagctttcta caggctctga agtatgaacc 540
cattgtggcc accatggcaa agacaccaac accttagcca ctcagggcag gacacagacc 600
ccaaagggct taaagggcat ttcccagtcc cccgtatccc tcaaatcttg gcccctctgc 660
cctcatagag ccaaaattcc ctcagacaaa tgcttgttcc cctgaatgcc tccccctgac 720
tcctcaaaag aagctgacct ctgtttattc cccgacactc cttgtaaaaa ttcctgctcg 780
ctttgcaact cccgccaatt gctacccttg ggaa 814

<210> 12

<211> 555

<212> DNA

<213> Homo Sapiens

<220>

<221> variation

<222> (31)..(31)

<223> BaySNP 11000, C31T

<400> 12
gggccaagcc tgcaaacttc ttgatattcc ytcgatatca caaagaccct cagaaaacat 60

cctcggcgac cacacaggca cgaggatgac gctgcacaga gaaggagcca ctgggcaccg 120

ggcggccccc ttcccaaaga cccacagtgt gggggcacac accacgatca tcatgtagcg 180

ccggccgccc tggtgcagct cctgcaccat ggccgggaag tcccggaagc catccttgtt 240

gaacgtgaag tccctccggg agtccatgta gtccaggtcg ttccactgga cgtcctgcag 300

ccacaacacg ggaccgtctg ctggggcctg aggagacgcg tcccggccag ccccttgcct 360

cccctgccac caccccaact caccaggggg aagtgggccc tggtcatgtt ctccaccacc 420

tggcgggtga tagcggtgga ggagtagccc cagcggcaca ggtggaagcc caggccccag 480

tatggcggca tgaacgggta tcctgcaggc caacgccgac ttcatgaggg agggaggagg 540

gagccttggg gcggg 555

<210> 13

<211> 1240

<212> DNA

<213> Homo Sapiens

<220>

<221> variation

<222> (1050)..(1050)

<223> BaySNP 5564, G1050T

<400> 13

```
tcaatggcta ggattcctca aagccattcc agctaagatt catacctcag agccaccaaa     60
gtggcaaatc ataaataggt taaagcatct ccccactttc aatgcaaggt attttggtcc    120
tgtttggtag aaagaaaaga acacaggagg ggagattggg agcccacact cgaattctgg    180
ttctgccaaa ccagccttgt gatcttgggt aaattcccta ccacctctgg actccatcag    240
taaaattggg cgtggactag gtgatctcat agatccttcc tgctggaaca ttctatggct    300
tgaattatat tctcctaatt attgtcaaaa ttgctgttat taagtatcta ctgtgtgcca    360
ggcactttaa ataaatattg tgtctaatct tcaaaacaaa tttgcaagga aggtttttgg    420
agataaggaa actgagactc aggattaagt aacacaccta aagtcacagg tgagcttgga    480
actgaaccca agtgtgcccc cactccactg gaatttgctt gccaggatgc caatgagttg    540
tagcttcatt tttcttagag actttcctgg ctgtggttga acaatgaaaa ggccctctag    600
tggtgtttgt tttagggaca cttaggtgat aacaattctg gtattctttc ccagacatgt    660
aacaagagta acatgtgtga aagcagcaaa gaggcactgg cagaaaacaa cctgaacctt    720
ccaaagatgg ctgaaaaaga tggatgcttc caatctggat tcaatgaggt accaacttgt    780
cgcactcact tttcactatt ccttaggcaa aacttctccc tcttgcatgc agtgcctgta    840
tacatataga tccaggcagc aacaaaaagt gggtaaatgt aaagaatgtt atgtaaattt    900
catgaggagg ccaacttcaa gctttttttaa aggcagttta ttcttggaca ggtatggcca    960
gagatggtgc cactgtggtg agattttaac aactgtcaaa tgtttaaaac tcccacaggt   1020
ttaattagtt catcctggga aaggtactck cagggccttt tccctctctg gctgcccctg   1080
gcagggtcca ggtctgccct ccctccctgc ccagctcatt ctccacagtg agataacctg   1140
cactgtcttc tgattattta tcaaagggag tttccagctc agcatacaca aggcagagag   1200
tgcagacaga acatcaaggg gacaattcag agaaggatcc                         1240
```

<210> 14

<211> 1405

<212> DNA

<213> Homo Sapiens

<220>

<221> variation

<222> (1207)..(1207)

<223> BaySNP 10388, A1207G

<400> 14
agaaaacaaa ttggaagaaa gtctcatatg ctagtcagag aggatgacaa cctaaattag 60

gcaggtctca ctcaaaggga aaggagtttg ggggtagagg aaggaggcac aggtagaacc 120

aacaggacct gcctacaggc tggataggaa tggtgaaaga ggagtaaaag ctgattctcc 180

cttttcatta ttgttatttt tttgttagct tgggtgattg gaaagtttga ctctcataat 240

ggaaatataa gagtgaaaaa cagatttggc tgcagaggtt ggatcaggtc agttagagac 300

aatttaaatt aaatgattct acattctttt tctatagtca acaagaaagg aggagaaatt 360

ggattacaaa aataagggca tttagttgtc aaggagtgcc aaatttattt tatgttgtat 420

ttgtagtagt ttaaattttt cagtgtgctg aggcagtaga taagaaagct atcaagaatt 480

taatatcttc tcttataggt tgtggttgtg catataaaaa taatgagata attctggagg 540

acaatttggc aatatctatt aaagttaaca tttaaaagaa tcacactgtt tgagcaaata 600

gttctatttc taggggtctg ttctacagaa aagtagctca tagaaccatt tagccagtgc 660

cctgggtggg atttgaaccc aggcctgtat gagtccaaaa tctgcaagtt taaccattat 720

gttataatac tctcttagag ccaggtgtgg tggcatatgc ctgtaatccc agcacttcgg 780

gaggctgagg tgggcggatc acctgaggtt ggaggttcaa gactagcctg accaacatgg 840

agaaacctca tctctactaa aaatacaaaa ttagccaagc atggtggcgc atgcctataa 900

tcccagctac ttgggaggct gaggcaggag aatcacttga acccaggagg cggaggttga 960

ggtgagctga gatcatgcca ttgcactcca gcctgggcga cggagtgaga ctccatctcc 1020

aaaaaacaaa acaaaaaaaa aacactctcc tagggacttt ttctttaaaa aaaatacttg 1080

tttaaaattc catggtaaat aagaagtata aaatattttt tatgtagaac tcccccaccc 1140

tatacacata tccatcaata gagaattggt taaattagtt taagatcatc cttataatgg 1200

aataatrtat agccaataaa aataatacat ttatttaaaa ggaaatacct gatacttcaa 1260

tatgtatgca tcagtatgat cacactttaa aatttatatt tataaaatta aatttgacaa 1320

tgatgtattc caaactttat atattatata taaatacata tatattactg tattatatag 1380

tttacttaaa tatggtattg tattt 1405

<210> 15

<211> 555

<212> DNA

<213> Homo Sapiens

<220>

<221> variation

<222> (286)..(286)

<223> BaySNP 11001, C286T

<400> 15

```
gggccaagcc tgcaaacttc ttgatattcc ctcgatatca caaagaccct cagaaaacat      60
cctcggcgac cacacaggca cgaggatgac gctgcacaga gaaggagcca ctgggcaccg     120
ggcggccccc ttcccaaaga cccacagtgt gggggcacac accacgatca tcatgtagcg     180
ccggccgccc tggtgcagct cctgcaccat ggccgggaag tcccggaagc catccttgtt     240
gaacgtgaag tccctccggg agtccatgta gtccaggtcg ttccaytgga cgtcctgcag     300
ccacaacacg ggaccgtctg ctggggcctg aggagacgcg tcccggccag ccccttgcct     360
cccctgccac caccccaact caccaggggg aagtgggccc tggtcatgtt ctccaccacc     420
tggcgggtga tagcggtgga ggagtagccc cagcggcaca ggtggaagcc caggccccag     480
tatggcggca tgaacgggta tcctgcaggc caacgccgac ttcatgaggg agggaggagg     540
gagccttggg gcggg                                                      555
```

<210> 16

<211> 555

<212> DNA

<213> Homo Sapiens

<220>

<221> variation

<222> (321)..(321)

<223> BaySNP 4856, A321G

<400> 16
gggccaagcc tgcaaacttc ttgatattcc ctcgatatca caaagaccct cagaaaacat 60

cctcggcgac cacacaggca cgaggatgac gctgcacaga gaaggagcca ctgggcaccg 120

ggcggccccc ttcccaaaga cccacagtgt gggggcacac accacgatca tcatgtagcg 180

ccggccgccc tggtgcagct cctgcaccat ggccgggaag tcccggaagc catccttgtt 240

gaacgtgaag tccctccggg agtccatgta gtccaggtcg ttccaytgga cgtcctgcag 300

ccacaacacg ggaccgtctg ctggggcctg aggagacgcg tcccggccag ccccttgcct 360

cccctgccac caccccaact caccaggggg aagtgggccc tggtcatgtt ctccaccacc 420

tggcgggtga tagcggtgga ggagtagccc cagcggcaca ggtggaagcc caggccccag 480

tatggcggca tgaacgggta tcctgcaggc caacgccgac ttcatgaggg agggaggagg 540

gagccttggg gcggg 555

<210> 17

<211> 492

<212> DNA

<213> Homo Sapiens

<220>

<221> variation

<222> (412)..(412)

<223> BaySNP 1574, C412T

<400> 17
tgaattctcc ttataatttc ttaccaaata tgtgtaaata ttttaaatga tctgtaccag 60

gaaagagtcc aaagaactag tgggactcct gaaattatgc acaaaattgt gtgtgtgcag 120

tgctccaggg agcaggtttt tcagggtccg gggaccccat gcgggatgaa gtcccctgct 180

ctggacctcg gcacatgctg ggagtgactt tggggctatg agtgtgatgg aatttcctgg 240

gctgcaatgc aggccaggaa aacactgact taatctaagt taaggacaaa gtgtgcttag 300

agagatagtg acatcctgga ttggaaaaga aacaaattct aattcagagt ctgattctag 360

aaaacacctc agaaagctag agtccacact gccctgggca atgaaaaagt tyttatgctt 420

gtttgaatgg gatgggtgtg tttttggtgt cttttggggg tgaccctggc tacatatttg 480

ctttctggtg ag 492

<210> 18

<211> 927

<212> DNA

<213> Homo Sapiens

<220>

<221> variation

<222> (194)..(194)

<223> BaySNP 2353, A194G

<400> 18
tggtacccat aacggaaact gccagaagca ctaggaagac aattcatgta gcctcgctcg 60

gggttggaca aggctgtgca ctggaaagct gagacatcaa aatgatggtc agaaaatatt 120

gcagtggaac tagagagtac ttggygtttg ttgagtgaac ccagttcatt caagcaacac 180

ttggagaact gaagattctt tataattccc tggacaaatg ggaagatggc tgtgttttct 240

ttgaatttca gccccctcac tgatcatggc actaattaaa agactaatta aycagaacat 300

tagttcctga gcactgttct tctaacacac aaaataaatt atggtccaag gaaagatttc 360

acgcagtctg aggacaacat atgggtcatg gatgtttata gatggtgcca aaaagaaaga 420

aaagaaagca cccctataaa atttgtctgt tttgcagttt ggtttttgtg ttatgttttg 480

ctactggaaa tcattctgtg ctggctttgg ctaggacaag gccagtgcct gatagtaaaa 540

actgcttgtt ttcaatatcc ttgctctcac tttaaagtga attaaaattt actgcttata 600

tatgcatcaa tactatcttt gtagctgaca ccatgcttga aacagtctca tcactgctaa 660

ttatgatcct tttcagaaga caggtgtgat gagagtttac actcaatcat gttctcatat 720

tctgctttcg aatttctaat atgatcctta gataagaaat tgtctattca tgctaatgtc 780

tacaagttta tcagccatcc agtttaaaaa aacagcaaga ttcattctta cacatatgaa 840

cctttcctgg ccaaacatta atcctttaat gaatctcaag acgagggtgc tcatcacctg 900

attcttagta agggcgattg gtttttt 927

<210> 19

<211> 652

<212> DNA

<213> Homo Sapiens

<220>

<221> variation

<222> (182)..(182)

<223> BaySNP 608, A182G

<400> 19
ctgcccacac ctgagaccca ataacaatgg ccccatcagg gtcacctctg gttccaggga 60

cgtgggccag ccagtggccc ctaccacaaa gccccttca atgccctggg cgttcagtcc 120

ttccagcccg acgcaggctt atccagggtg atggtcccca ggatacacaa gaagagacca 180

grgttcacag aggccaaggg ctcttggcaa gcaggggctg aactgggatc tgacacccac 240

caggctccat ggcccatgcg cctgactgtt aacacttcac ggggaygcct gccagcccca 300

gcagcctgga ctcagaccca gggcacacca ctcaggctcc agggaggccc agtggggtga 360

caccagcaca tggaggttct atgactgcgg cagccgctct gagacggtgc ctggtgccac 420

catcatcccc acccaccccc gccaagtcct gtgcctccag tcccctgacc ccactcacac 480

cccatccgtg gccaccctcg ccctcctgcc ccaccaccta ccgtgaccat ctggccggcg 540

cgcaggatgt acttgggcgt gaacttggag gcgatctcct ccccctccag acctggctct 600

tgattttcca gttcccaaag actgatcctg gagaaacgga cacactgacc tt 652

<210> 20

<211> 641

<212> DNA

<213> Homo Sapiens

<220>

<221> variation

<222> (348)..(348)

<223> baySNP: 11456, A348G

<400> 20
tggccagagg cttgcaggaa cctaaccctg tatttcccct atgagtgagg attcagtgtt 60

cacagtgact ttacggaaca taattaccgc aaacaatgag gattgattgt cctatgtgtc 120

aggccattgt aggtgtgtgg tgggacacag aggctgacaa gacatcgtcc ttgcccttga 180

gcctaaatta tcagggggag ctggatgcac gagccatgga taaatgggct gggggaagag 240
tgggtttagg ggtggggtag actggctctg agcaaagaga gccggggaag gcttcggggt 300
tcctgtggct gcctcggagg agggaatctc agcacctttt tgtccccrta gtaacaaggg 360
catggaacat ctgctcaaca tgaagtgcaa aaatgtggtc ccagtgtatg acctgctgct 420
ggagatgctg aatgcccacg tgcttcgcgg gtgcaagtcc tccatcacgg ggtccgagtg 480
cagcccggca gaggacagta aaagcaaaga gggctcccag aacccacagt ctcagtgacg 540
cctggccctg aggtgaactg gcccacagag gtcacaggct gaagcgtgaa ctccagtgtg 600
tcaggagcct gggcttcatc tttctgctgt gtggtccctc a 641

<210> 21
<211> 3350
<212> DNA
<213> Homo Sapiens

<220>
<221> variation
<222> (274)..(274)
<223> BaySNP: 6734, A274C

<400> 21
ggctccttac atggtaagaa tcaggatgtt gtaacttggt gaaaaataac tttattttat 60
tatttttcat gttgtatctg aatcttattg tgcagtccct tatagattgg taaatatttg 120
agcagttata tattgatacc ttttaattat agattgcctt tgtttttatt cactagtgta 180
attaaggaat gttgtagagg gaacagataa tagtagtaca tgtatgtgat tcaggtgctt 240
tcaaatgttc agattacttc ccagtagtaa atamagcttg aagatagtaa aacaggattg 300
caaattcagt aaataaagct ttaagatagt aaaataagat tgcaaattct ttcagatcca 360
ctttataaga gaggctatgt atgagatctt tatttatttc tagttgacct ccttttcttt 420
tctttttctt ttttttaagc gatgaggtct cgctgtgttg cccaggctgg tctcaatttt 480
gctgggctca ggcaatcctc tgactttggc ctccttgaag tgctggcatt acaggcatga 540
gccactgtgc ttagccctgt agttgatctc tttttgaaag gaacagactc acttatgcta 600
cttctagata aataagattt agtgaaaacg tatacattgc tgaagactgc agataagaag 660
ccattaggca cagggacttt ggctctacga gctggcgtct tgctttactt ctcccagagg 720

tcatacagtc ttttctttac ttctaacttt ttcctatggc catgacaagc ataactcttt 780
atgtcaattt accccctgct attaagaggc tcagctgggc atggtggctc ttgcctgtaa 840
tcctagcatt tttggaggcc aaggcaggat tgcttgagcc caggagttca agaccagcct 900
ggacaagaga gtgagaccca gtctcaacaa aaaattaaaa atttagacag gtgtggtggt 960
gtgtgcctgt agtcctagct actcgagagg ctgatgtggg cagatcgttt gagcctggga 1020
ggttgaggct gcattgagcc atgattgcgc cactgcactt ccgttcgggt gatggagtga 1080
gaccctgttg caaaaaaaaa agtctcagtg cttggaatca gattggtgca gtagggaaat 1140
tggccccatg tgtctgtttt gttaactgtt tcataggttg ctgaacgact gtatgagttt 1200
cctgttgctg ttttgacata gtactgtaaa cttagtggct tttttttttt tgggacggag 1260
tctcactctg ttgcctaggc tggagtgcag tggcactatc ttggctcact gcaacctctg 1320
cctcctgggt tcaagtgatt ctcctggctc agcttcccga gtagctggga ttgcagtcgt 1380
acaccaccac acccagctaa tttttgtat ttttagtaga gatggggtcc caccagactg 1440
gccagcctgg tgtcaaactg ttcttttttt tttgttatgt agaggctgat tatctttttc 1500
attattgaag acatttttaa aaaatgagca actttattat ttttattaac ttttgttgtt 1560
gttgtttgtt tgtttgtttt tgagacagag tcttgctctg tcgcccaggc tggagtgcag 1620
tggcgcgatc tcggctcact gcaagctccg cctcccgggt tcacgccatt ctcctgcctc 1680
agccacccga gcagccagga ctacaggcaa ccgccaccac gcccggctaa tttttgtat 1740
ttttagtaga gatggggttt cactgtgtta gccaggatgg tcttgatctc ctgacctcgt 1800
gatccacccg cctcggcctc ccaaagtgct gggattacag gcgtgagcca ctgcgcctgg 1860
cctattttta tcaacattct aaatagttga aacagaagta tataaagtag gaagtgaaag 1920
agtgcccgta caaaggatcg cttattgatc tgctgtctag gtaacagcaa tgtaccaatg 1980
ttaatgtcct gcttttgata ttatattgca gctattaaag acgtcactac tgcggcaagc 2040
tgagtgaagg actgtggact tcattttatt aatttttaac tttttttgaaa tgagatctct 2100
attgctgagg ctgagtgcag tggtgtgaat acagctcact gcagcctcga cctcctgggc 2160
tcaagcagtc ctcccatctc agcttcccaa ctagcaggga ccacaggtat gcaccaccac 2220
acccggctaa ttttttttgtt ttttgtagag atggggtctt gccatgttgc ccaggttggt 2280
cttgaactcc tggactcaag caatactctt atcttggcct ccaaagtgct gagatttcag 2340
gtgtgagcca ctacatctgt ctccatatac ttaaaaaatt taacagtttt attatattca 2400
cacactatac aatttactca cttaaagagt acaactaaat tatttttagt atattcacgg 2460
aattctgcaa ctgtcattac aatcagtttt acaacatttt catccccaga agaaaccctg 2520
aacctatggt ataattagca atcattcccc atttcccctc ctcaccccac cctctgctgt 2580

atacaaccag taatctactt tttgttccta tagatttgcc tattctggcc atttcatata 2640
aataaaaaca tacaatatgt tattcttttt tgttgttttt aagagacaga gtcttgcact 2700
gttgcccagg ctggaatgca gtggggcaat catagctcac ggctgccttg accttctggg 2760
ctcaagcaat cctctcacct cagctgagag ctcttgagta gctgagacca taggtgtgtg 2820
ccaccatgcc cggctaatta aaattttta aaaaaacatt ttaaaattgt ttatttatga 2880
atcattgcaa agaataaact gttatttatt tatttattta gagatgagtt ctgttttatc 2940
ttttgtcatt tatactctca tactttcagg ctctcttttt tttttttgg cagacagttt 3000
cactctatcc cccaggctgg agtgcagggg ctcaatctca gctcattgca acctccacct 3060
cctggattca agagattctc atgcctcaac ctcccgagta gctgggatta acaggygtgc 3120
accaccacac ccagctaatt tttgtatttt tagtagagat ggggtttcac catgttggcc 3180
aggctggtct caagctcctg acctcaggtg atccacccgc atcagcctct caaagtgcta 3240
ggattacagg tgtgagccat cacacctggc cgactcatat ctaagaaggc ttttccccac 3300
ccaaggtcac ctcttgctat gagacagccc aagctggtct tgaattccag 3350

<210> 22

<211> 458

<212> DNA

<213> Homo Sapiens

<220>

<221> variation

<222> (101)..(101)

<223> BaySNP:5320, A101G

<400> 22
tgttgctagg tgctcgggat atatagtaga aaaacaagcc tgtcttttt aatgtatgca 60
ggaaatctct ggagaagtaa gagaggttaa ggaaagctgg rtatgtgcct gctttacatg 120
gagtagtagt gattaagttg gccagtgccc agaagtgctt actgggtgcc aggattgttg 180
ctgtggaata tcgagtacca ctaacttta aattcttcaa agagggctgt gctgaagttt 240

gctgctgcca ctggagccac tccaattgct ggccgcttca ctcctggaac cttcactaac 300

cagatccagg cagccttccg ggagccacgg cttcttgtgg ttactgaccc agggctgacc 360

accagcctct cacggaggca tcttatgtta acctacctac cattgcgctg tgtaacacag 420

attctcctct gcgctatgtg gacattgcca tcccatgc 458

<210> 23

<211> 968

<212> DNA

<213> Homo Sapiens

<220>

<221> variation

<222> (152)..(152)

<223> BaySNP:152, A587G

<400> 23

tttccaggac ccaagacaga tgctgaagaa gacagcaggg accggagccc agcaaactgg 60

atgagagcag caaggccttc atgcatcttc aggctccatc agcagaggga gagaatgcat 120

tagacgggag tttcctgact cctattttct aatgtggcag tcctaatgag aagagggttt 180

gaagcgctaa gatgtgctga cagtcctaat ggtttgttat gagtttcctt agaaaaatta 240

aacactgaaa aacaaaactg cctccctccc tccctttgtg gttgctgaca tcagaggtca 300

ggaatataac aggcctgctc gtcagccaag ctctccacga gagccctctg ttccagaaga 360

gcaccaaggc agtgacctca acatacttgg gcaacattcc ctttcttggt aacccaggct 420

gagaaggtag aaagaatgtc tccaggttat gcaggaagaa agctagaagg gggaggggtg 480

gatattaaag tttcagttcc cagggaaacc ggatgttaca caaaaagggc aaaaatagcc 540

aagacccagg ggaagggcct gagcacagag gtgggaggtt ccagccrgcg ccctgcgagc 600

tggctgaatt acctccctct cctgggaggg tgacacacgc tcttggtacc attctccatt 660

aatcccattt tggaaattgg gaaagtacaa catgaatagt tgtcattatc ctgcctattt 720

cacagggttg gtcaggaagt aaaagaagac tgtgaatgtg aaatgctttc aaaatataaa 780

aatctcaaca taagttaact atactgaaat ccattaggac agaggttccc aaccttttag 840

gcaccaggga ctggtttcag ggaagacaat ttttccatgg acagggaggt ggggatggtt 900

tcagaatgaa actgttccac cccagatcat caggcattag tttgatcctc ataaggagtg 960

aacaacct 968

<210> 24

<211> 574

<212> DNA

<213> Homo Sapiens

<220>

<221> variation

<222> (390)..(390)

<223> BaySNP:1755, A390G

<400> 24
cttgtcctcg agccactgcc accctgtgta aaccctcatg cccagtcttg cgggtgccat 60

cccttctctt tgaagctgaa tggaccaaac atacccattg agtgttgggt ggggacatct 120

ctggaaagtc agcacctgga ccagctccac ccctctctga ggacaccttc tttccctttc 180

agaacaaaga acagccacca tgcagctctt cctcctcttg tgcctggtgc ttctcagccc 240

tcaggggccc tcccttcacc gccaccaccc ccgggagatg aagaagagag tcgaggacct 300

ccatgtaggt gccacggtgg cccccagcag cagaagggac tttacctttg acctctacag 360

ggccttggct tccgctgccc ccagccagar catcttcttc tcccctgtga gcatctccat 420

gagcctggcc atgctctccc tggggggctgg gtccagcaca aagatgcaga tcctggaggg 480

cctgggcctc aacctccaga aaagctcaga gaaggagctg cacagaggct ttcagcagct 540

ccttcaggaa ctcaaccagc ccagagatgg cttc 574

<210> 25

<211> 801

<212> DNA

<213> Homo Sapiens

<220>

<221> variation

<222> (583)..(583)

<223> BaySNP:4838, A583G

<400> 25
tttaaccaga accaagtgtt caccccccac agaatgtaca tgaaacacta gaggactgca 60

tgtttttccc tgagagaagc gtaagacaaa cagaagtcaa aaagtagtca ctgggagcgc 120

catccttcta agcaaatcct ccctttccct tttggaggat ttgcccgaac tacgtagcca 180

gtcagcactt agaccacctg cctcctcctc cccctataaa cccaccactc ccctcctcct 240

ttcccaaacc acttggggtg tcctaagccc tcactgcccc aagcccaaaa tatcaactaa 300

gatccttgtc tgtatttcca cagtcatacc taatgaattg ggaagtgggg cccctaaaaa 360

ccaattcaca tctatgcact tgtttccact ggatttggca gacaggcttt tttagttacc 420

gtaaccagat cttaagatta attaaaaact acataaagtg cttttaggtc cttagagata 480

catgatataa ataagcggct tatgatcaag aacgataaat acataggtaa aaatagctct 540

agcaaagatg accttatggc tctgagatgg gcaggcaggg carttcttcc ccattacatt 600

cttagtcatc ttattcatac ctatttttaa atggagtcca cagactaaag gtcatgttcc 660

gagactgaag ctttcaaatg accctagttc caatatagac actttcttca gtttatcagt 720

agcttttaaa ctctgaggtt aacacagctc actctttctg taatttagtg agcatactcc 780

tatacaaggg aaacttagag a 801

<210> 26

<211> 1746

<212> DNA

<213> Homo Sapiens

<220>

<221> variation

<222> (319)..(319)

<223> BaySNP3843. A319T

<400> 26
cagtataatt ttaaaagtta tggttgtaaa atgacaaaag aaaaaaacta gacatgtact 60

aaagaacaaa acaaaaactg gctgggggtg gtagcccaca cttgtaatcc cagcacttcg 120

```
ggaggctaag gcaggtggat tgtttgaggc caggaggtag aagttgcagt gagctgagat     180
tgccccactg cactccaacc tgggggacag agtgagaccc tgtctcaaaa attaaaatac     240
tacttaggct acattgtggg gggggtggta agtctatgga ttttgatttc ctttttgctg     300
ttttctattt ttggtaacwt gtttatgtaa cttcattttt taaaaaattg ctacattcaa     360
aatttccccc aaaaaagaaa cttcatatac aagttagttc atatacaagt tagaaaatga     420
aattttaaaa taccccataa gacacatcaa agaataattc tttaaaaatg gtaccagaac     480
cctacagaga atgtaaggta ccaaaggatg ttaaagacct aaatagatca cattcatggg     540
tgcaaagaag tgatactgta aaaagaaaga aactcaaaag atacctaaag gcaggtgaaa     600
agacatttac aacagataaa gggcatataa agaacaacag gaagaaacga gggaaatctt     660
acggctcagc cactgtcagg gctggggggt tctcggtgca ccctggaagc agctcccacc     720
accggggcca cagagaaacc cacacaagcc ccagacacgt ggacaagaat actcagagca     780
gcctggctct ccacaacttc tgaccaagaa ccaccggagg cccagcagag cagaacggag     840
cagtctgctg tgccgaggtg ggtttctgca gcggacatta ttcggctatg aaaatgaact     900
tgtggcagcc cctctgcatg ggcaaccctc atgccaagtg agcaacgtga gacttcagca     960
aacaccaaga tccacgtaca aagctcaaaa caggcaaaac tgagcatgtc caggacacgc    1020
acaggtggca aagccataaa gcaaaacgag gacatggtca caggctggga tgtggctaac    1080
ctcccaggtg acacctgggg atggtaaggg gctggccagg ttccatttca catgggtgca    1140
gcctaaactg catacagctc cttatagtta ccccacatgtc cacaggcctc acgcactttt   1200
cttctgtgtc acattccaca acaaaagaac accacacaca ggattctggg gaatcccaca    1260
ggctggaaga gccaggagcg tggcctcacc tggggttgat ctccagcgtg ggctgcagga    1320
gctgtgcgcg ctcctcctgg gtcttggcca gctgctgcat gcgcaggaag tggcgggcag    1380
cccccatctc cagcacggtg accatggcag ggtgggtgtc cagtcggagg gtcacctgtg    1440
agcaaagccc ggggttgagg gtgatagagg ttcccaatgt gagagggctg gcaggatctt    1500
accaggggtg aaggtcaccc agaccacgag gtagcaggcg ggtctcaag gactcccctg     1560
gaccagcgct gctccctccc cattacccac taacttgtgg gccctgagct gatgccccac    1620
gcaaagatac tgtgcccgag ggctccacc accctggtgc cccgctgctg cctccaggag     1680
ctgcccgaat ccatcgtcct ctgctccatg ccagcccacc ctgcatgagg ccccttcctc    1740
caagtg                                                               1746
```

<210> 27

<211> 615

<212> DNA

<213> Homo Sapiens

<220>

<221> variation

<222> (288)..(288)

<223> BaySNP10749, C288G

<400> 27
tgagaattta tgcggttaga ggcctggcag acctaaggct gtcaggcata tgtggctgtt 60

atcatcccta gttggtcttc cggatcagaa tactctagaa tctctctaga atcattaggt 120

ccagcctaga gacaggagag tagagattaa gataactgaa gctctttccc atccaactgt 180

ctcctatttt gctacaaacg taagagggaa tcttctgtca ggaaatgtcc agaaatcact 240

tccctgcttc agggttcaat gcatatctct tgaatttaat ttgcaaasca gggccacccc 300

attcccactg cggcatggaa cagctctacc tgacacaaca ttatctgctt cggaaaaccc 360

cttctttagg tccccttctct cgatcttcag ctcaggtcca taaaaggagt tgttctttat 420

agcatcctga ggatcacaaa gaagtttcag aaatgtggct tttagcaggg agggaaagat 480

aggttctgat tagggaaaga gaagctctct gaattgttga ctctctcttt ccataaatga 540

atctctatat gcttcacata cagacttgaa gtaagctcta ctatagccca gagaatcata 600

ttcctccatt tcctc 615

<210> 28

<211> 1302

<212> DNA

<213> Homo Sapiens

<220>

<221> variation

<222> (745)..(745)

<223> BaySNP 2203, C745T

<400> 28

```
ccccctgtgca gccgctgagc gaggagatgc tccttgggcc ctttgagtgc agtcctgatc      60
agagccgtgg tcctttgggg tgaactacct tggttccccc actgatcaca aaaacatggt     120
gggtccatgg gcagagccca agggaattcg gtgtgcacca gggttgaccc cagaggattg     180
ctgccccatc agtgctccct cacatgtcag taccttcaaa ctagggccaa gcccagcact     240
gcttgaggaa aacaagcatt cacaacttgt ttttggtttt taaaacccag tccacaaaat     300
aaccaatcct ggacatgaag attctttccc aattcacatc taacctcatc ttcttcacca     360
tttggcaatg ccatcatctc ctgccttcct cctgggccct ctctgctctg cgtgtcacct     420
gtgcttcggg cccttcccac aggacatttc tctaagagaa caatgtgcta tgtgaagagt     480
aagtcaacct gcctgacatt tggagtgttc cccttccact gagggcagtc gatagagctg     540
tattaagcca cttaaaatgt tcacttttga caaaggcaag cacttgtggg tttttgtttt     600
gtttttcatt cagtcttacg aatacttttg ccctttgatt aaagactcca gttaaaaaaa     660
attttaatga agaaagtgga aaacaaggaa gtcaaagcaa ggaaactatg taacatgtag     720
gaagtaggaa gtaaattata gtgaygtaat cttgaattgt aactgttctt gaatttaata     780
atctgtaggg taattagtaa catgtgttaa gtattttcat aagtatttca aattggagct     840
tcatggcaga aggcaaaccc atcaacaaaa attgtccctt aaacaaaaat taaaatcctc     900
aatccagcta tgttatattg aaaaaataga gcctgaggga tctttactag ttataaagat     960
acagaactct ttcaaaacct tttgaaatta acctctcact ataccagtat aattgagttt    1020
tcagtggggc agtcattatc caggtaatcc aagatatttt aaaatctgtc acgtagaact    1080
tggatgtacc tgcccccaat ccatgaacca agaccattga attcttggtt gaggaaacaa    1140
acatgaccct aaatcttgac tacagtcagg aaaggaatca tttctatttc tcctccatgg    1200
gagaaaatag ataagagtag aaactgcagg gaaaattatt tgcataacaa ttcctctact    1260
aacaatcagc tccttcctgg agactgccca gctaaagcaa ta                       1302
```

<210> 29

<211> 813

<212> DNA

<213> Homo Sapiens

<220>

<221> variation

<222> (89)..(89)

<223> BaySNP 1722, C89T

<400> 29
tgtttatcct cctcatgtcc cctccccacc caagttccag cttgcctggg gcttgttctg 60

ggaccctaag aaccccagga tgcccacayc gccaggccag cagggccaat ggtcatgcag 120

ggactgcggg acaggttccc atcatctcct gtgttagccc attccacctg ggaaaccaag 180

cgactgccgt ggctggctta tttctccctc tgccaacccc catctacaat gttctgggga 240

aaacaatgag aggtggaaaa ggtaacatac aaccaccttc cacccacatc acttccagtg 300

aggcagctaa gtccaccaac agagcgtggg agagtctttc atccaaaaat cccaacacgt 360

cgcatgcagc gacaggaggc tgtgctgggg aaacccaagg cctggctcag cctccatact 420

ctaagggcct cgggccagaa cgaccacgag aggggagcac ccaaaggcac gcagaggggc 480

ccacctgggt gcactgcaga ggggacagag gggctgtgag tgggaggtgg gccagtgctg 540

ttcactgccg cctgctcagg accaggggct gctgcagacc cagcccggtc tatgcccacc 600

cccgcaggtg cagcccagct tctgcacatc agatggagtg acgctgacta aaggacctgg 660

acagcgccgg cagatactcc caacactgtc cttctctttg ggagccaaag agaagccacg 720

cagatactcc caacactgtc cttttttttg ggagccaaaa gaaaaccccg gccaggcagc 780

caggcgagcc atttctccta caccaggcag ggg 813

<210> 30

<211> 688

<212> DNA

<213> Homo Sapiens

<220>

<221> variation

<222> (639)..(639)

<223> BaySNP:576, C639T

<400> 30
atgggacagc atccctggga cgttcaagta ccatcctggt ctcccttctc cagccttaga 60
gagtggacca gccagagcac ctcgtctgga ctctcagacc tgctgctttg tctctaccaa 120
ccttggcagg gatctaggat ccatttagtg ggatcaggtc ccagtcaata ccattggggc 180
tcaaataagt tcttagaacc acagagtcta gggccagggt cccaactcat aggtgacgga 240
gttcccttca agccacagat tctgtttttt ttgtgtgtgt gtgtgttttt ttttttttt 300
atcagagtcc catacctcac gggtattttc tcaatcagtg aacacctcaa gtactagccc 360
atgtgttttg agtaaaaagg gctcctttca acgaggatcc ccttctagag gctttgacta 420
accagtctct tggcacccct agaatgaagt tgagagcgtc acagggatgc ttaacgaggc 480
cgaggggaag gccattaagc tggccaagga cgtggcgtcc ctcagttccc agctccagga 540
cacccaggtg agtgtcctgc cacatcatcc aggggacctg gggggtggcc ttcctcgggg 600
caggtccctg ggacctcttt gcatcccttt tgcaggagyt gcttcaagaa gaaacccggc 660
agaagctcaa cgtgtctacg aagctgcg 688

<210> 31

<211> 455

<212> DNA

<213> Homo Sapiens

<220>

<221> variation

<222> (337)..(337)

<223> baySNP: 8168, A337C

<400> 31
ccccccatac aatacaagat cttccttcct cagttccctt aaagcacagc ccagggaaac 60
ctcctcacag ttttcatcca gccacgggcc agcatgtctg ggggcaaata cgtagactcg 120
gaggtaggca tccgtggggg ggcgccggct cgggcgtgcg gggagtgtcc gcttctgcta 180
tctgcctctc caaatatccc gactgctgcc ctggccccag ccctctctcc acttcggagc 240
actcctctgg cgttggcacc gctgaggaat gggcctgggc ggggaggtga agagaagcca 300
ggaatgtttt atgttttcct aatggagagg gggcctmggg agcccctgag ctaggaggac 360
acggaaaagg ggattggggt cctgagattg ggtctgttgg gcccaggacg cgttttctgg 420
atgggtctag gatgctcccc tgtcgcggga ccccc 455

<210> 32

<211> 650

<212> DNA

<213> Homo Sapiens

<220>

<221> misc_feature

<222> (85)..(85)

<223> n=Unsure

<220>

<221> variation

<222> (543)..(543)

<223> BaySNP:2109, A543G

<220>

<221> misc_feature

<222> (22)..(22)

<223> n=Unsure

<220>

<221> misc_feature

<222> (97)..(97)

<223> n=Unsure

<400> 32
atgcatgttt ccatagattt tntggactgg tgatcgacaa ataccatgaa gatttggagg 60

cctcatatcc cagtggaaga agcanagtta caggganagt caggaggtat agccacctgt 120

ttaggggcct gtaaaaccta cataaagaaa atgggcttaa gcagtgaagt ccaagggaaa 180

atactgccag ggaactggga gatkgactgt tcctcattcc cagccttctg gttccactga 240

gcctagcctc tttctgacat gcagatgaca ctgaaacaca attaagcaga aatacaggct 300

ggagaggctg aatgcaccag ggtagcagtg gcaaccagct gtcaactctt attaacttct 360

gcataaaaca taccttgagt gcggtttgtt atcaattact tgccgaaaag aaacaactgg 420

ggcaaggcaa gaccgccgct catatgaatt agagtgattg gttgattgat cagggcacct 480

gttgtaaatc ataactgttc caaacaatca ctagccggtg ctttaatttg taaacaaaat 540

tcrtgtcact gcagaattgc tttcactgaa gatgagcatt tgggttcctt tctaaatgaa 600

tctgttgtta ttatgtaggg aagaatgggg cacactttaa agagatgagc 650

<210> 33

<211> 600

<212> DNA

<213> Homo Sapiens

<220>

<221> variation

<222> (162)..(162)

<223> baySNP: 11637, A162C

<400> 33
actcaaacga gccagaaaaa aagaggtcat attaatggga tgaaaaccca agtgagttat 60

tatatgaccg agaaagtctg cattaagata aagaccctga aaacacatgt tatgtatcag 120

ctgcctaagg aagcttcttg taaggtccaa aaactaaaaa gmctgttaat aaaagaaact 180

ttcagtcaga ataagtctgt aagttttttt ttttcttttt aattgtaaat ggttctttgt 240

cagtttagta aaccagtgaa atgttgaaat gttttgacat gtactggtca aacttcagac 300

cttaaaatat tgctgtatag ctatgctata ggtttttttcc tttgttttgg tatatgtaac 360

catacctata ttattaaaat agatggatat agaagccagc ataattgaaa acacatctgc 420

agatctcttt tgcaaactat taaatcaaaa cattaactac tttatgtgta atgtgtaaat 480

ttttaccata ttttttatat tctgtaataa tgtcaactat gatttagatt gacttaaatt 540

tgggctcttt ttaatgatca ctcacaaatg tatgtttctt ttagctggcc agtacttttg 600

<210> 34

<211> 518

<212> DNA

<213> Homo Sapiens

<220>

<221> variation

<222> (109)..(109)

<223> BaySNP:1862, C109T

<400> 34
ggggaccacc ctctagttga ggccttgggg gatgaagagg gagggctcag ggccccattg 60

cagggccagc ctgtaggggc cgtggggcca cctggatgtg acgtctaayg ctggcggtgc 120

tcacagctcg ccacaggacc ctaaaagccc ctgccagacg agagctcatc ttttccttct 180

ttctcttcca gttctgtctt ccattccacc attgctgaat cccgctcacg gcggccctgg 240

gctgcagagg gcagatgggt gaggctcggc ccctgtctcc agagagctga agagggccct 300

cggccccacc cacgaggagg acagtcatga ggccgactgt ggagggggctc tggacagagg 360

tgtccgggga tccctgagcc tggctgcact ctccctcctg gttctttcct tgcgtgcaca 420

cgatcagcta tcgtttacaa agagtccttg gtgttgcctg gttggctgcc caggatggga 480

gctcctgccc ccatcggaca tgtaaacaaa ccaaggtc 518

<210> 35

<211> 1051

<212> DNA

<213> Homo Sapiens

<220>

<221> variation

<222> (215)..(215)

<223> BaySNP:11073, C215G

<400> 35
gctgcgtccg ggccccggc tgtcggcgcc ctcgcggctc agatcatccg ggcgttggag 60
agggaggcag agagcgggtc ccggagaagg aggaggaaga gccgaggact catcgctttc 120
gcgcctcagc tgtctagagt ccccccaccc cccctcccc aaccacccgt tccggagcaa 180
cagccgcctg aggacccgaa gctgcgggcc ggacsagcgc cgagaccact gctctctagc 240
tcccgcgcgc gctcccgtcc ctagcgccgc caccgccgcc gccgccgcct ctagcgcggc 300
cccgcgcagg cgcgctcacg accgcgtcgc ccctccccca cccctccgct cgccccgccc 360
cgctggctcc gccgccgcta cagctgcggc gcggcgcgca gctacagaga cccggatgtc 420
gttgggcagg aactgcggtg agggctggcg ggcgggggag gggccgccac agatgacgtc 480
actgccttac ccagcttctt tctctgccgc tgctgctgcc gccgcctttg ccttacggcc 540
tggagcacca ggccgaagcc gaagggagcg tccggatccc ggatagcagc caggacccgg 600
gccttcacgt tccggcgcca gggactggac agaatgtgcg gctattaggt gcccagtaga 660
tcgacagccc ctccgtgccg gcccttggcc aagtagggac tcggaagcta cccctacggc 720
gtccgcgccc ggagccctgg ctcttctacc tgcctgcaga tacaaacccc actccctgcg 780
aagatggtgt acacgcagcc ccgagcacag acccgaagct gtccaccctt cttgggcgcc 840
gtcgcggtct ggccgctgcc cttcgaaccc atggtgggtc ccaggcagct gccagacccc 900
cctaccccag agtcccactc ctccagagcc ttcctctccc agtgacccca ggaagcccaa 960
gcaggtgcca cggccacaca cctcgcgacc caaagggcag agacacaagc acgctaccac 1020
cgcccaccaa tgtgcgagtg caactgattc a 1051

<210> 36

<211> 668

<212> DNA

<213> Homo Sapiens

<220>

<221> variation

<222> (354)..(354)

<223> BaySNP:1056, A354G

<400> 36
agcctgtctg tcttgtctga atgtgggaaa ggacaaggga ccacctaggg taggactcac 60
aggcttgttc aggccgagag ggggatccag ggtaacagca tcagtccctg ctgtgtccca 120

gattccaggt ctgaggcagc gagtaccact gggatccata gctccggctg gaagaggcag 180
gcccccatca caggacttct gcaccagcca tgcggggctc agtccaggca gtcagcacct 240
accctgggca ggcaggggtg ggtgccagcg tctccaccct gtgggctgac ctgcatgtgt 300
gtctatgtgt ctgtgtgtat gcacgtgcgt gatctgcccc ctgcagccaa acarcaggac 360
ggggcagccg ccatggagat gcagcccctc aagagtgccg agggcggcga cgctgacgac 420
aggaagaagg ccagcatgca caagaaggag aagtccgtgc tgcagggcaa gctcaccaag 480
ctggctgtgc agatcgggaa ggcgggtgag tgcagcatgt gggaggcagg ggacagggct 540
cacaggggga ccccaggcag agccctgccc catgctcggc ctcagttacc tcatctgcaa 600
agtgggggc ttgaatgagt gcctgaagcc caccttttt taccacagca cccagtctga 660
gtcctgtg 668

<210> 37
<211> 555
<212> DNA
<213> Homo Sapiens

<220>
<221> variation
<222> (173)..(173)
<223> BaySNP:5245, A173G

<400> 37
tgagaccgaa tatcggcagt gatagcctat cacttctgct atactctatg cactagaaga 60
agcaagacac tactaatgca gcccaaactc aaggggattg cttaaggcta taatacatga 120
ggagatggag atcactgggg gctgcttaga tgctgcctac cacaggctac atrgctattc 180
ttttacaatc ttgattttta acttacagca acttgtactg gacagactgg aatagagaag 240
ctcctaaaat tgaaacgtca tctttagatg gagaaaacag aagaattctg atcaatacag 300
acattggatt gcccaatggc ttaacctttg accctttctc taaactgctc tgctgggcag 360
atgcaggtaa tactactgat ggaatgcaaa tagataacct ctcatcacag tgaatgtgag 420
cactaacaga ttttactaca aaatgaaatc ccatcactta gatctccttt ctttggtctg 480
taaactttta agtactagta aatttaagta ctagtaaatg agtcttaaaa tgaactttct 540
ggggtttggg tggag 555

<210> 38

<211> 549

<212> DNA

<213> Homo Sapiens

<220>

<221> variation

<222> (464)..(464)

<223> baySNP: 8241, A464G

<400> 38

aacaatactg ggcagccttt tttttttttt ttttaattgc aacaatgcaa aagccaagaa 60

agtataaggg tcacaagtct aaacaatgaa ttcttcaaca gggaaaacag ctagcttgaa 120

aacttgctga aaaacacaac ttgtgtttat ggcatttagt accttcaaat aattggcttt 180

gcagatattg gataccccat taaatctgac agtctcaaat ttttcatctc ttcaatcact 240

agtcaagaaa aatataaaaa caacaaatac ttccatatgg agcatttttc agagttttct 300

aacccagtct tatttttcta gtcagtaaac atttgtaaaa atactgtttc actaatactt 360

actgttaact gtcttgagag aaaagaaaaa tatgagagaa ctattgtttg gggaagttca 420

agtgatcttt caatatcatt actaacttct tccacttttt ccaraatttg aatattaacg 480

ctaaaggtgt aagacttcag atttcaaatt aatctttcta tattttttaa atttacagaa 540

tattatata 549

<210> 39

<211> 757

<212> DNA

<213> Homo Sapiens

<220>

<221> variation

<222> (71)..(71)

<223> BaySNP:8480, C71G

<400> 39
gatcctaaaa gagggtgata ggctcagggg gcttaagttc attcattaaa gttaggatga 60
ccaatacatt sttacactat tctcaaaaac tttatggact tttctccctt ttcaaaataa 120
tgttgacttg tgctatctga aaacacttcc tgcgtttcag actcagcctg caatatgtta 180
acagccaaca gttgaacaat attctgtgtg tgcatttgta gcgcagtaaa ccatttactc 240
aaagaaacaa aaacccaaaa caacttcccg cctccccaa tgaagacagc agaagagaac 300
taactgattt gtctgttgtc tttcctgtca agatcgccct cgcctttgct ttggtcagcg 360
ggaaggactt tatgtatgag tcatacaaat gttttgccag ggcccggagg tcagcggact 420
ctggattcag ctggtcgata tcactggaga tctccgccaa cagcttctcc ttctcggcct 480
gtggcatccg cccaaacctg atggctatag aggaagagga atgacaggat gaatcattgg 540
attactactg ctctgaacac acactgtgct ctcccagccc tcgttcccac acagtagaag 600
ccctcctcct ccactgctct tgagtcctgg tttctgtcta ccactttgct catgttttta 660
tttcacaaaa gtagcacaga ccatcagtaa aaattaaacc gttaactttc atttttaaaa 720
gttaatgcat actcctgctg agaaagtgaa agaattc 757

<210> 40
<211> 1014
<212> DNA
<213> Homo Sapiens

<220>
<221> variation
<222> (675)..(675)
<223> BaySNP:118 , C675T

<400> 40
acaaatttta gtcaatttga aaaatcaaaa actataaaaa actggtatac aggttaggca 60
tccctaatcc aaaaatccaa aacctgaaat gttccaaaat ccagcttttc agagcaccaa 120
catgacacca ccaggaggta attccatacc cgacctcgtg tgacgggtcg cagtcaaaac 180
ttggtttcag gccaagggca gtggtccaag cctgtaaacc cagtgctctg gggggccaag 240
ataggagact cgcttgagtc cagcagttca aggctacagt gagctgtaac ggcaccactg 300
caataaataa tttatttagt taaaatatta aactaccttc aggctattta agatgaatat 360

gaaacatgaa tgaatttcat gtttagactg ggtcccatcc ccaagatatc tctctctctg 420
tctctctctc tatatatata tattgcaaaa tctgaatcac ttcctgtccc aagcatttca 480
gataagggat attcaacctg tattacttct ccccgcaatg ttctctgcag tttacctgtt 540
tctcccacac acttaaatac tatgtacaac atattaatat atgctatatc tttaaagttg 600
ctgattaatg ttatttctaa catacctttt ttggaactgt tttgattctt cttggtggtg 660
ctgctatctg ctttyttaac attggcactt ttcacagatt ttttactttt agaagtagct 720
ttctgtttag gtttttctct tttggctgtc ttttttggtg gctgttacaa aagaaagtaa 780
aagtacacat attgatgaga ttcaatgcta tcccatccca tccctctact tcccctatgc 840
cccttaataa tattagagaa aaccaatata tttggtatca gcaattaaaa attctttttg 900
agagatgact aatatacaac aattttatcc caacacaagt ctaagcatga caaatcacaa 960
tcacacacac acaagagttg cccatattta ttcaacacta tggataaact acat 1014

<210> 41

<211> 739

<212> DNA

<213> Homo Sapiens

<220>

<221> variation

<222> (120)..(120)

<223> BaySNP:738, A120C

<400> 41
tcgagctcgg tacctgccat cttgccgctg ctccttctca acgccacttc cactcactgc 60
caccaacacc aacatgaacg ggagctcaac agcttccact aggtgttcat tgagaagggm 120
acattcctct tcacctcaga gttggtgggg gaaggcccac ccagataaga tctgtgacca 180
gatcagtgag gctgtccttg atgcccacct tcaacaaaat cctgatgcca aagtagcttg 240
tgaaactgtt gctaaaactg gaatgatcct tcttgcacgt gaaattacat ccagagctgc 300
tgttgactaa cagaaaatgc ttcgtgaagc tattaaacac attggatatg atgattcttc 360
caaagggttt gactacaaga cttgtaatgt gctggtagcc ttggagcaac agtcaccaga 420
tatttctgaa ggtgttcatc ttgacagaaa tgaagaagac attggtgttg gagaccaggg 480
cttgatgttt ggctacgcca ctgatgaaac tgaggagtgt atgtaggcct ttaaccattg 540

tcttagcata caagcttaaa gccaaactgg cagaactacg ccataatggc actttgccct 600
ggttatgccc tgattctaca actcaagtta ctgtgcacta tatgcaggat tgaggtgcta 660
tgcttcccat cagagtccac acaattgtat atctgtttgg catgattaaa aggtcgtctt 720
ggtgagatga gggatgccc 739

<210> 42
<211> 3350
<212> DNA
<213> Homo Sapiens

<220>
<221> variation
<222> (3116)..(3116)
<223> BaySNP:8148, C3116T

<400> 42
ggctccttac atggtaagaa tcaggatgtt gtaacttggt gaaaaataac tttattttat 60
tatttttcat gttgtatctg aatcttattg tgcagtccct tatagattgg taaatatttg 120
agcagttata tattgatacc ttttaattat agattgcctt tgtttttatt cactagtgta 180
attaaggaat gttgtagagg gaacagataa tagtagtaca tgtatgtgat tcaggtgctt 240
tcaaatgttc agattacttc ccagtagtaa atamagcttg aagatagtaa aacaggattg 300
caaattcagt aaataaagct ttaagatagt aaaataagat tgcaaattct ttcagatcca 360
ctttataaga gaggctatgt atgagatctt tatttatttc tagttgacct ccttttcttt 420
tctttttctt ttttttaagc gatgaggtct cgctgtgttg cccaggctgg tctcaatttt 480
gctgggctca ggcaatcctc tgactttggc ctccttgaag tgctggcatt acaggcatga 540
gccactgtgc ttagccctgt agttgatctc tttttgaaag gaacagactc acttatgcta 600
cttctagata aataagattt agtgaaaacg tatacattgc tgaagactgc agataagaag 660
ccattaggca cagggacttt ggctctacga gctggcgtct tgctttactt ctcccagagg 720
tcatacagtc ttttctttac ttctaacttt ttcctatggc catgacaagc ataactcttt 780
atgtcaattt accccctgct attaagaggc tcagctgggc atggtggctc ttgcctgtaa 840
tcctagcatt tttggaggcc aaggcaggat tgcttgagcc caggagttca agaccagcct 900
ggacaagaga gtgagaccca gtctcaacaa aaaattaaaa atttagacag gtgtggtggt 960

gtgtgcctgt agtcctagct actcgagagg ctgatgtggg cagatcgttt gagcctggga 1020
ggttgaggct gcattgagcc atgattgcgc cactgcactt ccgttcgggt gatggagtga 1080
gaccctgttg caaaaaaaaa agtctcagtg cttggaatca gattggtgca gtagggaaat 1140
tggccccatg tgtctgtttt gttaactgtt tcataggttg ctgaacgact gtatgagttt 1200
cctgttgctg ttttgacata gtactgtaaa cttagtggct ttttttttt tgggacggag 1260
tctcactctg ttgcctaggc tggagtgcag tggcactatc ttggctcact gcaacctctg 1320
cctcctgggt tcaagtgatt ctcctggctc agcttcccga gtagctggga ttgcagtcgt 1380
acaccaccac acccagctaa tttttgtat ttttagtaga gatggggtcc caccagactg 1440
gccagcctgg tgtcaaactg ttcttttttt tttgttatgt agaggctgat tatcttttc 1500
attattgaag acatttttaa aaaatgagca actttattat ttttattaac ttttgttgtt 1560
gttgtttgtt tgtttgtttt tgagacagag tcttgctctg tcgcccaggc tggagtgcag 1620
tggcgcgatc tcggctcact gcaagctccg cctcccgggt tcacgccatt ctcctgcctc 1680
agccacccga gcagccagga ctacaggcaa ccgccaccac gcccggctaa tttttgtat 1740
ttttagtaga gatggggttt cactgtgtta gccaggatgg tcttgatctc ctgacctcgt 1800
gatccacccg cctcggcctc ccaaagtgct gggattacag gcgtgagcca ctgcgcctgg 1860
cctattttta tcaacattct aaatagttga aacagaagta tataaagtag gaagtgaaag 1920
agtgcccgta caaaggatcg cttattgatc tgctgtctag gtaacagcaa tgtaccaatg 1980
ttaatgtcct gcttttgata ttatattgca gctattaaag acgtcactac tgcggcaagc 2040
tgagtgaagg actgtggact tcattttatt aatttttaac ttttttgaaa tgagatctct 2100
attgctgagg ctgagtgcag tggtgtgaat acagctcact gcagcctcga cctcctgggc 2160
tcaagcagtc ctcccatctc agcttcccaa ctagcaggga ccacaggtat gcaccaccac 2220
acccggctaa tttttttgtt ttttgtagag atggggtctt gccatgttgc ccaggttggt 2280
cttgaactcc tggactcaag caatactctt atcttggcct ccaaagtgct gagatttcag 2340
gtgtgagcca ctacatctgt ctccatatac ttaaaaaatt taacagtttt attatattca 2400
cacactatac aatttactca cttaaagagt acaactaaat tatttttagt atattcacgg 2460
aattctgcaa ctgtcattac aatcagtttt acaacatttt catccccaga agaaaccctg 2520
aacctatggt ataattagca atcattcccc atttcccctc ctcaccccac cctctgctgt 2580
atacaaccag taatctactt tttgttccta tagatttgcc tattctggcc atttcatata 2640
aataaaaaca tacaatatgt tattcttttt tgttgttttt aagagacaga gtcttgcact 2700
gttgcccagg ctggaatgca gtggggcaat catagctcac ggctgccttg accttctggg 2760
ctcaagcaat cctctcacct cagctgagag ctcttgagta gctgagacca taggtgtgtg 2820

ccaccatgcc cggctaatta aaatttttta aaaaaacatt ttaaaattgt ttatttatga 2880

atcattgcaa agaataaact gttatttatt tatttattta gagatgagtt ctgttttatc 2940

ttttgtcatt tatactctca tactttcagg ctctcttttt ttttttttgg cagacagttt 3000

cactctatcc cccaggctgg agtgcagggg ctcaatctca gctcattgca acctccacct 3060

cctggattca agagattctc atgcctcaac ctcccgagta gctgggatta acaggygtgc 3120

accaccacac ccagctaatt tttgtatttt tagtagagat ggggtttcac catgttggcc 3180

aggctggtct caagctcctg acctcaggtg atccacccgc atcagcctct caaagtgcta 3240

ggattacagg tgtgagccat cacacctggc cgactcatat ctaagaaggc ttttccccac 3300

ccaaggtcac ctcttgctat gagacagccc aagctggtct tgaattccag 3350

<210> 43

<211> 479

<212> DNA

<213> Homo Sapiens

<220>

<221> variation

<222> (263)..(263)

<223> BaySNP:1657, A263G

<400> 43

ctggcacaag ctaaaacaaa acagaacagc aaaatacctt ttgttttagc tagtgctact 60

tttgtacttt tatgtaatta gcattaaaat aatttccaca gcagctattc tctatttcat 120

aagatctcta ccaagcctcc tagaacattt caaaaaccaa aaacttgcca agaatatggt 180

gaaaaccatc aaggaagtca taacctttag attgaacatg ctgaattatc gcctcctggg 240

gacagctgcc agtcagagtt gargcaccaa gaagcagagc cacaccagac ccagagggtg 300

cagacagcag agtatttctg ctctgccagg ctcagtgagt cacgcatttg gcctcctcca 360

ctaagatcat gtattctacc agatttcgat tcatggagtc caaagatgaa tgaatcaaac 420

actgcttaag ggcaatgtat acattgggat tcctccccac ctcttcccta aggtgttag 479

<210> 44

<211> 637

<212> DNA

<213> Homo Sapiens

<220>

<221> variation

<222> (289)..(289)

<223> BaySNP:533, A289G

<400> 44
ggctgagcag agcagcacag ccgccgagct ggcccacatc ctccaggagc cccacttcca 60

ggttcctggc tgctagggct ggggtgaggg agcaggaggt gggtggactg gggcatgctg 120

ctgttggatg ggccagcagg aagttggatc tgggatggga agagacccgg gacactgccc 180

cattgtccct tctcttccca ccacccccca gtccctcctg gagacgcacg actctgtggc 240

ctcaaagacc tatgagacac caccccccag ccctggcctg gaccctacrt tcagcaacca 300

gcctgtacct cccgatgctg tgcgcatggt gggcatccgc aagacagccg gagaacatct 360

ggtgaggact gggcagggcc agaggtggtg ctggtgaggg tgggggatt gagaataacc 420

aatgaacgga caaaaaaggc caagtgtggt ctgaagatga agatggggcc agctttgtgc 480

agggaaggat tgatgcagca aagggtcggg ggaagaccca ggagaccata gacactgcac 540

acacacctgt gtccgcacct ctccagtctg cccacctctc ccctcattag tacctgctgt 600

aagtgaagaa tttaggcaga aggatggagg aggactt 637

<210> 45

<211> 626

<212> DNA

<213> Homo Sapiens

<220>

<221> variation

<222> (452)..(452)

<223> baySNP: 11462, G452T

<400> 45
tgctggcttt ttggacaccc actcccccgc caggaggcag ttgcaagcgc ggaggctgcg 60

agaaataact gcctcttgaa acttgcaggg cgaagagcag gcggcgagcg ctgggccggg 120

gagggaccac ccgagctgcg acgggctctg ggctgcgggg gcagggctgg cgccccggagc 180

ctgagctgca ggaggtgcgc tcgctttcct caacaggtgg cggcggggcg cgcgccggga 240

gaccccccct aatgcgggaa aagcacgtgt ccgcatttta gagaaggcaa ggccggtgtg 300

tttatctgca aggtaagcgc cccttcgctc gaggtgtggt ttaattgtct cattttgttt 360

gaaatcctgc ggtgagaaac cagtcgtgtt gagaacaata aaagaccaaa aaacgatcac 420

caaaaccaac tgtcctgaaa gctactggaa akttggaaaa tgcatgcttt gattaaatgt 480

cttcattcaa gacactggca agttaactta tttagtttgt gccgtgagct ctgggttgat 540

tgtgctaata tgaataactg aaaaacattt tatttcccta tggttttcct cgatggactt 600

ccccactatg ggtgaaatga caatgg 626

<210> 46

<211> 2246

<212> DNA

<213> Homo Sapiens

<220>

<221> variation

<222> (1960)..(1960)

<223> BaySNP:5717, A1960G

<400> 46
tcactggaaa aaggaattct gattgttcaa aagcacagga tatattaagg gcctcatata 60

atgcctggca cataagagac ctcagcaaat tacgggcatt catattatgt ttatacagtg 120

aaggcatcaa ggttataagc attctctttt ttcttttggg tagactgaag ctcagagagg 180

ttgagtggct tacttaaagc tgcacagcta ttagtaggca gatcaagatt agagttcaga 240

acttctcact ccctgcccag tttctgcttt ctttaccctt tgcctctttc aagttgtggg 300

tctgccggcc aggtgggagg tgctgtctgc aaagggcttc cctttctctt tggccactat 360

ctggctgggg agaggcctca cctagatgtt gttgaaggcc tgttacagcc gctttattgg 420

ggattttctg gcgatgagaa cgtgtgaatg tcctggcctt tagtcaactc cctacacctc 480

tgagagtgtc agacaagaga gcccatcaca ctggtgggat tgcaatcttt gcctctacca 540

ctgcttagct gcctttcctt agggcaagtt acttaatggt tctgtgactc agtttccctg 600
tttgtgaaaa gtgaaggtta atagtaccca ccatataggg ctgttagaat ggagtggaat 660
aattcatgta gaataagtat gtataacagt ggctaaaaca tagtcaggct gggcgcggtg 720
gctcacgcct gtaatcccag cactttggga ggccaaggca tgtggatcac gtgaggtcag 780
gagctcaaga ccagcctggc caacatggtg aaaccccgtc tccactgaaa atacaaaaat 840
tagctgggct tggtggcggg tgcctgtaat cccagctact cgggaggctg aggcaggaga 900
atcacttgaa cccaggaggc agaggttgca gttagccaag atcacaccac tgcactccag 960
cctgggcaac agagtgagac tccgtctcaa aaaaaaaaaa aaaaaaaata gccagttgcc 1020
tagaatagaa ccaactaaca gtggttttat ttttactgca aaaaataaaa ataaaaatag 1080
gagtagtgca agcactgggc cacatcacta caaaacaagt gtatctcagc atctcccacg 1140
agaataccac tcaggtcaaa acatgatata gtgaagtggg gatgaaaagg atccaaccat 1200
gggcagaacc tggggtctgg tgccagtgga gacagcccca gtgtctagca tgagacacgg 1260
ggaatgttcc gttggagggt gggtatgatg actctcctga aagcttccct ccctccagtc 1320
cagatggccc ctccatccct caacgtgacc aaggatggag acagctacag cctgcgctgg 1380
gaaacaatga aaatgcgata cgaacacata gaccacacat ttgagatcca gtacaggaaa 1440
gacacggcca cgtggaaggt gagggccttt gcccagggag gggagaaaca ctggggaggg 1500
cgggagaagg gaaagcaacc agaggcattc cacctgcaag gcgtcgggcc cttggcaggt 1560
gaccagtgag aggtagccac tgggacgtgg tgatcactag gctgtgtggt cagcaggtca 1620
ctgtcctgtc tcttggtgaa gtaactgagg tttggaaaag tggcgtggct tggccaacgt 1680
gaacagctga ccctgagtcc ccaggcaaca gaagaccctc tgggcaggga ggggttgaaa 1740
ggccactggg aagaaggttt tcaaaagtca tgaaagtttg gggttatttc ctcagaggaa 1800
tctcatctgg acacacatgg aggctcagac agagctgctt ctaatgagtc gggggtgcgc 1860
ccaggccagg gctcggtccc ctgcctccac agagcccaga acagaaacca cagaaccaac 1920
cccacacctt cagtctagaa atggggcaac tgaggctagr agggaggtgg gccagtggtg 1980
gagccaggag cgggccctgg ggtcctgaac ccccattctc agggtccaga gtccagtcgg 2040
cctgcactgc gttcctaaaa aggccacaat atgggtgcaa gctgccccag aagggctggg 2100
agctgagaag gctcaaaata gggtgggaca ggtggcttca gggttctggg cctcagtgtt 2160
gtcaatgtca ggggctgcac tgacaggtgg agtccccggt gccatccgaa gtgctgtccg 2220
tgggtgggcc ctcagggagg atccac 2246

<210> 47

<211> 472

<212> DNA

<213> Homo Sapiens

<220>

<221> variation

<222> (302)..(302)

<223> BaySNP:2376, C302T

<400> 47
ttttctgttg ggcccactta gttattggtc tcagcagatc tattcactgt gaaaatatca 60

aacgatagag cagggcagga ctatttattg gccttatagt ctattcttat caaaatgttg 120

cttttggata taaaataata tccacgctga ttctgaagat ttgaatgccc ccagaaaata 180

tttaaatcta agagaaaagg aagaaagaac agccggtttt ctggctccag tcaaaaacac 240

tgttttggac acataagtct ccttctccca gtcttacctc catccagttg ataaaccggg 300

cyacatcctg tcccaccagt ttggtgtagc ccgcggacac tgggtaatgc tcctgagccc 360

gtgacagcca gtccaccaca atgacattgg agtctggttc tctcttgtac agggcggcca 420

caagttttgg cacccaactc tcatacattc ctgttacctg aagatgatga ca 472

<210> 48

<211> 590

<212> DNA

<213> Homo Sapiens

<220>

<221> variation

<222> (377)..(377)

<223> baySNP: 11558, A377C

<400> 48
gagttcaaga ccagcctggt caacatggtg aaactgtgtc tctactaaaa atacaaaaat 60

tagccaggca tggtggcacg tgcctgtaat cccagctact cgggaggctg aggcaggaga 120

atcgcttgaa cctgggaggt ggaggttgca gtgggctgag atcacgccat tgcactccag 180

cctgggcgac agagtgagac tctgtctcaa aaaaaaaaaa aattatgaaa aaagttatgg 240

gattaaagaa agtcaggata aaaattttaa aaagcaggcc actgtcagca aagcctggag 300

aagtggggcc ggaggctccg cccccatcat gtgcctgcca ccccttccca gtcatccctt 360

tactcttaca gtagcamata agacccctgt ctaatggggg gagacaaatg tgtagaccct 420

tagccacctt ggccagggct gactccttaa atttctggat gatgatgatt gttatttaat 480

agccagaggc tcatataatt ggcctctttg gaagaggcct catggcctcc ttactctcac 540

caaagcaatt tttccctcag gggggctccc atcttcttac acagagaggc 590

<210> 49

<211> 603

<212> DNA

<213> Homo Sapiens

<220>

<221> variation

<222> (113)..(113)

<223> baySNP: 10785, C113T

<400> 49
caatataatt gggactaata gttaatactt caaccgcata cacagattca tgtcctaatc 60

ttgatgtata gccaaggcct ttcatttgag cctaagtcta ctgcattatt aaygatttac 120

aggttcaatt gtttaaagtg gaataagaat ttcaatctat tggtataaca atatatgagt 180

gtaaaatcct tctagattta tctgatttcc cctccctaca gtctttttat tgctgatctt 240

acaactaatt ccacagcaat gttttaatga ctcatcttta ttgaatcaaa gtgaagtgct 300

tagcattcag ctaaagaaaa gatcatagcc cactggggag attaggaaag gcttcacaga 360

agtggcattt gagctacgcc atgaagataa gttcctaagt atatctcaga ggcaatacct 420

taggcaacaa aattatctgt ccaagtgacc tccatggtat ttcccctcca gcacctcaat 480

ctacctcaac tacgacgtta gcttctacca tgacgaggac agtacctgcc accacaagag 540

cccccgggac caccgtccac agatccacct accagaacca cagcacagag acaccaagcc 600

tga 603

<210> 50

<211> 503

<212> DNA

<213> Homo Sapiens

<220>

<221> variation

<222> (415)..(415)

<223> BaySNP:2085, G415T

<400> 50
gggcagtccg tatggtggtg gttatggatc tggtggtgga agtggtggat atggtagcag 60

aaggttctaa aaacagcaga agagggctac agttcttaag aggggagaga gcaagaagtt 120

gttaggaaag ctgcaggtta ctttgagaca gtcgtcccaa atgcattaga ggaattgtaa 180

aaatctgcca cagaaggaac gatgatcgct agtcataaaa gttactgcag cttaaacggg 240

aaacccttct tgttcaggat tgccatagcc acagtttgca aaaagtgcag ctattgatta 300

atgcaatgta atatcaatta gatgtacatt tctgaggtct tttatctgtt gtagcttttt 360

cttttcatta catcaggtat attgcactgt aaattgtggt agtgttacca gaaakaaaaa 420

attaargaat ttttaacttt tcaaaaaaaa gaaaagaaat aagatttctt taagttcttc 480

agtgtctctg attctaaaag agg 503

<210> 51

<211> 385

<212> DNA

<213> Homo Sapiens

<220>

<221> variation

<222> (211)..(211)

<223> BaySNP:614, C211T

<400> 51
tgccttatac ttcctaactc ccagtgggcc tgaatagaca ctggccagat tttcaaagta 60
gcttcttgaa gatggatgat gatctgttta gaatgatcca ttcacctgtt ctgtttcagg 120
gtccaaatca gtatcataaa aacagggctg ggtggggagt ccattgtgct catcgttcct 180
actaaagggt acagaaaacc agccccgatg ytggcatgga tgttgcgaat gggcagaatg 240
aatcctgtag ggacaccttt tcgctctggg ttgtgagaca aggacaagtg tgttttggcc 300
atgcagatgg ggagattccc aaagccctgc tttgtgtaga cttcagcttt gtgttgagct 360
ttgaggagta attcaatgtt gtctg 385

<210> 52
<211> 455
<212> DNA
<213> Homo Sapiens

<220>
<221> variation
<222> (199)..(199)
<223> baySNP: 6396, C199T

<400> 52
taaaatgtgg gtcagacgtg tttggtttta taacctttaa aaggtgagtg agtccaggtc 60
ctggtgaacc ctttatccag ggcaaaggga agtagtaatt tgctgcctca ctcacgtagg 120
tataagctag tttatttgga taaaataagt tcactttctc tctttagagc aataatttta 180
ttccttgaga aacagttgyc cttgctctgc tccttgtttc ttctcctgag ctgaggctcc 240
tgccttgacc tttcttttta tagaataaaa ggaacataga taatttaaag acagacggga 300
tctctgagat ctactaattt aattttgctt tgtttttcag gaatgagcaa ttggtgactc 360
agtgagttag agcattatca atgcaccagt aaaagatttt gtattagaat cccagttttc 420
ctgcttggtg gttcagtgaa ataccctatc ctata 455

<210> 53
<211> 618
<212> DNA
<213> Homo Sapiens

<220>

<221> variation

<222> (93)..(93)

<223> baySNP: 9940, C93T

<400> 53
cagcatctcc tggaagaaca atttcttcca ttctccagca accttggcca cagacagtgg 60
ctttcttcag aatccaaaag aattctgtta acyacctatt gggccctgac tgattacttc 120
tttctttcat gcacttatgt ttgccctgaa cccacagata tttatttcac atcagccatc 180
aaatgaatct tcagagaagt tcaattttca tgaccattct gaataaaaca catgactact 240
atagtagaga acttattgga tgaaatttct aatcacacac acttcccttc accttcagtc 300
aaaaatcata gcaaaacact gtgaaacaaa gaggagaaac agcactttaa acttttgcca 360
ctgtattaaa tgattatata atacaatgac cccaatgttt ctgtattcac aaaatgcatc 420
aactcactcc gctatttctg acaatgacag acttagaaag acaaaagagg ccaggtacgg 480
tggctcacac atgtaatccc agcactttgg gaggccaagg caggcagatc atgaggtcag 540
gagactgaga ccattctggc taacacggtg aaaccctgtc tctactaaaa catacaaaaa 600
attagccggg catggtgg 618

<210> 54

<211> 582

<212> DNA

<213> Homo Sapiens

<220>

<221> variation

<222> (194)..(194)

<223> BaySNP 2353, A194G

<400> 54
cctccacagt atacagaaga cgacgtgaaa tttgatctgc aagaaaactg agtccatatt 60
cacatatgta tcaaatttgc acttcattta gaagtgtctg tcatcaagta cagcactgaa 120
ttgaaactga aaacaagagt caagaaagag caaagtcagc catctttata ttccacatga 180

atcctttccc tttrtggtct tatttgtttc tcctcagaaa agacaaaaag ctgagctgta 240

taaacacctg tgggctgggg gttgagggat aaatgagggg cgaaatggaa gctgaaggaa 300

ctgttggtca ggtagaaatc ttcccagatg cactgaagga aacacacttc atgtttgacg 360

taggaggtgc caccacacaa aacgtttcat ggaaggattt aaaggatctc atgattttta 420

gtattccaag aattttcttt caccaagggc gatttaatat gggtcattca tactgaaaga 480

aaaacaaaag ataataagag tttaaaaatt gcaaaacttg gagtgttagt agtaaaggta 540

aatattcatt agagatgaga agaggagcaa ggaaatgctt tc 582

<210> 55

<211> 606

<212> DNA

<213> Homo Sapiens

<220>

<221> misc_feature

<222> (513)..(513)

<223> n=Unsure

<220>

<221> variation

<222> (175)..(175)

<223> BaySNP52, C175T

<400> 55
ttgaggccag gagtttgaga ccagcctgga caacatagca agatcccacc tgtagtccta 60

gatacttggg agagtgagga gggagggtta cttgagccca ggagattaag gctataatag 120

tgagggatga ttgcaccact gcactccagc ctgggcaaca gagtgagacc ctgtytctaa 180

aaamaaaaaa aaattattaa aaaaaaaaaa gttttcttaa gagtccagac ttgtgaattg 240

ccagattagt gtaattttta aaatatgttt ctattataaa ttacccatac tcataaaaat 300

ataaatcaat ttattacacc ctctagaatt cactattaat tttcaacatt tttttcattc 360

tttttccatg catatttttt cacaattcta tgcatasttt tgcattataa aatatttctc 420

aatataaaat cttcttcaag gccaggcgca gtggctcatg cctgcaatcc cagcacttta 480

gaaggccaag gcgagcagat cacttgaggt cangaattca agaccagcct gaccaacatg 540
gtgaaacccc ttctctacta aaaatacaaa aattagccgg gcatggtggt gcgcgcctgt 600
aatccc 606

<210> 56
<211> 525
<212> DNA
<213> Homo Sapiens

<220>
<221> variation
<222> (210)..(210)
<223> BaySNP:1757, A210G

<400> 56
vttctggaat gcctctcttg gcacccctgc cccaacaggc tgctgggatc tgcacgtgga 60
atcacagggc tggttgcatg caacggcaaa gggcatgctc ttctgcctgg gggagggcct 120
cttgtccctc ccagcaggtc ccgggacagg gctggcagcc accccagcac ccaaaataag 180
atgacaaaca agaggaaaca aaccaaaatg raggagagcc atccgggctg gaggagggca 240
gaggctcccc aaggggatct ggggagggct tcaacttatg aatgcatcag gccttggaag 300
acgtggatgg aagaggcgag ggcaaaagga agagatgagg ggcatggaga gagactcagg 360
gaagaaggag aaagagcaat catgcagctt gggacaaatc ttttgttgct ttatcagatt 420
ctcagtcaat caattggtgt ttgctagaac cggggtacgc aggggagtgt tgaagagaga 480
gtgcgcgcgc gagaagagag agatcaagag aacgggcatc gccag 525

<210> 57
<211> 663
<212> DNA
<213> Homo Sapiens

<220>
<221> variation

<222> (284)..(284)

<223> BaySNP1524, A284C

<400> 57
ggtgtggtgg cacatgcctg taatctcagc tattcaggag gctgaggcag gagaattgct 60

tgagcctggg agacagaggt tgcagtgagc agagatcgcg ccactgcact ccagcctggc 120

cgacagagcg agactctgtc tcaaaaaaaa aaaaaaaaaa aaaaaaaaaa agaaaagaaa 180

agaaaaagaa aaagaaaagg aaaaagaaaa ccagaagtag gagcagcctg aagaaatgac 240

agggagttga tttcccactg ggagccctct caaagcccac acamccgcct gcctggggta 300

acagtatctc ctcggacatc ctgcaccctc ccaygctccc ccctccctac agtagtgaaa 360

gacctaggca ggatgacccc agctcctctg tgagaatttc acaccctagt gtgaagtcat 420

agccttgtaa ctttcccttt aagaactgtc agagctgggg aggctggcca agctcaggct 480

ggaggtgggg acagagggaa gaaagaaaaa aaaaaaagag agagaggcag gaaaagtttt 540

ttcagaggaa aatgcagggt ttgtccttca ccctgacgtc agatcttgct ttataaaaac 600

ccccaaggct gcggagaagc atatctggtg ctcctgatgg gcggccagtc tgggcccagc 660

tcc 663

<210> 58

<211> 921

<212> DNA

<213> Homo Sapiens

<220>

<221> variation

<222> (133)..(133)

<223> baySNP: 4912, C133G

<400> 58
cccttcattg cggcgggctg cgggccaggc ttcactgagc gtccgcagag cccgggcccg 60

agccgcgtgt ggargggctg aggctcgcct gtccccgccc cccggggcgg gccgggggcg 120

gggtcccggc ggggcggagc catgcgcccc cccctttttt ttttaaaagt cggctggtag 180

cggggaggat cgcggaggct tggggcagcc gggtagctcg gaggtcgtgg cgctgggggc 240

tagcaccagc gctctgtcgg gaggcgcagc ggttaggtgg accggtcagc ggactcaccg 300

gccagggcgc tcggtgctgg aatttgatat tcattgatcc gggttttatc cctcttcttt 360

tttcttaaac attttttttt aaaactgtat tgtttctcgt tttaatttat ttttgcttgc 420

cattccccac ttgaatcggg ccgacggctt ggggagattg ctctacttcc ccaaatcact 480

gtggattttg gaaaccagca gaaagaggaa agaggtagca agagctccag agagaagtcg 540

aggaagagag agacggggtc agagagagcg cgcgggcgtg cgagcagcga aagcgacagg 600

ggcaaagtga gtgacctgct tttgggggtg accgccggag cgcggcgtga gccctccccc 660

ttgggatccc gcagctgacc agtcgcgctg acggacagac agacagacac cgcccccagc 720

cccagctacc acctcctccc cggccggcgg cggacagtgg acgcggcggc gagccgcggg 780

caggggccgg agcccgcgcc cggaggcggg gtggaggggg tcggggctcg cggcgtcgca 840

ctgaaacttt tcgtccaact tctgggctgt tctcgcttcg gaggagccgt ggtccgcgcg 900

ggggaagccg agccgagcgg a 921

<210> 59

<211> 3051

<212> DNA

<213> Homo Sapiens

<220>

<221> variation

<222> (368)..(368)

<223> BaySNP:6957
C368T

<400> 59
gggtccaaac cagggctgac tccctgtgtc ccctgacccc tggcctgcgc tgtttatgtc 60

actgtctgta tcaaatgaac tctcctttcc cgctcaaatt tgcacatttt aacaggaaac 120

tttatctcac cagggaaact ggaaaaacca tgtcactttc caagtattag aagagataac 180

gggacaagca gatccaggaa aacaaaaccg ggatatgaaa tcccagcccc ggggtctggc 240

aagggcgggc gacattcacg gcagctcagc ctcagcctga ggacaggaca gacgagggct 300

gagtgygtaa gtctaacaag gtgggcccas gctgacaggt tggacacaga gccagacttg 360

tgtccccycc aaccagaaga ggggtccagc ccacagtccg cctggagggc ttcctgcagg 420

aggtgtctgg gagcccacag tcagcctgga gggcttcctg caggaggtgc ctgggcagag 480

cccctgagca ggcagaaggt gggtagtgga gacccagagc agctcagttt cagcacctga 540
gaagcaggct gccctgggga ggggtgaagc tgaagcctct gaaagcaaac taggcctcaa 600
gccaggtctt caccctccat cgaggggcct gagcctgtgc ccactagggc cgcgtgaaaa 660
gaacaccagt gtgcccagcc cccacggcca gtaactccca ctgccttcgc ccaggtgtct 720
gaccatccac ctctctgcag acagggccag gcccgtctca gccccacctc ggtctgaaca 780
tcccgtccag cccagggaca cagctggtgc ttaatgtttg ctggaattaa gatgcacagt 840
ggggccaggc gcagtggctc acacctgtaa tcccagcact ttggaaggca gaggcgggcg 900
gatcagctga ggttgggagt tcgagaccag cctaaccaac atggtgaaac cccgtctcta 960
ctaaaaatgc aaaattagcc aggcgtggcg ggcacctgta atcccagcta ctcagaaggc 1020
tgaggcagga gaatggcgtg aacccgggag gcggagcttg cagtgagctg agatcgcgcc 1080
accgcactcc agcctgggcg acagagcgag actccttctc aaaaaaaaaa aaaaaaaaaa 1140
aaaaagacgc accgtgggat ttttgcctcc ctggttcaca ggtgaggaaa ctgaggccca 1200
gggagtgggt aacactcggc tcattccaag gccccaaacc caccagaact gccccacccct 1260
gtccatacct tcctttttta gacagattct cgctctgtcc cccaggctgg agtgcagtgg 1320
cttgatctcg gctcactgaa acctccgcct cctgggttca agcaattctc ctgcctcagc 1380
ctccccagta gctgggatta caggcgcccg ccactaatcc acctaatttt tgcattttta 1440
gtagagactg ggtttcgcca tcttggccag gctggtctca aatctctgac ctcaggtgat 1500
cctcctgcct cggcctccca aagtgctggc attacaggtg tgagccccac ccacgcccag 1560
catgtccatg cctttatttt tatttattta tttatttttt gagacccagt ctcactctgt 1620
tgcccaggcc agagtgcagc ggtgagatct tggctcactg caagctccgc ctcccgggtt 1680
cacgccattc tcctgcctca gtctcatgag tagctgggac tacaggtgcc cgccaccacg 1740
cctggctaat ttttttgtat ttttagtaga gacggggttt cactgtgtta gccaggatgg 1800
tctcgatctc ctgacctcgt gattcaccca cctcggtctc ccaaagtgcc aggattacag 1860
gcttgagcca ccgcgcctgg ccttcttctg cctcagcctc cggagtagct gggattacag 1920
gctgggcacc accacaccag gctaattttt gtatttttag taaagatggg gtttcacgat 1980
gttggtcagg ttggtctcga acctctgatc tcaagtgatc ctcctgcctc ggcttcccaa 2040
agtgctggga tcaggggtgt gaaccaccgc acccggcctg tccatgcctt tacacgccct 2100
gcccaggagg gaagtccatc ccgaacaccc ccaacgtgct ttcttcaaag caaatgggca 2160
gaactgggca gggaggggcc cccacggggt cagaacccag cgtctgggaa gggcctgcga 2220
ggtccgtgtg atccggccgg cctcccgcct ctgggcacct tccctggcca gggcagacgt 2280
ttggaatctt ggtcgtcact tccctccacc ctgaccctgc tgcccccgag cacagcctgg 2340

gcctcccgcc cgccggaaac tccaggcctt tggtcccagc ccccacaggg attgccactt 2400

tccccatcct gtccccttcc tgcccccgcc cgagcttcct gcgggcccag ctgctctgca 2460

tccatcccgt tcgaaccatg ggagcgcttg cccgccagct gggctggcgc caggaagccc 2520

ccagggaggc caatgccggc aacacccagc tccagggacc cacgagctcg acgtcacgtc 2580

ccccgctggc cagctccagg gacccaggag ctcgacgcca cgtccctcgc tggctcaaac 2640

gagggcggtg ggaagggaaa ggggtatctc cctgcctgag gccacgcagc cagcctctgc 2700

ccccagcaag ctgccccaga cccaggtgga tgccccaacc tgatctgtcc cgtcctgcgt 2760

cccccttcaaa gcatgttggg tcctggcctg cccggtccac gcatctccct caaagcacac 2820

tgacgactct tcccagccca caccctcccg ggagccagcg gcccccgagg ccccacccag 2880

cccggtggca cccacgctgc agccccccacc tggacaccga gtcctccagc agccgtgtct 2940

tctgctcgtc gtcctgcatc tgcctcctca ggtcctcatc cccctctgag gccgaggacg 3000

gcgtcccctc cagcagccag cgctcccgca gtgccttgga ctgagcagca g 3051

<210> 60

<211> 538

<212> DNA

<213> Homo Sapiens

<220>

<221> variation

<222> (181)..(181)

<223> BaySNP:8816, C181G

<400> 60

gaaaagggtg tagcacctgg gaaaggcgat caactcccag tcagggagcc cacggtgata 60

acaccagtca gacgatcaca ccatggaagg gtccaatgag atccaatgga aagacttaga 120

acatctaagg ttgggggagg tgcaaccttc tgctattcag cccctctgct ccaagcggaa 180

scttttttct caggaggtaa tctctaataa caagcagagt gccctctgga gcctcctcgc 240

tggtatctca gtgcctggac agaggggac acaccacagc acaaacacgt ggcacagact 300

caatcccaac acacagccag tcaacgagcc tctggccccct tcctctgggt cctgatacag 360

agctggcagc gagggcctct gaagaggtta cagggagccc aagggagtgt caggtagagg 420

cagcaccagg tcccggaggg acagatgagg gatccctagt aaacagctcg tggacgcact 480

tgactagcag ttcgaaagca aaaagagatt cggattacaa gagacttttc ccttgcaa 538

<210> 61

<211> 833

<212> DNA

<213> Homo Sapiens

<220>

<221> variation

<222> (166)..(166)

<223> BaySNP:1062, A166G

<400> 61
cctgctgtgc aagaaggaaa ttcacactac accaatgtcc cctgaggcac taagccactc 60

acatccrttt ttctttttca gaacaataaa tctgagaaac mctgtgctcc agcaggtgct 120

ggagggaagg aatgaggtcc tgtgcgtttt gacacagaag atcacracga tgcagaagtg 180

tgtgatctct gagcacatgc aggtcgagga gaagtgtggt ggcatcgtgg gcatccagac 240

caagacggtg caggtgcgtg cagtcgggct gccatgcgtt catggctgag cagtcctgga 300

gaccttggtt gggggcctct ttgggtggtg tcttcattag tcaggactct ttctgtagca 360

agttaacaga aacccaactc acgttgtctt aaatgagcaa gagtgtattg gctcatgtag 420

ctgaaacact tggggaaact ggctccagtc actgctggat ccaggccttg aggatatagg 480

gagctgcttt ccctgggttg atgccctctc agatgggctc tccctatggt gctggcagct 540

ccaggcttgc tcctgatttg ctgcaactcc aggggtgagg ggggcctagg actgggtctc 600

accagaccgt cccgggatat gttcattcct gaagcagggc agggttgcag gcactcgaat 660

gggccaggcg ggctggcgtc aggtgctctt tctgtgggcg cagatgtttc cccaagaaaa 720

tgaacacaca gcactgatca ggccaaatgc agaaaatccc tagagccttt caagctgggc 780

tttacaatga ttacttggaa tagctgctgc aaatcctttg tgggttgagt tag 833

<210> 62

<211> 593

<212> DNA

<213> Homo Sapiens

<220>

<221> variation

<222> (479)..(479)

<223> baySNP: 2463, C479T

<400> 62
```
ggaacccccc gagcgccctg cagacgcagc agcctcttga ggggagggtc tcccccacct      60
cgggctggac aaagacagct tttccccacg tccctctggg ttctctagag caacagcaat     120
acccgcccgg caggggtgtg gcttagagcc ccgcacctcc tcgccgcgcg gcgggcctga     180
cttctagcca cgggtctccg cagttggccc agcgcttcgg gccggtgttc acgctgtacg     240
tgggctcgca gcgcatggtg gtgatgcacg gctacaaggc ggtgaaggaa gcgctgctgg     300
actacaagga cgagttctcg ggcagaggcg acctccccgc gttccatgcg cacagggaca     360
ggggtgagtc cgcgtccctg gcacggagcg gggggtgcat aacacgcccc gggacagtta     420
cgggcgctag ccacgtcggc gatggccaaa taataaacta acagtaatat tatagtaaya     480
gcatccgaag gatgagatca ggattagggc gatggccccc gcgcgttgcc tgcggagcga     540
ggcgcactga gtcgcccagg aatccggcct ctcggcgact gtgcgggaga gtt            593
```

<210> 63

<211> 634

<212> DNA

<213> Homo Sapiens

<220>

<221> variation

<222> (187)..(187)

<223> BaySNP4527, A187G

<400> 63
gggtttcctc tgcccaatcc tcccaccttc cccttcaaac ttaaactcga gcctgctgct 60
ctccggccta gcattgctct tgccatggct gtcagtgatg ctgacatgcg aaagcagata 120
aagcatatga tggctttcat tgaataagaa gccaatgaga aagcagaaga aatagatgca 180
aaggcaraag aagagttcaa catagagaaa ggtcagcttg tggaaaccca aagactaaag 240
attatggaat atcatgagaa gaaagagaaa cagattgagt agcagaagaa aattcagata 300
tccaatttga tgaatcaagc aaggctcaaa gtcctcagag cgagagatga ccttatcaca 360
gacctactaa atgaagcaaa acagagactc agcaaggtgg taaaagatac aaccaggtac 420
caagtgctgc tagatggact ggttctccag ggtttgtacc agttgctgga gcaccgaatg 480
attgttcgtt gcaggaaaca agaccttcct ctggtaaagg ctgcggtgca aaaggcaatc 540
cctatgtaca aaattgccac caaaaacaat gttgatgtcc aaatcgacca ggagtcctac 600
ctgcctgagg acatagctgg tggagttgag atcc 634

<210> 64

<211> 586

<212> DNA

<213> Homo Sapiens

<220>

<221> variation

<222> (274)..(274)

<223> baySNP: 11531, A274G

<400> 64
ccaggggctg gggaaggggc gagccccgtg gggcctggag acagctgagt gactgtgtgc 60
ctcctcccca ggctggaatg agctgctcat cgcctccttc tcccaccgct ccatcgccgt 120
gaaggacggg atcctcctgg ccaccgggct gcacgtccac cggaacagcg cccacagcgc 180
aggggtgggc gccatctttg acaggtgggg gtggtcccgg gaggggcgag ggcgcttcgg 240
tcacctccgc caccaggcca gctgagttca gccrccttgg ccccggtgca catcctgcct 300
agtattattt cagtgcatga agggtgcaca catggaaaaa gagaaaagca tgaggaggag 360
gctcagagcc ctgtgccagc cctgcctctg ggcatctgt aaccacaggg agcacttggc 420

ctatctccct ccatctcttc ttttttcacg atcccatgta tttatccgtg ggaaggaaag 480

cttcgtttct ttttaaagtg attataccgt agatgcagtt ttgtaaaagt gtcaattcca 540

gttggcatgt cgcaggctgc ttgaggctgg cacggtaatg ccgtgg 586

<210> 65

<211> 721

<212> DNA

<213> Homo Sapiens

<220>

<221> variation

<222> (50)..(50)

<223> baySNP: 11536, C50G

<400> 65
tgctgggacc cacctccccc tccccggcca catgccgcgt ccctgccccs acccgggtct 60

ggtgctgagg atacagctct tctcagtgtc tgaacaatct ccaaaattga aatgtatatt 120

tttgctagga gccccagctt cctgtgtttt taatataaat agtgtacaca gactgacgaa 180

actttaaata aatgggaatt aaatatttaa gagctgactg gaagctgact cagttacttg 240

catgtttttc ctggggctga cagggctcca cgcctcctcc acatccagta ctggagggca 300

aaggaggctt tgggctccaa aaccctcccc tgcctccacc tcgctttgct caccgcttgt 360

cagtcaggtg gacgactatg ccatttccgc cctgcagaga gaatttgggg tgtgagggga 420

caaaggactt gtggtgccct ggcctcacct ggtggagcac ttggggtctg gggaagggga 480

aggcccctgg aggaggcgga tgcaggactc aatagatcaa agccagtttt tcatcaccac 540

aagagatcac ggctttcctc tcctttgctg ccacccagct ctctcctgtc tttcctgagt 600

gccatctccc cagcggtcca gtcgagccca gcccccggca gccatgggtt ttgtttgcag 660

tgtgaggcca ggtcagggtg tgtaacagat tatgtgttta gtcaggaaaa aactacagct 720

a 721

<210> 66

<211> 899

<212> DNA

<213> Homo Sapiens

<220>

<221> variation

<222> (173)..(173)

<223> BaySNP:10811, A173G

<400> 66

cctagactca gatggtgaga gacaaccctt tcccttctct ggaatccttc agatttactg 60

aagtgcgagg tgttggaagt cgtcacttct gtgaaatgta gaagatggat tggtttaaaa 120

tttagggtgt agagtcaaac caatgtgagt ttcagttcca gctctaccat gtrgcagcat 180

tgcatcttag gctacttaac ctttgtgagc ctctgatcac atgatttagt gcccacctca 240

ccaggctgtt gtgaggaata aatgaggcag tatgtagaga gcactgttgg catggctggc 300

ccttagtaac acttattctt tgtgtataat aacatggaca attgtagtgc cttctccctc 360

tcagcactgt ggctttgcat ccactcaggt ttttgggatt tccttacaca ttttttcata 420

taaactcagg aggttcccaa gggaacacac tccaagaatt agatgaaaaa aactgcagaa 480

aaatccacct cacatgaaaa tctctgtatt taatattttt aaaaaattac cactgtgttt 540

gttgcagctg tgacctctac aagttccaga gactactctc tgacttttgg tgcaatcaac 600

caaacatggt cctaccgcat ccaccagaac atcacttacc aggtgtgcag gcacgccccc 660

agacacccgt ccttccccac cacccagcag ctgaacgtgg accgggtctt tgccttgtat 720

aatgacgaag aaagagtgct tagatttgct gtgaccaatc aaattggccc ggtcaaaggt 780

aaggtttcct tttttgttgt tgggtaaaaa tgacatcctt ttaagtagag ttagaatgat 840

tcacagaggt tactaagggt tacctagtgc catgctgtgt tatgtgctag gtataagga 899

<210> 67

<211> 2956

<212> DNA

<213> Homo Sapiens

<220>

<221> variation

<222> (541)..(541)

<223> BaySNP:288, C541G

<400> 67
cgccagcgtc ctgctgaagc actcaaaggc cgacggcctg gcgggcagca gacacaggta 60
tgccgagcag gaaaacggaa tcaaccaggg gagtgcccag atgctctctg agaatggcga 120
actgaagttt ccagagaaaa tgggattgyc tgcagcgccc ttcctcacca aaatagaacc 180
cagcaagccc gcggcaacga ggaagaggag gtggtcggcg ccagagagcc gcaaactgga 240
gaagtcagaa gacgaaccac ctttgactct tcctaagcct tctctaattc ctcaggaggt 300
taagatttgc attgaaggcc ggtctaatgt aggcaagtag aggcagcgtg ggggaaagga 360
aacgtggctc tcccttatca tttgtatcca gattactgta ctgtaggcta aaataacaca 420
gtatttacat gttatcttct taattttagg tttctgttct aaccttgtca ttagagttac 480
agcaggtgtg tcgcaggaga ctggtgcata tgctttttcc acgagtgtct gtcagtgagc 540
sggcgggagg aagggcacag caggagcggt cagggctcca ggcatccccg gggaagaaag 600
gaacggggct tcacagtgcc tgccttctct agcggcacag aagcagccgg gggcgctgac 660
tcccgctagt gtcaggagaa aagtcccgtg ggaagggccc tgcaggggtg cagggttgca 720
cgcatgtggg ggtgcacagg cgctgtggcg gcgagtgagg gtctcttttt ctctgcctcc 780
ctctgcctca ctctcttgct atcggcatgg gccggggggg ttcagagcag tgtcctcctg 840
gggttcccac gtgcaaaatc aacatcagga acccagcttc agggcatcgc ggagacgcgt 900
cagatggcag atttggaaag ttaaccattt aaaagaacat ttttctctcc aacatatttt 960
acaataaaag caacttttaa ttgtatagat atatatttcc ccctatgggg cctgactgca 1020
ctgatatata ttttttttaa agagcaactg ccacatgcgg gatttcattt ctgcttttta 1080
ctagtgcagc gatgtcacca gggtgttgtg gtggacaggg aagcccctgc tgtcatggcc 1140
ccacatgggg taaggggggt tgggggtggg ggagagggag agagcgaaca cccacgctgg 1200
tttctgtgca gtgttaggaa aaccaatcag gttattgcat tgacttcact cccaagaggt 1260
agatgcaaac tgcccttcag tgagagcaac agaagctctt cacgttgagt ttgcgaaatc 1320
tttttgtctt tgaactctag tactgtttat agttcatgac tatggacaac tcgggtgcca 1380
cttttttttt tttttcagat tccagtgtga catgaggaat tagattttga agatgagcat 1440
atattactat ctttaagcat ttaaaaatac tgttcacact ttattaccaa gcatcttggt 1500
ctctcattca acaagtactg tatctcactt taaactcttt ggggaaaaaa caaaaacaaa 1560

aaaaactaag ttgctttctt tttttcaaca ctgtaactac atttcagctc tgcagaattg 1620
ctgaagagca agatattgaa agtttcaatg tggtttaaag ggatgaatgt gaattatgaa 1680
ctagtatgtg acaataaatg accaccaagt actacctgac gggaggcact tttcactttg 1740
atgtctgaga atcagttcaa ggcatatgca gagttggcag agaaactgag agaaaaggga 1800
tggagaagag aatactcatt tttgtccagt gtttttcttt ttaagatgaa cttttaaaga 1860
accttgcgat ttgcacatat tgagtttata acttgtgtga tattcctgca gtttttatcc 1920
aataacattg tgggaaaggt ttgggggact gaacgagcat aaataaatgt agcaaaattt 1980
ctttctaacc tgcctaaact ctaggccatt ttataaggtt atgttccttt gaaaattcat 2040
tttggtcttt ttaccacatc tgtcacaaaa agccaggtct tagcgggctc ttagaaactc 2100
tgagaatttt cttcagattc attgagagag ttttccataa agacatttat atatgtgagc 2160
aagatttttt ttaaacaatt actttattat tgttgttatt aatgttattt tcagaatggc 2220
ttttttttt ctattcaaaa tcaaatcgag atttaatgtt tggtacaaac ccagaaaggg 2280
tatttcatag tttttaaacc tttcattccc agagatccga aatatcattt gtgggttttg 2340
aatgcatctt taaagtgctt taaaaaaaag ttttataagt agggagaaat ttttaaatat 2400
tcttacttgg atggctgcaa ctaaactgaa caaatacctg acttttcttt taccccattg 2460
aaaatagtac tttcttcgtt tcacaaatta aaaaaaaat ctggtatcaa cccacatttt 2520
ggctgtctag tattcattta catttagggt tcaccaggac taatgatttt tataaaccgt 2580
tttctggggt gtaccaaaaa catttgaata ggtttagaat agctagaata gttccttgac 2640
tttcctcgaa tttcattacc ctctcagcat gcttgcagag agctgggtgg gctcattctt 2700
gcagtcatac tgcttattta gtgctgtatt ttttaaacgt ttctgttcag agaacttgct 2760
taatcttcca tatattctgc tcagggcact tgcaattatt aggttttgtt tttctttttg 2820
tttttagcc tttgatggta agaggaatac gggctgccac atagactttg ttctcattaa 2880
tatcactatt tacaactcat gtggactcag aaaaacacac accacctttt ggcttacttc 2940
gagtattgaa ttgact 2956

<210> 68

<211> 802

<212> DNA

<213> Homo Sapiens

<220>

<221> variation

<222> (72)..(72)

<223> BaySNP:2371, A72C

<400> 68
ctgcggtgta acggtgtcct ggagggcatc cgcatctgca ggaaagggtt cccaaacagg 60

attctctatg gmgattttaa acaaaggtgt gtaataaaca tgttctatat gcttggggat 120

gagatgtacg aacactggaa tttgagaggg agaaagattc gtatgaaaag acctatttcg 180

aaagaacaga tatggacctc catgtaaact gaacaaatct accatttggc caagttatat 240

atatttgttt cttccatgtt tttctctgga atttatctga tatacaaaat ggcaggaaaa 300

gctctgactg aagagtcaag agatctagat tctagactgc tctctagcaa tgtgactccr 360

agctagttcc ctcatctcct gggaccttgg ccttcygcct ataggaagag aggcctgaac 420

tacatgttct ccaaggtttg tgtctttgcc aacagtctgt gatgatttcc rtgtctacaa 480

tgcagatacc gagtgctgaa tgccagtgca atccctgagg gacaattcat tgacagcaag 540

aaagcctgtg aaaagcttct ggcatccatt gatattgacc acactcagta caaatttgga 600

cataccaagg taatgcatca gttctgacag atgctggcct tttcgtcctc ttgacacagc 660

ttctctagga aactgactca tgtcaccctt ctgccccata aggtgttctt caaaggctgc 720

ttgctgggaa ccctggaaga gatgcgggat gaccgcctgg caaactaata ccccgacaca 780

agctgtgtgc agagggtttc tc 802

<210> 69

<211> 801

<212> DNA

<213> Homo Sapiens

<220>

<221> variation

<222> (343)..(343)

<223> BaySNP:4383, A343G

<400> 69
tcaacagtaa gtgtggcagt gatgatggca ggacgtgcag acactgaaca cgaaaccagg 60
accacggctg gacaaatcca acaatcgtct acccctgcag agtcaggtgt taaagcatta 120
ggtggcatat tagttttcag agataactac ttacacaatt atcttcaatg tgccacaagg 180
tagagtcatt ttgtagaaat gtgtgtaaat gtgcaccaca cttacaggca cccacccctc 240
tgcaacccaa gcagcacttt gttgcagaca gcaatatgta ccagaacaag agtccctaaa 300
cccaccctct cctagcgcct tacacagaac gctgctgaac ccrggccgaa tctgagcccc 360
aaagctggtg gtgtatgtgc caccctcaca tatgcatgca ttacaagggg catgaccaga 420
ggggtggaca cagccctcag catgctcgct ccttctagaa ctcaacgaaa agtgcctgaa 480
ccgtttctca tcatgaaggt tttaacaaag gtaactggtt gcctagaata cttacctcat 540
aaagtcactc tctcaatttc agaataactt tggagcaggc ggtcacacag ccaccaccca 600
ctaaagtcag agacactctg ggctctcacc tggacgaggc tcctgtccat cactacacca 660
cacaaaacct gggactgagg gcccgccgtc ttaatgtcct gtaaattctg gctctcgatt 720
ctgaggtggg aaggagagca cacatcaaca gtcctgacaa caatgctgct tggaaccctg 780
ctgcttgtgc acttgggccc c 801

<210> 70

<211> 501

<212> DNA

<213> Homo Sapiens

<220>

<221> variation

<222> (402)..(402)

<223> baySNP: 11654, A402G

<400> 70
ggggattgga aatgctgctc aagagaggga aaataggata tcacgattgg ctcaacaatt 60
ccctgtgtta agagcggtcc ttttctattt ggtaggttgt ggggcaaata catagctagc 120
tcaggtgatg aaatctttca tctctttagt ttgtgtactt ttaaccaagg actgcgtatc 180
tcttgccttt cttgtggttt tcacctgcaa cttaataatt ataccattgt acaccttatc 240
ctcttttccc atattcaaga aagattatct ccaactctta ctgttttaaa actcatgtgc 300
ttacttcaaa tatttctgca ctaaagctgt aatattacaa gtttgttttc ctattagagg 360

tttccatttc catccattta tcttttactt gaaaggacac crtatttttc acctaatccc 420

tcaattttat tggtggggaa ggcataagag aagttcactg catacaatct aaggtctaag 480

atcccccaaa atttccagca g 501

<210> 71

<211> 501

<212> DNA

<213> Homo Sapiens

<220>

<221> variation

<222> (331)..(331)

<223> baySNP: 11655, A331C

<400> 71
ggggattgga aatgctgctc aagagaggga aaataggata tcacgattgg ctcaacaatt 60

ccctgtgtta agagcggtcc ttttctattt ggtaggttgt ggggcaaata catagctagc 120

tcaggtgatg aaatctttca tctctttagt ttgtgtactt ttaaccaagg actgcgtatc 180

tcttgccttt cttgtggttt tcacctgcaa cttaataatt ataccattgt acaccttatc 240

ctcttttccc atattcaaga aagattatct ccaactctta ctgttttaaa actcatgtgc 300

ttacttcaaa tatttctgca ctaaagctgt matattacaa gtttgttttc ctattagagg 360

tttccatttc catccattta tcttttactt gaaaggacac cgtatttttc acctaatccc 420

tcaattttat tggtggggaa ggcataagag aagttcactg catacaatct aaggtctaag 480

atcccccaaa atttccagca g 501

<210> 72

<211> 1004

<212> DNA

<213> Homo Sapiens

<220>

<221> variation

<222> (489)..(489)

<223> baySNP: 11450, A489T

<400> 72
taaataaata aaataattct tggaatgttt tttcttctat atcccactat ccttggtcag 60
atttttttct tatttgtgtt actgtctatt aaatgtattt tctcttaagc tgagacacag 120
atctcttaag actgatggtt tagaatatag cagaccatat ttttatgctg ctttagactc 180
ttgtccaggt gaaaaaattt ttaataaatg ttgtgaactt ctgataacaa tctctatttt 240
gtttagatac atctcttttt ataattaaaa agtagtcaga tactaagcaa aaacagaaga 300
acaacaacaa aaccccacag ctactaaaag atgaagagac attgaattga gaattaagca 360
aaaccccttgc aggcagcagg acaggccaga gctgaagaag ctgggagtgg tgaggtgccc 420
ctaggggaca ctgacagagc cttgaaccca ctggtgcagg aggcagggat gagtgaagcc 480
tgtctctgwc ttcaagatta tggtcccaca gtcctctctt tacctcatac gcattgcctg 540
attttgaaga gttaaggttt ccttgaagca caggtgcctc aacctctata ccacgaaaga 600
cacctctaca gattctaaga atcaccttac acatgggtgc catatgtttt acctggggcc 660
actgttcttt aatatcatcc cttccctcag gtttccatct ggattccaca aaagccaccc 720
ttcctgaagt tggtccctct caccggattt atccgaagac ctcctctctc attttccgac 780
cattccttta ggtctgcctc taaacaagag gccaagcttg aaattcaagt gtgaaacaaa 840
ctcctggcaa aatggcagta tcttagaagg tgtgagatat tcttgccttc caggaatagt 900
tgcatttaaa aagacagatg aggccaagga gggcagatca cctgaggtca gcagttcgag 960
accagcctgg tcaacacggt gaaacccccct ctctactaaa aata 1004

<210> 73

<211> 1004

<212> DNA

<213> Homo Sapiens

<220>

<221> variation

<222> (414)..(414)

<223> baySNP: 11448, A414G

<400> 73
taaataaata aaataattct tggaatgttt tttcttctat atcccactat ccttggtcag 60
attttttctt tatttgtgtt actgtctatt aaatgtattt tctcttaagc tgagacacag 120
atctcttaag actgatggtt tagaatatag cagaccatat ttttatgctg ctttagactc 180
ttgtccaggt gaaaaaattt ttaataaatg ttgtgaactt ctgataacaa tctctatttt 240
gtttagatac atctcttttt ataattaaaa agtagtcaga tactaagcaa aaacagaaga 300
acaacaacaa aaccccacag ctactaaaag atgaagagac attgaattga gaattaagca 360
aaacccttgc aggcagcagg acaggccaga gctgaagaag ctgggagtgg tgargtgccc 420
ctaggggaca ctgacagagc cttgaaccca ctggtgcagg aggcagggat gagtgaagcc 480
tgtctctgtc ttcaagatta tggtcccaca gtcctctctt tacctcatac gcattgcctg 540
attttgaaga gttaaggttt ccttgaagca caggtgcctc aacctctata ccacgaaaga 600
cacctctaca gattctaaga atcaccttac acatgggtgc catatgtttt acctggggcc 660
actgttcttt aatatcatcc cttccctcag gtttccatct ggattccaca aaagccaccc 720
ttcctgaagt tggtccctct caccggattt atccgaagac ctcctctctc attttccgac 780
cattccttta ggtctgcctc taaacaagag gccaagcttg aaattcaagt gtgaaacaaa 840
ctcctggcaa aatggcagta tcttagaagg tgtgagatat tcttgccttc caggaatagt 900
tgcatttaaa aagacagatg aggccaagga gggcagatca cctgaggtca gcagttcgag 960
accagcctgg tcaacacggt gaaacccccct ctctactaaa aata 1004

<210> 74

<211> 1516

<212> DNA

<213> Homo Sapiens

<220>

<221> variation

<222> (1137)..(1137)

<223> baySNP: 4018, C1137T

<400> 74
gcccagctga ccatgaagga cagcaaccag ttccacgcca cctgcctcga caccttcccg 60
cccatctctt acctcaatgc catctcctgg cgcatcatcc acctggtgca ccgcttcaac 120
gcccaccacg gggacaccaa ggtgacgcgg gccgggaggg cagaggctgc gctcgcgctg 180

tagcgcgtgt ctaggcttgt gctgccgtgg agacggcccc ggggctcgcg tgcagggcaa 240
tctgtgtctg aaagctccac cctgctgtgt gcgaagctca ctgaccatct gggttcttct 300
gtttcatttt gtttcttgac ttgcccgggt cacggcccca ccaccttctt cctgggttca 360
caggcagacg tgtcaagtac caggcacttg ggtcacagc tccactccag tgtggccctg 420
agggcctaga ctgagctctt ggagagccca aggccgcctg gcagaccaga gggttgggga 480
gacaagggtc aggcaccacg tgaatagcca gggtgactgc tgtgggttcc aggtggcgta 540
caccttgac gcgggcccca atgccgtgat cttcaccctg gacgacactg tggctgagtt 600
tgtggctgct gtgtggcacg gctttccccc aggctcgaat ggagacacgt gagtgtggac 660
cccgcccctc ctcctttttg ggagatggtg ccaggaactc tggtcggaag agacggaggc 720
acctgaaggc tgtggcgctg agacagggag tcccagggca ggcagcagtc ccccgagtgt 780
gggagccacg ggcagggtgt ccccatagag ccatcccaca gcccacgtcc acagggaaga 840
aagagaggcc gctcacgcgt gtgtgagcaa cgcccagggt ctcccacctc ctgagtgaca 900
gcgtcccctg cagctcggga ccccgcaggc tcttattcta tagaacagtg gcttaggaaa 960
cacgcctgca ctcccagatg tgtgcgtaag tgtccgacat tttgaaggaa aggagtcaaa 1020
gcccctgatt tccttcttac tgaagcgagt tagagccagt caaggcggcc gggtgttgac 1080
cccgccccgg cagttgggtg tcttttcttg ggtggggtcc ggcagctgca tcttggygcc 1140
acagagcccc catgtacgcc ctcctcggtg cacagcttgg ccctgaaccg cctcttcccc 1200
tgcgctgctc tgtgggggtg ccacagagcc cccatgtacg ccctcctcgg tgcacagctt 1260
ggcccagcct ccttacgccc cggggctgtt gatttcttcc ctcccgtgtt ttcatggtgt 1320
cttattctca atgtgagagg cgcctcccat ttaagggtgt ctggaagcgt gtggggatgt 1380
gcgtctgtcc cgggtgggag caataacacc ttctaatggt caggggcctg ccctgtgggg 1440
tggactcaga cccacacccc aggactgccc gcctgggaag caccaggctc gtggctgctg 1500
ctccgggtgg ggcttg 1516

<210> 75

<211> 662

<212> DNA

<213> Homo Sapiens

<220>

<221> variation

<222> (281)..(281)

<223> BaySNP:2217, G281T

<400> 75
cagtgcccat tttccatgga cagcctgtat tgaagtttta tttttttct atacacgtga 60

ttgattaaca atataattaa tgggatgtca ttttataccc tgacaagttt aaatgtaaat 120

aaattggcac cacctttaat cagactgatg tcctgacttg cacaggaaac acgcacctaa 180

cctcaccaac ctcaagccct ccctcctcaa gtctccatgc aaagactgtg ccagtcagag 240

tggagctgtc ccccgacga gcccccagaa gcacccggcc kgccatggct gtcattttga 300

gggcggaaaa taactggatt tctggttaat tggtgacatc ctcaagagct gtggcaagcc 360

atgtctcggc cgcgtgatgg ggcagacatg gtgggtcctg acggccttgt cttcaccaag 420

tgctcctaac tctcagcaaa cttggggttc atgaccgtcg tggtggcgct cttgaaaagg 480

ggattatcct ggtgggaaat gcaaacaggg gcttgtgagt gtgggaggtt ttcagagcag 540

aatctttctc cctgaggaca ctccccgcat ggaagccgtc actttgagga agagctagac 600

gtggggcagc ccccccaggg tcccagcacc ggcagccaag ctcacccaca gcggcggacg 660

cc 662

<210> 76

<211> 1025

<212> DNA

<213> Homo Sapiens

<220>

<221> variation

<222> (807)..(807)

<223> baySNP: 4966, A807G

<400> 76
aggtctcacc taatatcatc agaattctgt ctttaactct tattctgtaa atgtgttttg 60

tagagtgttg acttatgctt gggtgttgca gaacatgttg gaccatgtag gagccatgtc 120

aagacatgct gggtgtgctg taccatgtca gggtgtatca ttgctctata gtgttgttgc 180

agcctagtcc ggtccccttt atgcccccca ccctcctttc ttcttctgcc catggcctcc 240

ttcctgctat gctgggcttg gagaaaagac gtccaaacct ctggctggga gccaccctcc 300

atacccaaag tccctcctct attcctctcc atggaccctg atggtcctca ttcatgtcag 360
atgccattac agacaaggac atagtcttct acaagaccct gaagccctgg ctgggtaagt 420
aactgtaggt ggacgggacg gggaccaact tttgtggcca ggggaagatt ctgcccttgc 480
ccacagcctt tggctgccgt actaggggat gggctcttgt taagtgttgg tgacaagtgg 540
agacaccacc gtcgcttgtg acgcctgcct tccatttcaa catcctgaag ccctatataa 600
agattttcag caagagtgca aacatcatgc atgtgagtgc cttgaactca gcatcccagc 660
tgcagccttg ggtggagggg atcacataca attgggtctg gaatgttggc tctcctgggt 720
gggtttgggg ccattgctct tcctctctgt gccttgattt ccccatgagg ctaataatcc 780
tcactaaaag gtggtaggag catgtartgg actcatgtct ctgacactta gtaggtggtc 840
agaaggagtc aatttccaca tttttctcac agaagccctg taaactcaag aaaggaatga 900
tgacacgtgc atagtagtca ttgctgagta cagatcactt caaaactaat tggtttagag 960
caataagggt ttatatttgc taacaaatct gtgggactgt tggccagccc ttctgcatgt 1020
ggata 1025

<210> 77

<211> 1281

<212> DNA

<213> Homo Sapiens

<220>

<221> variation

<222> (220)..(220)

<223> BaySNP:2284, A220G

<400> 77
catggtcaca actgtctgtg tgacctgtct gtgggagggc agaccccccct cctgccaagc 60
cacagtagca gctgacctgc caggcaaaag cattcctttg taaaggaaac cctcccagac 120
cctctgacca aaggggacgg gtggggactt cctggcactg cagggcccct tgcctggtac 180
tcccagcaac tggttgcggt ggggagggca gatctagggr tggggatggg gaggagaagt 240
gggaatggga aattgggagt cagaggggca agtgggagag aagggcgcac ctgccgggat 300
aacaggccag atgaagtaaa tagaaaatcc tctgagctcc cctactggct ccagctgtgg 360
agaaggggga ggagaaaacc ctgtgggaca ggggaggagg gtgagggctc ctcttaggaa 420

gttatttaag agccaactgt cttgtctttc ccgagtccgt ttgaggaagt ccccgaggcg 480

cacagagcaa gcccacgcga gggcacctct ggaggggagc gcctgcaggt aagccaccga 540

ccctgcaccc tatgagccag ggcccgctgc gtccaccttc tgcacctcgg tttcctggtt 600

gaaccagcaa gcggcttgct ctgggccctg tggcgccggt cacaggcagc tccacttgcc 660

caatcctggc ttcccgcccc caactccgca cctgcccagc ccagcttcta agcgggactt 720

ctctcaggct ctcgaggcag ctctcccgaa gccgttgctt gcttagaatg ccaccaaatg 780

gggatggggg agttggaggt gcttccccaa gggacgagcc ccctttcctc agggagcaga 840

ttatggagct ggaacacaaa aggtggaggg agtggggcca agtgagaaca gcctgctaga 900

ctggaacccc gccggcttgg ctccccactc ctgcgcgcct ccccactcct gcgcatcgcc 960

tgcctccgtc ccaatttgga gctgaactcg agccacttct cctccttaga cccgcccaga 1020

agcgagggct cctgcccgcc gcactcaccc agccaggcgt gcacctgggg ctcccccctt 1080

ctccccctta gcactgtgtc tcccgacagc gcgccacact gttctgcacc cctggcaatg 1140

ccgcagccta ccttcggtat ttctcactag aggatggctc tcgcagcctt ctccctatca 1200

ttaacccttt ttcgctcttc tccctccctt cccccgtcag ctcaccaatg actgtaaata 1260

tatttatcta tacttatgga t 1281

<210> 78

<211> 823

<212> DNA

<213> Homo Sapiens

<220>

<221> variation

<222> (290)..(290)

<223> BaySNP57, C290T

<400> 78
tgtcactgaa gtgtagagct cctgaagtct ctcaatacat ctatcaggtc tacgacagca 60

ttttgaaaaa ctaacaagac tggtccagta cccttcaacc atgctgtgat cggtgcaagt 120

caagaactct taactggaag aaattgtatt gctgcgtaga atctgaacac actgaggcca 180

cctagcaagg tagtaactag tctaacctgt gctaacatta gggcacaacc tgttggatag 240

ttttagcttc ctgtgaacat ttgtaaccac tgcttcagtc acctcccacy tcttgccacc 300

tgctgctgct atctgtcctt acttgtgggc ttctccatgc tgtgccaatg gctggctttt 360
tctacaccct cttttgagtg tagtttggta ttttgtaatt gagagctcat ttcaaaagca 420
gaaaaagaca acaaatatta aagcaaggaa aagtgtaact gaaacactgc actttactgt 480
tttatacttt tgtacatatg agaaatcaag ggattagtgc aaccagtaga aagcattgaa 540
atgactgtca ttaaccacac agtcctggag gcagagatgc agttacctac cctagctttt 600
gatgggttct cttacctgta gtagccttat ccctggtcat ttggattttc agtttgcttt 660
tttcttttt tcccctccaa actccttttc cttggccaag ccttcatgct tcccccttc 720
catattataa tctcatttga ttgctctgca gttgggaacg gagatcctct tgaatgatgt 780
ttcagtgtgc aaaactatag agcctgtcag caccaagctg aca 823

<210> 79

<211> 1191

<212> DNA

<213> Homo Sapiens

<220>

<221> variation

<222> (1049)..(1049)

<223> BaySNP:11614, C1049T

<400> 79
ggctaatttt ttttttaat ttttagtaga gacagtgttt caccatgttg gcccggctgg 60
cctcgaactc ctgacctcaa gcgatttgcc caccttggcc tcccaaagtg ctaggattac 120
aggcatgagc caccgcgccc agtctggtta tgcagttagt tgataccaga gaggagtagc 180
ttgaatgtgt ggacttgcct tcgctataat aaaacatagc ctctatcccc attataaaat 240
ggggaaagaa gttagaaaac caacctgatt ttaagatgat gaggtgaaga aaaagcagct 300
gcttagaagt tgctgtatgt atcatttagt tgtttggtgt tggttagaag aatcactctt 360
attttaatat ctaatgaact gaaaagtgag agtgaggtat tttccttccc tttttattta 420
tttgtaattt gattcccagg gaaattaaga cttgtgacaa gcccaaatga agatattttg 480
gaatttgaca ggagagtttt ttaacttatg cctaagcttc aaattatatt aggcaataaa 540
ttattgatag tttttaatca gatactggaa tatggaattt tcaaaatcac aactcagaaa 600
aatgaccgct cacaggcctt ctgttttata tttattcttc ctttttagta ttctccaaaa 660

ttaatcatca tcgtacatgt gtacatttat tcttattatt accacaagag ccttctgatg 720
atcgagagga gagagaccct cagttcacct ctggaaggga agttgttgta catgagcaca 780
gggaggaatc agtctcaaga gccagacctc ctgaaaacct gcagcgttgc tgagagaatc 840
agcagcttta ctggctgaac agcagctcgg gttagcccat ccccgtggcc aggtcatttg 900
ctggctcctc cgcagccagc ttccattgtt gtctaggctg acagctccgc cctactcatg 960
acgtgactct ctctatggga ttgtttctct cctttttttt ttttacattc agttttcaga 1020
agacagaatg gaatacttct cattcattyc tccagcaaag tgcccctggt gggcagagct 1080
ctctctccca ggccttaact ggcctaaata aagtgggtgg cagctgcctt ccagacagat 1140
gccagcctgg tactagggta gtatgattgt gtgatacaaa gctgccaatg t 1191

<210> 80

<211> 488

<212> DNA

<213> Homo Sapiens

<220>

<221> variation

<222> (89)..(89)

<223> BaySNP:11645, A89G

<400> 80
ctggtggcac cgtgcacctg gagatcggcc tgctgctccg caacttcgac cgctacggcg 60
tggagtgctg agggactctg cctccaacrt caccaccatc cacaccccgg acacccagtg 120
atgggggagg atggcacagt ggtcaagagc acagactcta gagactgtca gagctgaccc 180
cagctaaggc atggcaccgc ttctgtcctt tctaggacct cggggtccct ctgggcccag 240
tttccctatc tgtaaattgg ggacagtaaa tgtatggggt cgcagggtgt tgagtgacag 300
gaggctgctt agccacatgg gaggtgctca gtaaaggaga gcaattctta caggtgtctg 360
cctcctgacc cttccatcct tcaggtgtcc tgttgccccc tcctcccact gacaccctcc 420
ggaggccccc atgttgacag accctcttct cctaccttgt ttcccagcct gactctcctt 480
ccgttctg 488

<210> 81

<211> 846

<212> DNA

<213> Homo Sapiens

<220>

<221> variation

<222> (375)..(375)

<223> BaySNP:384, C375G

<400> 81
ctgcggtgca gcgtgggtcg tcatcgctct cacggcccac actgtattcg tcagctcgcc 60

cctgctgggc agcgtcatcc tctcccacag gccccctcag gtcccggctg gtcctcctga 120

catgttgtcc tgaggagctc ctgccccaga gccttgtgtg tctcccctgc actacccggc 180

atagcccagc ggtggggctg gagaggggtc tacgcctgag accaccgccc gtctgtccca 240

ggagcctgtc cagggccagg cgctgcactg gctcaggcct gcacggtggt ggagaagacg 300

tgcgtggtga acacgacgcc ctccccgccg atctccgttt ccaggccatc tggatgatgg 360

accccaagga tgtcsgtgag tggcaacatg aggagttcta ccgctacgtc gcgcaggctc 420

acgacaagcc ccgctacacc ctgcactata agacggacgc accgctcaac atccgcagca 480

tcttctacgt gcccgacatg gtgaggcact gtggcagtcc cacggccctc agggtgactc 540

tgttgtcccc tgtggcatca gctctgcaga tgtcacatga ttaacatggc agtgtgaggg 600

catgaaaaac ataggtagtg ctgggtgtgg tggctcacgc ctgttatccc agcactttgg 660

gaggctgaga ggtggatcac ctgagcccag gagttgcaga ccaggctggc cacatagtga 720

gaccccatct ctgaaaaaaa aaaaaaaaaa catatgcctt tgggtgggga accccagagg 780

ttccttttc tcaggggtca cacatggcga ggaaggattt gatgggatct cattgcagct 840

ggggtg 846

<210> 82

<211> 832

<212> DNA

<213> Homo Sapiens

<220>

<221> variation

<222> (402)..(402)

<223> BaySNP:542, A402G

<400> 82
tgaggatcaa gtaacttgcc aaggtcacag agttcactta ctactcagtg agcttgggta 60

aatggctgaa ttttgctgtt tcagtttctc cataataaaa ggattattat aataatacgt 120

aaagtgttgc tgacaaggtg aaatacataa tttcactttc tgagtactta agaacagtta 180

gctgttatta tcaagcaaat ttgtatcaag tgttcccaca cattcagcca tgttgctgtg 240

gtactgcctg gcttgtatag gctgctggac aggcagaggc tccttcttcc taaaagccct 300

tggcagtcac attatattta cctgtcttct ggtattaagc cgtaatttgc atgattgagc 360

cagttgttta tctttcgctc catcaaccaa gtcacaattg grgttgggag ggaatttctc 420

aacatgttct gaaagcgtgt catgaacacc atgtcccatg ggtagcccga gtcaaagatt 480

cggctgatca cccatccccc tccggtggtg ctgaggaaca cctggaagca atcaaagaca 540

tcccttcatt tgacactgtg aacacggctg gctggttctg ccagagattc aagaagtgtc 600

atgaatggaa aggcactgaa aagtctgcac tctcaaaata caccccctgac aagatagtct 660

tggttcttct aacttctatt ttaaagttaa tccttttgag attaggattc agagcatcta 720

gagtattaaa atgccttaag acatattatt gatacttatt atctgccagc ctttagtaaa 780

ccatagggac cttaagtcta cgtaagaaac aatgaacagt gattgctact tg 832

<210> 83

<211> 582

<212> DNA

<213> Homo Sapiens

<220>

<221> variation

<222> (404)..(404)

<223> BaySNP2290, A404G

<400> 83
atggcaattg ggagtaagac tttttagtaa agaaactaaa cacaaagtca ttagactctg 60
taaaagtctt accaaatttg attctggaac acctattcta tttccgtaaa gatgatgaat 120
tcggagccaa atgttctttt catgaaggat ttgaaaactg tccatgaaaa taacgcaatc 180
aacctttttag cttgagactc tattcactga ttagattttt ttaaatactg atgggcctgc 240
ttctcagaag tgacaaggat gggcctcaat ctcaattttt gtaatacatg ttccatttgc 300
caatgagaaa tatcaggtta ctaatttttc ttctattttt ctagtgccat ttccatgtgg 360
aagagtttct gtttcacaaa cttctaagct cacccgtgct gagrctgttt ttcctgatgt 420
ggactatgta aattctactg aagctgaaac cattttggat aacatcactc aaagcaccca 480
atcatttaat gacttcactc gggttgttgg tggagaagat gccaaaccag gtcaattccc 540
ttggcaggta ctttatactg atggtgtgtc aaaactggag ct 582

<210> 84

<211> 549

<212> DNA

<213> Homo Sapiens

<220>

<221> variation

<222> (229)..(229)

<223> BaySNP:2093, C229T

<400> 84
agtgcttatt ggacagcctt cagcccgaaa ggggcaaata agagaccagt ccccggtgga 60
ggaggggcac ggggcctccg agctccagct ccgttcccaa ggatactcgt gaagacccca 120
tctgtgttca tggcctggaa agagacttct cccatagcaa agaggctgtt ataaaagcaa 180
taacttttgt gtttgtgtgg gatgatttat ttaatttttt agtttcccyt ttgattgctg 240
agagccattt tcctttacac ataactacac ctgacaccag gctctgctgg atgtgagttt 300
ccactgcatg ggctgtgggc tgggcctgtg gtgcctgccg agtggtcact gtcagtggga 360
aacccgttgt tcctcccgtc ttcagatgct gagccaactg cttggacagc agccagcgcg 420
tcatgacgtg catgagaggg ggaccctggt gctcatcttc tcttgtcatt catccaggca 480
tgggctgcca ggttttgtcc ctgctcgttc aacagtgtga gcatttgtct ctgttatcta 540
atgatgttc 549

<210> 85

<211> 590

<212> DNA

<213> Homo Sapiens

<220>

<221> variation

<222> (467)..(467)

<223> BaySNP:11585, G467T

<400> 85
aaacagaaga accacccagc cgagacagaa gaaccgccca gccaagccca gcctcaatca 60

ctgacctgca gatgcatgag cctaataaat gattattgtt tgaggtcact gatatttgag 120

gtgatttgtt atgcagcatt attttgtcaa tagataactg atacaaactc atataaaagg 180

ccccatctgt ttactcgcat attcaggtgt tccaacacaa ccttgtgaaa cacccactct 240

gtaaccaggg atagagcttc agcaaagcct tcgtgaagcc cacagctcag aagagagaga 300

aagattaaga cccaagtgct cactacacgt gattaagtga agagcccagg catagcctat 360

ggcaagagca cacagccaag gcttgagaga tcaaggaagt cttcccggag gaggagtgat 420

caaaatagag gcccgaagaa gaaggggggag aagacagatt gggggtkgca tccaggcagg 480

aacagaagaa gttccgggtg gaaaaaacaa ggataaatgc cgtggctggg tggcggggag 540

tgcgtggatg tcacagcctg gctgcttgga agaattgcaa ggcctttggg 590

<210> 86

<211> 481

<212> DNA

<213> Homo Sapiens

<220>

<221> variation

<222> (266)..(266)

<223> baySNP:777, C266T

<400> 86
acgccccgct gcggcccggc ctgggggcgc cgcgagctgg cgcggcgcgc cccgcccctc 60
cccgcccctt ccgcccttca ttcattcttc tttctcccag cgacaggcct cggctgggag 120
ctgcgaggcc cagctgtctc ggagggccgc tagctctgct ggggcaggcc aggaggatgt 180
aggagcctag agaaggggcc tccgcttccc tgcgcgaggc cccacagac ccaaaccagc 240
tgcagccggg aactcggatc agagcyggag aggccctgtg gaccccagg acactggtgg 300
tcgccttggg gaggggaggg aggcgggccg ggttttgtcc ctctctctga ggcctgcatt 360
gaggccaggc tgggcccggg gaaggtgacg gtccgcgtct caggcctcgc gaatgagcag 420
caccagagaa aggagcgagc ccctgccagg cggtgacagg tgtactcttc tcagtgccga 480
t 481

<210> 87
<211> 1161
<212> DNA
<213> Homo Sapiens

<220>
<221> variation
<222> (349)..(349)
<223> baySNP: 2000, C349T

<400> 87
aaccacccta accctgactt ccctaattcc ccccatcctt accaccctcg ttaaccctaa 60
caaaaaaaac tcataccccc attatgtaaa atccattgtc gcatccacct ttattatcag 120
tctcttcccc acaacaatat tcatgtgcct agaccaagaa gttattatct cgaactgaca 180
ctgagccaca acccaaacaa cccagctctc cctaagcttc aaactagact acttctccat 240
aatattcatc cctgtagcat tgttcgttac atggtccatc atagaattct cactgtgata 300
tataaactca gacccaaaca ttaatcagtt cttcaaatat ctactcatyt tcctaattac 360
catactaatc ttagttaccg ctaacaacct attccaactg ttcatcggct gagagggcgt 420
aggaattata tccttcttgc tcatcagttg atgatacgcc cgagcagatg ccaacacagc 480
agccattcaa gcaatcctat acaaccgtat cggcgatatc ggtttcatcc tcgccttagc 540
atgatttatc ctacactcca actcatgaga cccacaacaa atagcccttc taaacgctaa 600

tccaagcctc accccactac taggcctcct cctagcagca gcaggcaaat cagcccaatt 660

aggtctccac ccctgactcc cctcagccat agaaggcccc accccagtct cagccctact 720

ccactcaagc actatagttg tagcaggaat cttcttactc atccgcttcc accccctagc 780

agaaaatagc ccactaatcc aaactctaac actatgctta ggcgctatca ccactctgtt 840

cgcagcagtc tgcgccctta cacaaaatga catcaaaaaa atcgtagcct tctccacttc 900

aagtcaacta ggactcataa tagttacaat cggcatcaac caaccacacc tagcattcct 960

gcacatctgt acccacgcct tcttcaaagc catactattt atgtgctccg ggtccatcat 1020

ccacaacctt aacaatgaac aagatattcg aaaaatagga ggactactca aaaccatacc 1080

tctcacttca acctccctca ccattggcag cctagcatta gcaggaatac ctttcctcac 1140

aggtttctac tccaaagacc a 1161

<210> 88

<211> 598

<212> DNA

<213> Homo Sapiens

<220>

<221> variation

<222> (272)..(272)

<223> baySNP: 2297, C272T

<400> 88

agcactttgg gaggctgaag tgggtggagc acctcaggtc aggagttcaa gaccagcctg 60

gtcaacatgg ggaaacccta tctctactaa aaatacaaaa aattagccag tcatggtggt 120

gggcacctgt aatcccagct actcaggagg ctgaggcagg aaaatcgctt gaacccggga 180

ggcagaggtt gcagtaagct gagatggcgc tgttgcactc cagcctgggt ggcagagtga 240

gactccatct caaaaagaaa aagccaatgc ayggcagatt ataatttaag caactgaaaa 300

tttcttccat ctttttttcca gttttgcaca tttaaagata cccttattgt cacatgcttt 360

ttaaaaaatg atactcgtat catgcctatt aatttgcatg gttttccagc taaacaaaac 420

ctactcctcc aaagcttaca atacagcctg ttcatggggc agagagccca gcctagtgag 480

cagcctgcag tgtggggcca aacagtgagc tacagaagtg cctcattagg gactcctggt 540

actacagtca aaacaaggga acattttatc atggagcatt gggatattct aggaacaa 598

<210> 89

<211> 640

<212> DNA

<213> Homo Sapiens

<220>

<221> variation

<222> (491)..(491)

<223> baySNP:1583, C491T

<400> 89
tgctaaagga atgtttttta atctgacttt tataggaacc gttggaaact ggagacagtc 60

atatgggtgc attcagatgt gtgtgtgaca gggaaggagc agataagtac agcatatcag 120

aatgggtctc taatcctgtg tgtgaccaac actgctctgc gtatttattc ctattgatgg 180

tgtgatcatg ctattggctg taatgcagcc agcattacat gtcagcaagc atgcaacttc 240

ctgaagattc tctttactgc ccgctgctga ccctggtgct caatttctga tgctctctct 300

ctctgtcccc aggctcaaca agggctttca cttgccattg cagaaggtcc tgttattcaa 360

cagaatattc ctatgggacc tgcactgtca tgggtattaa ccacagattc tgctgcctct 420

gagggatgag aacagagaga aatatattca taatttactt tatgacctag aaggaaactg 480

tcgtgtgtcc yatacattgc catcaacttt gtttcctcat ctcaaataaa gtcctttcag 540

caagttcttt tgtgtttgtg cttttctggt gtttgataat tcaggattct tcagatgcaa 600

aaacaaaaac ccaagtcgta tctcagaaca ctagctcttc 640

<210> 90

<211> 1020

<212> DNA

<213> Homo Sapiens

<220>

<221> variation

<222> (773)..(773)

<223> BaySNP 5093, A773G

<400> 90
tggggaaaca attcacatct ttctcaatct gacctcaaga cagctcagag tgggtgacat 60

ttgtcccata ctgcaaacga ggaaagcaga gaagggggag gaagcacagc ctggcagcgg 120

gactggggct gggcagctgc acggcagggc cctcaggacc ctggagcctg caccccaccc 180

cactgcaagt gactgcttgg agcccaggca ctactgtggc ctctgcaagt gcctcacagg 240

cctttctgga gcagagcaag gcacacataa ccacacatga cccctgagct cccacctgag 300

gcccgccagg tctccagaaa catcccagcc acgtgtcccc gcagccaccc acgtgtgctg 360

tgggttaggc tcgcaccacc ccagacctgg ggagggcact ccgagtgtct ttacggggaa 420

gcactaccgc ggctgctgag ggacacgggg cagaggaagg aaccatgcaa agggacggac 480

cttgggctgc tgtgccagca atcccgcctc agcccctgaa ccccacccaa gcatgccagt 540

gcccacctgc tcctgcagct ccgccagcct ggtggcccgc tcctcctccg agtccgagct 600

gcctgagtcc gaagagctct cctcactgct acggctgctc tcagcgccct tgctcaccat 660

gggggccgcg gggcaggca gcgccggtgc ctccacgggc tcatctggca tcttggcaaa 720

cctcatctca aacacgtcct gcggcagaac agaggctgcc ctgaagacat ggrtgcccat 780

ggggctgccc cttggtgtgg gccctcaggg tttctgaggt gcccctgagg cagcacccac 840

agctgttacg aaggtggggc taggaggaga cagggtcccg cctgcctggg gggctgtggt 900

tgcttccagc ttgcccaccc tccctcccca ggcaattcct gcggacaggc ctgggaagag 960

gggacacagg agctggccca cagctccctt cagcaactcc atgagccctc tctagaagat 1020

<210> 91

<211> 723

<212> DNA

<213> Homo Sapiens

<220>

<221> variation

<222> (307)..(307)

<223> baySNP: 1275, C307G

<400> 91
ctgaagtgtg aggccaacac cagggagccc ctaggggaga acagagttga gggggctct 60

agggctcaag gtttggctga gccaccccag cagcccccat tctcctgctg cctcacctgg 120

gccccaggca gcagaaccag cagcagcccc agaaggagga ggtgtagggt ggtgccacac 180

acccttggga ggaagagacg ttcaggtggt gtcatgggga gaacctgcag agaaagagag 240

agagagagag agagcagtga gcggggcggg gcacgcggcg gaagacagac ctcccgccct 300

gggagasagc accccccgac ccccgagaga gagatcgaca gagaagggga caagatgcag 360

tcagagaaac cccaaggtga gcagagggag acagagagag acaggaaggg aacagagagg 420

aatcatggca gaaacagaga atgtgtgaca gagacaatga gactgacaga tggagagtca 480

gagacagaga aggaaaccaa aaccaaaccc accaaggccc aggcccaggc aggccgggga 540

tccaggcagc aggtgcagga gggaccgagg cccaggcaga gggcaggaca ctgctgggcg 600

gtagtccaaa gcacgaagca cgggcagccc aaggagatgg ggcaggagaa cctcacctgc 660

tgtgcggagc ccctggcccg gacgctcagg tcctttata aagaaccgga ttggcaacct 720

tgc 723

<210> 92

<211> 903

<212> DNA

<213> Homo Sapiens

<220>

<221> variation

<222> (317)..(317)

<223> baySNP:1669, C317T

<220>

<221> misc_feature

<222> (561)..(567)

<223> n: Unsure

<220>

<221> misc_feature

<222> (559)..(559)

<223> n: Unsure

<220>

<221> misc_feature

<222> (572)..(574)

<223> n: Unsure

<220>

<221> misc_feature

<222> (576)..(577)

<223> n: Unsure

<220>

<221> misc_feature

<222> (712)..(712)

<223> n: Unsure

<400> 92

```
gcatcactga caacatgttc tgtgctggca agtctgtgca gggcgggctg agggaacagt      60
ggggcccaag ctgggagaac tgagttgtgc ctgggttcaa gccatgtgac tttgagcaag     120
ttgcctaacc tcttggtggc tcagtttctt cctctgtaaa atggaggtaa aagtctctat     180
cccataaggt tatgggaggg ttaaatgaag tagtatatat taatgtactt ggcatagtat     240
cagtcaccag tgagctcaga tagcagcaag aggctgcggg tagggaaatg ccattcattc     300
agtcactcag caaataytta ttgagcgcct atcacgttcc aggcagcgtt ctagggtata     360
cagcagggac ccagacggac aatgtctgtg ccctcagaga gcttccttcc taggagggca     420
catccataaa cagatctaaa acagcaatcc ctgaccagtg ctgtgaagaa aatgaagcac     480
```

agggagagag aacggctgat gaagtgggct tctaaatagg gtggccagac caggctgggt 540
catatctctg gaggggcang nnnnnnnaac cnnncnnggg caccacgggc aaaaccccgc 600
ccttcctaaa aaaccaaaat taactgggcc tggggaccca tgccttgtgt tgcactttct 660
taggaaggct tgaggcggag aatcggctga cccacggagc ggaggttgaa tnagcttaaa 720
cttgtccttt ttgccttcca ctgggtaacc tgccgactcc ttgtttgttt gccaattatt 780
cataaggggc cagaaatgct cggaaaggtc cccataatgg cactcttgcc cggcttgtgc 840
aggctacgca agggcccgcc ctttttgtg gcgcaccccc tgtggcgatg aacgtcctaa 900
aaa 903

<210> 93

<211> 500

<212> DNA

<213> Homo Sapiens

<220>

<221> variation

<222> (225)..(225)

<223> baySNP: 11248, C225T

<400> 93
acgcccctgg cctcccaccc tccaacccca aaaagctctg gaagccgggg gagggacgga 60
gcgaagcccc ggctccctga gttgaacagc acttggcgac agccggtgcg cgccctgcca 120
cttcttgctt gctatagtta gaaaaaaata tatatatatg catctacccg gcgcccaata 180
actagccaga cccggctgaa ggaaagctaa ctcccctgac gcgaygccaa gcgaaagaat 240
cagcagtcga gtgagccggt cgctttggta agattttccc tcccttaccc ttcaactcct 300
cccgctgcca ggagagctgc caggtaggac ggcgggcaaa gcgcttcttc aagttcctgc 360
gctggagtca ccacccgaga ggcatcgccg tctggcgttg gtgtctgagc agaagccgca 420
ggaaccagcg ggagcagcag gaggagctgc gggactcgtt tgccgaggct cgtgttctgc 480
tcaaaagttt gtagacgcgc 500

<210> 94

<211> 851

<212> DNA

<213> Homo Sapiens

<220>

<221> variation

<222> (516)..(516)

<223> BaySNP 2321, G516T

<400> 94
ctgcgcttgc tgcgcgggga gctgggccgc tttccgcccg agagtctccg ccggcgccgt 60

cgcgctctct ggccccggcc ccggtgggga cgtgcgctcc gcccgaaggg gtgcccgcct 120

gcggccggcg gcccgcgcgc ctcctgcctc tccgggaaca ccgggccctg tgcaccttgg 180

gtctcatcat gggcaccttc actctctgct ggttgccctt ctttctggcc aacgtgctgc 240

gcgccctggg gggcccctct ctagtcccgg gcccggcttt ccttgccctg aactggctag 300

gttatgccaa ttctgccttc aacccgctca tctactgccg cagcccggac tttcgcagcg 360

ccttccgccg tcttctgtgc cgctgcggcc gtcgcctgcc tccggagccc tgcgccgccg 420

cccgcccggc cctcttcccc tcgggcgttc ctgcggcccg gagcagccca gcgcagccca 480

ggctttgcca acggctcgac gggtaggtaa ccgggkcaga gggaccggcg gctcagggtc 540

gggaagcatg cgatgtgtcc gtgggtcaac tttttgagtg tggagtttat taagagaagg 600

tgggatggct ttgcttggag agaaaaggga acgaggagta gcgaaccaaa atgggaccca 660

gggtcctttt ctttccgatc cagtcactag ggtagaagca aaggagggcg agcgggccgt 720

cgttcctcac ccaagaccca aggtgcgcca ccggaagcgc tggcggtgtc ccgaggactc 780

tcgctcgcct ggtcgcttta gggatttttt ttttttttaa tagaaacagg gttttgtttt 840

tgtggccagc g 851